(19) Europäisches Patentamt
European Patent Office
Office européen des brevets

(11) Publication number: 0 027 599 B1

(12) **EUROPEAN PATENT SPECIFICATION**

(45) Date of publication of patent specification: **03.10.84**

(21) Application number: **80106112.8**

(22) Date of filing: **08.10.80**

(51) Int. Cl.³: **C 07 D 501/36,**
C 07 D 501/46, A 61 K 31/545
// C07D285/08, C07D209/48,
C07D405/12

(54) **Cephem compounds, processes for their preparation and pharmaceutical compositions containing them.**

(30) Priority: **12.10.79 GB 7935538**
**21.12.79 DK 5542/79**
**07.03.80 US 128260**
**18.06.80 US 160904**
**22.08.80 US 180295**

(43) Date of publication of application:
**29.04.81 Bulletin 81/17**

(45) Publication of the grant of the patent:
**03.10.84 Bulletin 84/40**

(84) Designated Contracting States:
**AT BE CH DE FR GB IT LU NL SE**

(58) References cited:
**FR-A-2 137 899**
**FR-A-2 384 780**

**The file contains technical information submitted after the application was filed and not included in this specification**

(73) Proprietor: **Fujisawa Pharmaceutical Co., Ltd.**
**3, Doshomachi 4-chome Higashi-ku**
**Osaka-shi, Osaka 541 (JP)**

(72) Inventor: **Teraji, Tsutomu**
**No. 6-20-6, Kofudai**
**Toyono-gun Osaka-fu (JP)**
Inventor: **Sakane, Kazuo**
**No. 6-60-6, Higashi-sonoda-cho**
**Amagasaki (JP)**
Inventor: **Goto, Jiro**
**A-401 Senridai-sukai-taun**
**No. 21 Kashikiriyama Suita (JP)**

(74) Representative: **Türk, Dietmar, Dr. rer. nat. et al**
**Redies, Redies, Türk & Gille Patentanwälte**
**Brucknerstrasse 20**
**D-4000 Düsseldorf 13 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 027 599 B1

## Description

The present invention relates to new cephem compounds and pharmaceutically acceptable salts thereof. More particularly, it relates to new cephem compounds and pharmaceutically acceptable salts thereof, which have antimicrobial activities and to processes for preparation thereof, to pharmaceutical composition comprising the same, and to the same for use therapeutically in the treatment of infectious diseases in human beings and animals.

Accordingly, it is one object of the present invention to provide new cephem compounds and pharmaceutically acceptable salts thereof, which are active against a number of pathogenic microorganisms.

Another object of the present invention is to provide processes for the preparation of new cephem compounds and pharmaceutically acceptable salts thereof.

A further object of the present invention is to provide pharmaceutical composition comprising, as active ingredients, said new cephem compounds and pharmaceutically acceptable salts thereof.

Still further object of the present invention is to provide a method for the treatment of infectious diseases caused by pathogenic bacteria in human beings and animals.

7-Thiadiazolyl substituted cephem compounds are disclosed in FR—A—21 37 899, but these compounds are not exemplified. Further, there is no specific disclosure of the 1,2,4-thiadiazolyl group of the present invention nor of the 5-amino-1,2,4-thiadiazolyl group.

The FR—A—23 84 780 discloses cephem compounds bearing an optionally amino substituted 1,2,4-thiadiazolyl group, but does not present any working example on such compounds. Furthermore, the compounds described therein have quite different substituents in the 3-position of the ring system.

Test results show that the compounds of the present invention have superior antibacterial properties as compared to the compounds disclosed in the above references.

The object new cephem compounds are novel and can be represented by the following general formula (I).

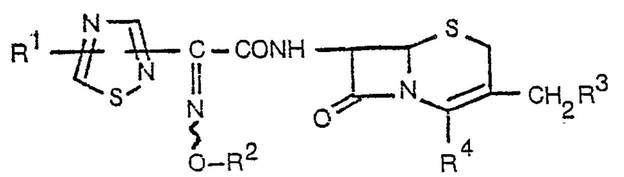

(I)

wherein

$R^1$ is amino or protected amino;

$R^2$ is hydrogen, $(C_1—C_6)$-alkyl which may be substituted with phenyl, carboxy, esterified carboxy, $(C_1—C_6)$-alkylthio or halogen, $(C_2—C_6)$-alkenyl, $(C_2—C_6)$-alkynyl, cyclo$(C_3—C_6)$-alkyl, cyclo$(C_3—C_6)$alkenyl, or tetrahydrofuryl substituted with an oxo group;

$R^3$ is a group of the formula:

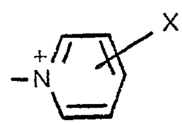

wherein X is hydrogen or carbamoyl; and

$R^4$ is $—COO^-$; or

$R^3$ is 2-$(C_1—C_8)$-alkyl-5-oxo-6-hydroxy-2,5-dihydro-1,2,4-triazinylthio; and

$R^4$ is carboxy or esterified carboxy.

According to the present invention, the object compound (I) can be prepared by the following processes.

PROCESS 1

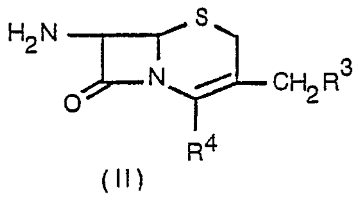

(II)

or its reactive derivative at the amino group or a salt thereof

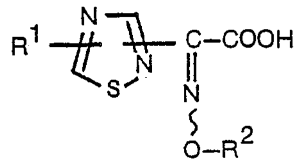

(III)

or its reactive derivative at the carboxy group or a salt thereof

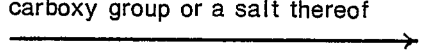

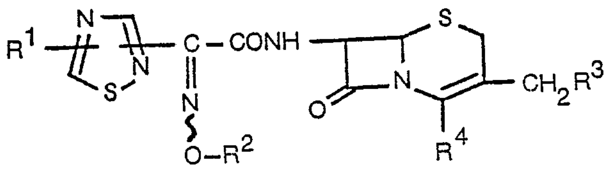

(I)

or a salt thereof

PROCESS 2

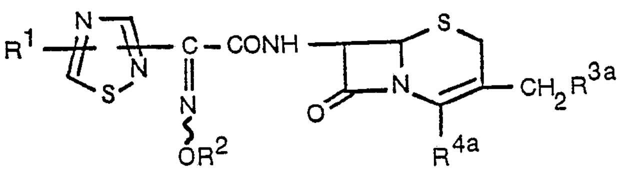

+ $R^{3b}$



(XIII) (IV)

or a salt thereof

or its reactive derivative

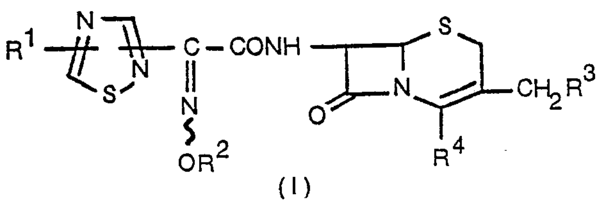

(I)

or a salt thereof

PROCESS 3

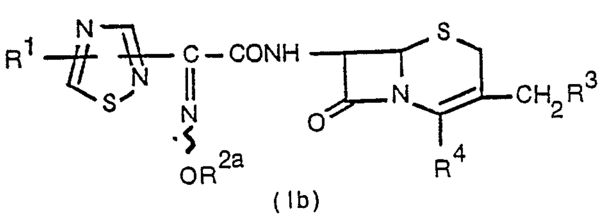

(Ib)

or a salt thereof

Elimination of the protective group of carboxy

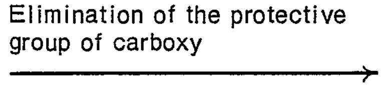

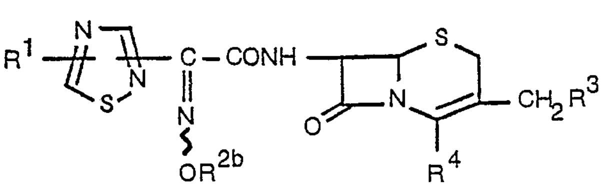

(Ic)

or a salt thereof

PROCESS 4

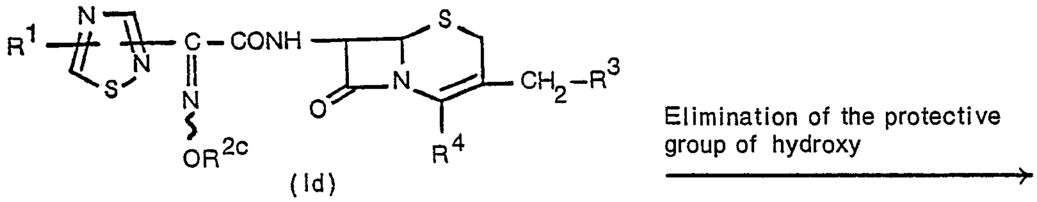


(Id)

or a salt thereof

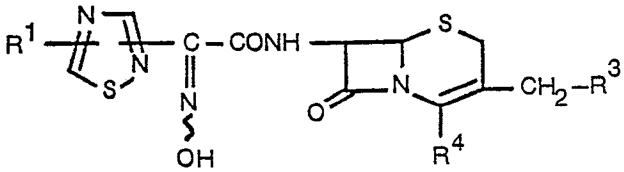

(Ie)

or a salt thereof

wherein

$R^1$, $R^2$, $R^3$ and $R^4$ are each as defined above;

$R^{3a}$ is a group which can be substituted with a group of the formula: $R^3$ wherein $R^3$ is as defined above;

$R^{3b}$ is a compound of the formula:

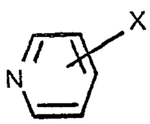

wherein

X is as defined above and

$R^{4a}$ is carboxy; or

$R^{3b}$ is a compound of the formula: $R^{3c}$-H wherein $R^{3c}$ is 2-lower alkyl-5-oxo-6-hydroxy-2,5-dihydro-1,2,4-triazinylthio and

$R^{4a}$ is carboxy or protected carboxy;

$R^{2a}$ is a protected carboxy(lower)alkyl;

$R^{2b}$ is a carboxy(lower)alkyl; and

$R^{2c}$ is a protective group of hydroxy.

Among the starting compounds of the present invention, some of the compounds of the formula (Ia) are novel and can be prepared by the following methods.

1.

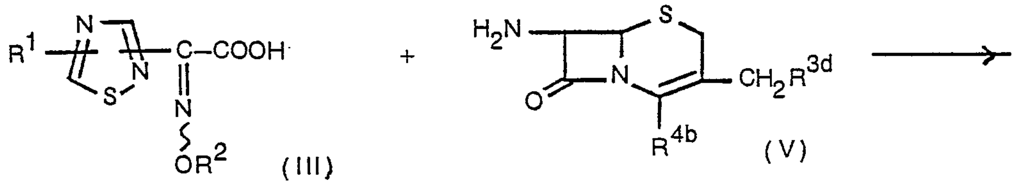

(III) + (V)

or its reactive derivative at the carboxy group or a salt thereof

or its reactive derivative at the amino group or a salt thereof

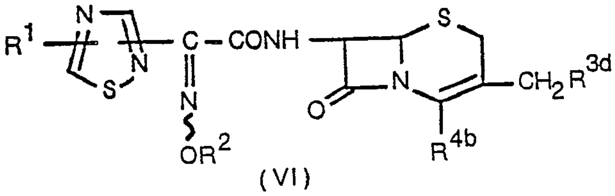

(VI)

or a salt thereof

2.

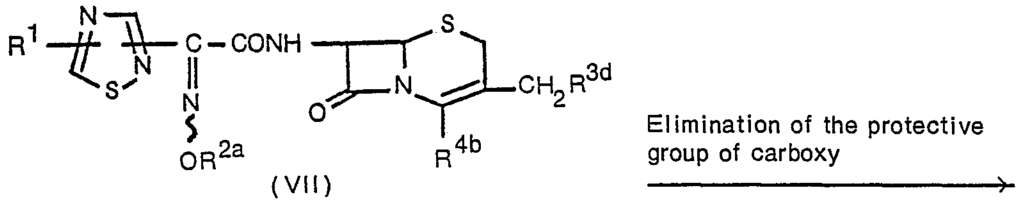


(VII)

or a salt thereof

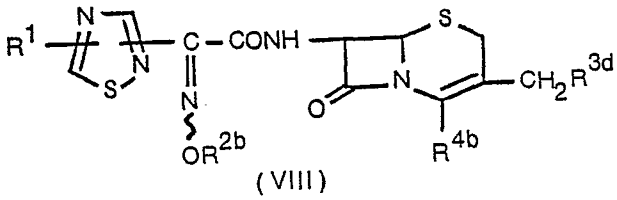

or a salt thereof

(VIII)

wherein $R^1$, $R^2$, $R^{2a}$ and $R^{2b}$ are each as defined above, $R^{3d}$ is lower alkanol(lower)alkanoyloxy and $R^{4b}$ is carboxy or protected carboxy.

Further, some of the compound (II) can be prepared by the following methods.

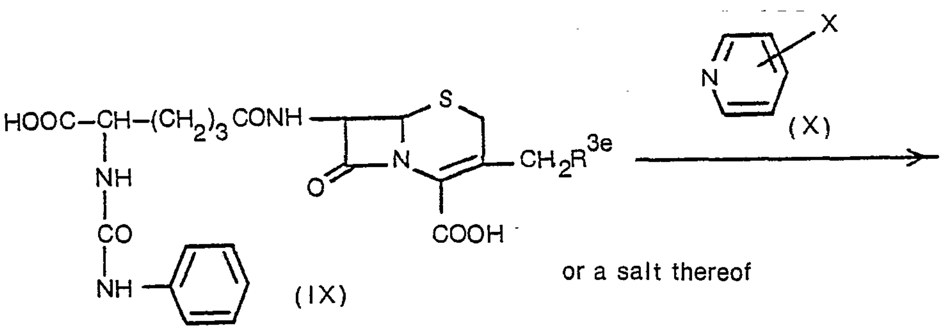

(X)

or a salt thereof

(IX)

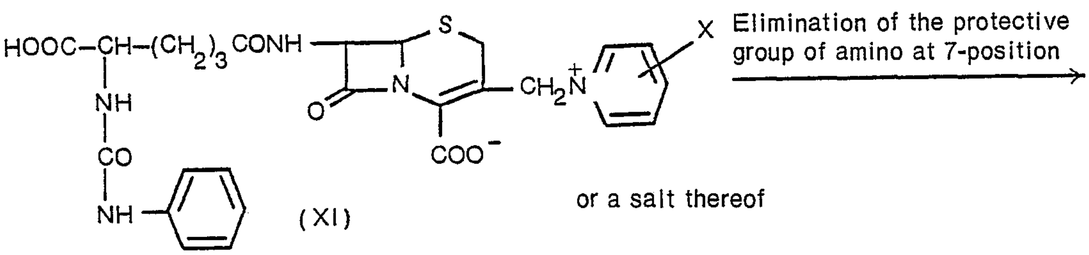


or a salt thereof

(XI)

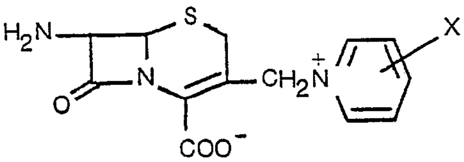

or a salt thereof

(XII)

wherein X is as defined above and $R^{3e}$ is a group which can be substituted with a group of the formula:

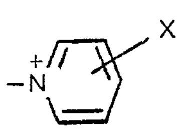

Among these compounds, the compound (XI) is novel.

Regarding the object compounds (I), (Ib), (Ic), (Id) and (Ie) and the starting compounds (III), (VI), (VII), (VIII) and (XIII), it is to be understood that they include tautomeric isomers.

That is, in case that the group of the formula:

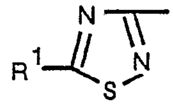

($R^1$ is as defined above) is contained in the molecules of said object and starting compounds, said group of the formula can also be alternatively represented by its tautomeric formula:

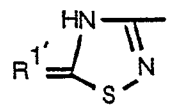

($R^{1'}$ is imino or a protected imino group). That is, the both of said groups are in the state of equilibrium each other and such tautomerism can be represented by the following equilibrium.

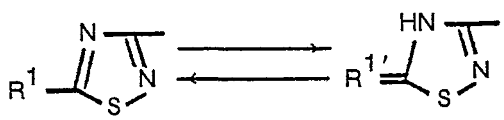

wherein $R^1$ and $R^{1'}$ are each as defined above.

These types of tautomerism between the amino-compound and the corresponding imino-compound as stated above have been well known in the literature, and it is obvious to a person skilled in the arts that both of the tautomeric isomers are easily convertible reciprocally and are included within the same category of the compound *per se*. Accordingly, the both of the tautomeric forms of the object and starting compounds as mentioned above are clearly included within the scope of the present invention. In the present specification and claims, the object and starting compounds including the group of such tautomeric isomers are represented by using one of the expressions therefor, that is the formula:

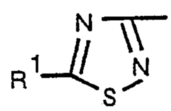

Furthermore, regarding the object compounds (I), (Ib), (Ic), (Id) and (Ie) and the starting compounds (III), (VI), (VII), (VIII) and (XIII), it is to be understood that said object and starting compounds include syn isomer, anti isomer and a mixture thereof. For example, with regard to the object compound (I), syn isomer means one geometrical isomer having the partial structure represented by the following formula:

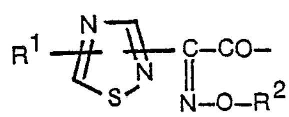

(wherein $R^1$ and $R^2$ are each as defined above) and anti isomer means the other geometrical isomer having the partial structure represented by the following formula:

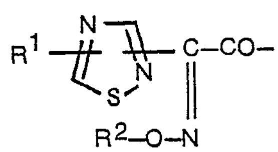

(wherein $R^1$ and $R^2$ are each as defined above).

Regarding the other object and starting compounds as mentioned above, the syn isomer and the anti isomer can also be referred to the same geometrical isomers as illustrated for the compound (I).

Suitable pharmaceutically acceptable salts of the object compounds (I) are conventional non-toxic salt and include a metal salt such as an alkali metal salt (e.g. sodium salt, potassium salt) and an alkaline earth metal salt (e.g. calcium salt, magnesium salt), an ammonium salt, an organic base salt (e.g. trimethylamine salt, triethylamine salt, pyridine salt, picoline salt, dicyclohexylamine salt, N,N'-dibenzylethylenediamine salt), an organic acid salt (e.g. acetate, maleate, tartrate, methanesulfonate, benzenesulfonate, formate, toluenesulfonate), an inorganic acid salt (e.g. hydrochloride, hydrobromide, hydroiodide, thiocyanate, sulfate, phosphate), or a salt with an amino acid (e.g. arginine, aspartic acid, glutamic acid).

In the above and subsequent descriptions of the present specification, suitable examples and illustrations of the various definitions which the present invention include within the scope thereof are explained in details as follows.

The term "lower" is intended to mean 1 to 6 carbon atoms, unless otherwise indicated.

Suitable "protected amino" for $R^1$ may include an acylamino or an amino group substituted by a conventional protecting group such as ar(lower)alkyl which may have at least one suitable substituent(s), (e.g. benzyl, trityl).

Suitable acyl moiety in the term "acylamino" may include carbamoyl, aliphatic acyl group and acyl group containing an aromatic or heterocyclic ring. And, suitable examples of the said acyl may be lower alkanoyl (e.g. formyl, acetyl, propionyl, butyryl, isobutyryl, valeryl, isovaleryl, oxalyl, succinyl, pivaloyl); lower alkoxycarbonyl (e.g. methoxycarbonyl, ethoxycarbonyl, propoxycarbonyl, 1-cyclopropylethoxycarbonyl, isopropoxycarbonyl, butoxycarbonyl, tertiarybutoxycarbonyl, pentyloxycarbonyl, hexyloxycarbonyl); lower alkanesulfonyl (e.g. mesyl, ethanesulfonyl, propanesulfonyl, isopropanesulfonyl, butanesulfonyl); arenesulfonyl (e.g. benzenesulfonyl, tosyl); aroyl (e.g. benzoyl, toluoyl, xyloyl, naphthoyl, phthaloyl, indancarbonyl); ar(lower)alkanoyl (e.g. phenylacetyl, phenylpropionyl); ar(lower)alkoxycarbonyl (e.g. benzoyloxycarbonyl, phenethyloxycarbonyl), and the like.

The acyl moiety as stated above may have at least one suitable substituent(s) such as halogen (chlorine, bromine, fluorine and iodine), lower alkanoyl as stated above.

Suitable "lower alkyl" for $R^2$ is one having 1 to 6 carbon atom(s) and may include methyl, ethyl, propyl, isopropyl, butyl, isobutyl, tert-butyl, pentyl, tert-pentyl, hexyl, and preferably one having 1 to 4 carbon atom(s).

Suitable "lower alkyl" being the substituent on 1,2,4-triazinylthio for $R^3$ and lower alkyl moieties the terms "protected carboxy(lower)alkyl" and "carboxy(lower)alkyl" can be referred to the ones as exemplified above.

"Lower alkyl" for $R^2$ may be substituted with 1 to 3 suitable substituent(s) such as halogen (e.g. chlorine, bromine, fluorine or iodine); carboxy; protected carboxy as mentioned below; lower alkylthio (e.g., methylthio, ethylthio, propylthio, butylthio); aryl (e.g., phenyl, tolyl, xylyl, mesityl, cumenyl).

Suitable lower alkenyl may include vinyl, allyl, isopropenyl, 1-propenyl, 2-butenyl, 3-pentenyl preferably one having 2 to 4 carbon atoms.

Suitable lower alkynyl may include one having 2 to 6 carbon atoms, for example, ethynyl, 2-propynyl, 2-butynyl, 3-pentynyl, 3-hexynyl, preferably one having 2 to 4 carbon atoms.

Suitable cyclo(lower)alkyl may include one having 3 to 6 carbon atoms, for example, cyclopropyl, cyclobutyl, cyclopentyl, cyclohexyl preferably one having 4 to 6 carbon atoms.

Suitable cyclo(lower)alkenyl may include one having 3 to 6 carbon atoms, for example, cyclopentenyl, cyclohexenyl, preferably one having 5 or 6 carbon atoms.

Suitable O containing 5-membered heterocyclic group may include saturated or unsaturated one, for example, dihydrofuryl, tetrahydrofuryl, which is substituted with 1 or 2 oxo group(s).

Suitable protected carboxy and protected carboxy moiety in the term "protected carboxy(lower)alkyl" may include esterified carboxy in which said ester may be the ones such as lower alkyl ester (e.g., methyl ester, ethyl ester, propyl ester, isopropyl ester, butyl ester, isobutyl ester, t-butyl ester, pentyl ester, t-pentyl ester, hexyl ester, 1-cyclopropylethyl ester), wherein lower alkyl moiety may be preferably one having 1 to 4 carbon atom(s); lower alkenyl ester (e.g., vinyl ester, allyl ester); lower alkynyl ester (e.g., ethynyl ester, propynyl ester); mono(or di or tri)-halo-(lower)alkyl ester (e.g., 2-iodoethyl ester, 2,2,2-trichloroethyl ester); lower alkanoyloxy(lower)alkyl ester (e.g., acetoxymethyl ester, propionyloxymethyl ester, 1-acetoxypropyl ester, valeryloxymethyl ester, pivaloyloxymethyl ester, hexanoyloxymethyl ester, 1-acetoxyethyl ester, 2-propionyloxyethyl ester, 1-isobutyryloxyethyl ester); lower alkanesulfonyl(lower)alkyl ester (e.g., mesylmethyl ester, 2-mesylethyl ester); ar(lower)alkyl ester, for example, phenyl(lower)alkyl ester which may be substituted with one or more suitable substituent(s) (e.g., benzyl ester, 4-methoxybenzyl ester, 4-nitrobenzyl ester, phenethyl ester, trityl ester, diphenylmethyl ester, bis(methoxyphenyl)methyl ester, 3,4-dimethoxybenzyl ester, 4-hydroxy-3,5-ditertiarybutylbenzyl ester); lower alkoxycarbonyloxy(lower)alkyl ester (e.g., methoxycarbonyloxymethyl ester, ethoxycarbonyloxymethyl ester, ethoxycarbonyloxyethyl ester) which may be substituted with azido; a heterocyclic ester, preferably benzotetrahydrofuryl ester which may be substituted with oxo group, more preferably phthalidyl ester; aroyloxy(lower)alkyl ester (e.g. benzoyloxymethyl ester, benzoyloxyethyl ester, toluoyloxyethyl ester); aryl ester which may have one or more suitable substituent(s) (e.g., phenyl ester, tolyl ester, tertiary-butylphenyl ester, xylyl ester, mesityl ester, cumenyl ester).

Preferable example of protected carboxy may be lower alkoxycarbonyl (e.g., methoxycarbonyl, ethoxycarbonyl, propoxycarbonyl, butoxycarbonyl, t-butoxycarbonyl, t-pentyloxycarbonyl, hexyloxycarbonyl) having 2 to 7 carbon atoms, preferably one having 2 to 5 carbon atoms and phenyl(lower)-alkoxycarbonyl which may be substituted with nitro (e.g., 4-nitrobenzyloxycarbonyl, benzoyloxycarbonyl, 4-nitrophenethyloxycarbonyl).

Preferable example for $R^{3a}$ and $R^{3e}$ may include acyloxy, halogen, azido and the like, wherein acyl moiety in the term "acyloxy" and halogen can be referred to the ones as exemplified hereinbefore.

Suitable protective group of hydroxy may include aforesaid acyl, ar(lower)alkyl (e.g., benzyl, trityl).

Suitable lower alkanoyl(lower)alkanoyloxy may include acetoacetoxy, propionylacetoxy, acetopropionyloxy.

Preferred embodiments of the object compound (I) are as follows.

Preferred embodiment of $R^1$ is amino;

$R^2$ is hydrogen, lower alkyl, ar(lower)alkyl[more preferably triphenyl(lower)alkyl], lower alkylthio(lower)alkyl, halo-(lower)alkyl[more preferably trihalo(lower)alkyl], carboxy-(lower)alkyl, esterified carboxy(lower)alkyl[more preferably lower alkoxycarbonyl(lower)alkyl or phenyl(lower)alkoxycarbonyl(lower)alkyl], lower alkenyl, lower alkynyl, cyclo(lower)alkyl, cyclo(lower)alkenyl, or tetrahydrofuryl substituted with oxo group:

$R^3$ is a group of the formula:

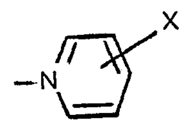

wherein X is hydrogen or carbamoyl and $R^4$ is $-COO^-$; or

$R^3$ is 2-lower alkyl-5-oxo-6-hydroxy-2,5-dihydro-1,2,4-triazinylthio and $R^4$ is carboxy.

The processes for preparing the object compounds are explained in details in the following.

*Process 1*

The object compound (I) can be prepared by reacting the compound (II) or its reactive derivative at the amino group or a salt thereof with the compound (III) or its reactive derivative at the carboxy group or a salt thereof.

Suitable reactive derivative at the amino group of the compound (II) may include conventional reactive derivative used in amidation, for example, Schiff's base type imino or its tautomeric enamine type isomer formed by the reaction of the compound (II) with a carbonyl compound; a silyl derivative formed by the reaction of the compound (II) with a silyl compound such as bis (trimethylsilyl)acetamide, trimethylsilylacetamide; a derivative formed by reaction of the compound (II) with phosphorus trichloride or phosgene.

Suitable salt of the compound (II) may include an acid addition salt such as an organic acid salt (e.g., acetate, maleate, tartrate, benzenesulfonate, toluenesulfonate) or an inorganic acid salt (e.g., hydrochloride, hydrobromide, sulfate, phosphate,); a metal salt (e.g., sodium salt, potassium salt, calcium salt, magnesium salt); ammonium salt; an organic amine salt (e.g., triethylamine salt, dicyclohexylamine salt).

Suitable reactive derivative at the carboxy group of the compound (III) may include an acid halide, an acid anhydride, an activated amide, an activated ester. The suitable example may be an acid chloride; an acid azide; a mixed acid anhydride with an acid such as substituted phosphoric acid (e.g., dialkylphosphoric acid, phenylphosphoric acid, diphenylphosphoric acid, dibenzylphosphoric acid, halogenated phosphoric acid), dialkylphosphorous acid, sulfurous acid, thiosulfuric acid, sulfuric acid, alkylcarbonic acid, aliphatic carboxylic acid (e.g., pivalic acid, pentanoic acid, isopentanoic acid, 2-ethylbutyric acid, acetic acid or trichloroacetic acid) or aromatic carboxylic acid (e.g., benzoic acid); a symmetrical acid anhydride; an activated amide with imidazole, dimethylpyrazole, triazole or tetrazole; or an activated ester (e.g., cyanomethyl ester, methoxymethyl ester, dimethyliminomethyl[$(CH_3)_2N^+=CH-$] ester, vinyl ester, propargyl ester, p-nitrophenyl ester, 2,4-dinitrophenyl ester, trichlorophenyl ester, pentachlorophenyl ester, mesyl phenyl ester, phenylazophenyl ester, phenyl thioester, p-nitrophenyl thioester, p-cresyl thioester, carboxymethyl thioester, pyranyl ester, pyridyl ester, piperidyl ester, 8-quinolyl thioester, or an ester with N,N-dimethylhydroxylamine, 1-hydroxy-2-(1H)-pyridone, N-hydroxysuccinimide, N-hydroxyphtalimide or 1-hydroxy-6-chloro-1H-benzotriazole. These reactive derivatives can be optionally selected from them according to the kind of the compound (III) to be used.

The salts of the compound (III) may be salts with an inorganic base such as an alkali metal salts (e.g., sodium or potassium salt), or an alkaline earth metal salt (e.g., calcium or magnesium salt), a salt with an organic base such as trimethylamine, triethylamine, pyridine, a salt with an acid (e.g., hydrochloric acid or hydrobromic acid).

The reaction is usually carried out in a conventional solvent such as water, acetone, dioxane, acetonitrile, chloroform, methylene chloride, ethylene chloride, tetrahydrofuran, ethyl acetate, N,N-dimethylformamide, pyridine or any other organic solvent which does not adversely influence to the reaction. Among these solvents, hydrophilic solvents may be used in a mixture with water.

When the compound (III) is used in free acid form or its salt form in the reaction, the reaction is preferably carried out in the presence of a conventional condensing agent such as N,N-dicyclohexylcarbodiimide; N-cyclohexyl-N'-morpholinoethylcarbodiimide; N-cyclohexyl-N'-(4-diethylaminocyclohexyl)carbodiimide; N,N-diethylcarbodiimide; N,N-diisopropylcarbodiimide; N-ethyl-N'-(3-dimethylaminopropyl)carbodiimide; N,N-carbonylbis (2-methylimidazole); pentamethylene-ketene-N-cyclohexylimine; diphenylketene-N-cyclohexylimine; ethoxyacetylene; ethyl polyphosphate; isopropyl polyphosphate; diethyl phosphorochloridite; phosphorus oxychloride; phosphorus trichloride; phosphorus

pentachloride; thionyl chloride; oxalyl chloride; triphenylphosphine; N-ethyl-7-hydroxybenzisoxazolium fluoroborate; N-ethyl-5-phenylisoxazolium-3′-sulfonate; 1 - (p - chlorobenzenesulfonyloxy) - 6 - chloro - 1H - benzotriazole; so-called Vilsmeier reagent, for example (chloromethylene) dimethylammonium chloride produced by the reaction of dimethylformamide with thionyl chloride or phosgene, a compound produced by the reaction of dimethylformamide with phosphorus oxychloride.

The reaction may be also carried out in the presence of an inorganic or an organic base such as an alkali metal hydroxide, an alkali metal bicarbonate, alkali metal carbonate, alkali metal acetate, tri(lower)alkylamine, pyridine, N-(lower)alkylmorpholine, N,N-di(lower)alkylbenzylamine, N,N-di(lower)-alkylaniline as exemplified below. When the base or the condensing agent is in liquid, it can be used also as a solvent. The reaction temperature is not critical, and the reaction is usually carried out under cooling or at ambient temperature.

In the present reaction, a syn-isomer of the object compound (I) can be obtained preferably by conducting the reaction of the compound (II) with a syn isomer of the starting compound (III).

*Process 2*

The object compound (I) or a salt thereof can be prepared by reacting the compound (XIII) or a salt thereof with the compound (IV) or its reactive derivative.

Suitable salt of the compound (XIII) can be referred to the ones exemplified for the compound (II).

Suitable reactive derivative of the compound (IV) may include a metal salt such as an alkali metal salt (e.g., sodium salt, potassium salt).

The present reaction may be carried out in a solvent such as water, phosphate buffer, acetone, chloroform, nitrobenzene, methylene chloride, ethylene chloride, dimethylformamide, methanol, ethanol, ether, tetrahydrofuran, dimethylsulfoxide, or any other organic solvent which does not adversely affect the reaction, preferably in ones having strong polarities. Among the solvents, hydrophilic solvents may be used in a mixture with water. The reaction is preferably carried out in around neutral medium. When the compound (XIII) or the compound (IV) is used in a free form, the reaction is preferably conducted in the presence of a base, for example, inorganic base such as alkali metal hydroxide, alkali metal carbonate, alkali metal bicarbonate, organic base such as trialkylamine. The reaction temperature is not critical, and the reaction is usually carried out at ambient temperature, under warming or under slightly heating.

The present reaction includes, within its scope, the case that protected carboxy group is converted to free carboxy group during the course of the reaction.

*Process 3*

The object compound (Ic) or a salt thereof can be prepared by subjecting the compound (Ib) or a salt thereof to elimination reaction of the protective group of carboxy.

Suitable salt of the compound (Ib) can be referred to the ones as exemplified for the compound (II).

The present reaction is carried out in accordance with a conventional method such as hydrolysis reduction.

In case that the protective group is an ester, the protective group can be eliminated by hydrolysis. Hydrolysis is preferably carried out in the presence of a base or an acid. Suitable base may include an inorganic base and an organic base such as an alkali metal (e.g., sodium, potassium, etc.), an alkaline earth metal (e.g., magnesium, calcium, etc.), the hydroxide or carbonate or bicarbonate thereof, trialkylamine (e.g., trimethylamine, triethylamine, etc.), picoline, 1,5-diazabicyclo[4,3,0]none-5-ene, 1,4-diazabicyclo[2,2,2]octane, 1,8-diazabicyclo[5,4,0]undecene-7, or the like. Suitable acid may include an organic acid (e.g., formic acid, acetic acid, propionic acid, trifluoroacetic acid, etc.) and an inorganic acid (e.g., hydrochloric acid, hydrobromic acid, sulfuric acid, etc.).

The reaction is usually carried out in a solvent such as water, an alcohol (e.g., methanol, ethanol, etc.), a mixture thereof or any other solvent which does not adversely influence to the reaction. A liquid base or acid can be also used as the solvent. The reaction temperature is not critical and the reaction is usually carried out under cooling to warming.

Reduction can be applied preferably for elimination of the protective group such as 4-nitrobenzyl, 2-iodoethyl, 2,2,2-trichloroethyl, or the like. The reduction method applicable for the elimination reaction may include, for example, reduction by using a combination of a metal (e.g., zinc, zinc amalgam, etc. or a salt of chrome compound (e.g., chromous chloride, chromous acetate, etc.) and an organic or inorganic acid (e.g., acetic acid, propionic acid, hydrochloric acid, etc.); and conventional catalytic reduction in the presence of a conventional metallic catalyst (e.g., palladium-carbon, etc.).

*Process 4*

The object compound (Ie) or a salt thereof can be prepared by subjecting the compound (Id) or a salt thereof to elimination reaction of the protective group of hydroxy.

Suitable salt of the compound (Id) can be referred to the ones as exemplified for the compound (II).

The present elimination reaction can be carried out according to substantially the same manner as that of acidic hydrolysis in *Process 3.*

The preparation for preparing the starting compounds are explained below in detail.

Preparation 1

The compound (VI) or a salt thereof can be prepared by reacting the compound (III) or its reactive derivative at the carboxy group or a salt thereof with the compound (V) or its reactive derivative at the amino group or a salt thereof.

Suitable reactive derivative and salt for the compound (V) can be referred to the ones as exemplified for the compound (II).

The present reaction can be carried out in substantially the same manner as that of *Process 1.*

Preparation 2

The compound (VIII) or a salt thereof can be prepared by subjecting the compound (VII) or a salt thereof to elimination reaction of the protective group of carboxy.

Suitable salt of the compound (VII) can be referred to the ones as exemplified for the compound (II).

The present reaction can be carried out in substantially the same manner as that of *Process 3.*

Preparation 3

The compound (XI) or a salt thereof can be prepared by reacting the compound (IX) or a salt thereof with the compound (X). Suitable salt of the compound (IX) can be referred to the ones as exemplified for the compound (II).

The present reaction can be carried out in substantially the same manner as that of *Process 2.*

Preparation 4

The compound (XII) or a salt thereof can be prepared by subjecting the compound (XI) or a salt thereof to elimination reaction of the protective group of amino at 7-position.

The present elimination reaction is carried out by a method by reacting the compound (XI) or a salt thereof with iminohalogenating agent and then with iminoetherifying agent, and, if necessary, subjecting the resulting compound to hydrolysis.

The present reaction is carried out according to a conventional method.

In the aforementioned reactions and/or the post-treating of the reactions of the present invention, the aforementioned geometrical isomer and/or tautomeric isomer may occasionally be transformed into the other geometrical isomer and/or tautomeric isomer and such cases are to be also included in the scope of the present invention.

In case that the object compound (I) has a free carboxy group and/or a free amino group, it may be transformed into its pharmaceutically acceptable salt as aforementioned by a conventional method.

The object compound (I) of the present invention exhibits high antimicrobial activity and inhibits the growth of a number of microorganisms including pathogenic Gram-positive and Gram-negative bacteria.

For therapeutic administration, the cephalosporin compounds according to the present invention are used in the form of pharmaceutical preparation which contain said compounds in admixture with a pharmaceutically acceptable carriers such as an organic or inorganic solid or liquid excipient suitable for oral, parenteral or external administration. The pharmaceutical preparations may be in solid form such as capsule, tablet, dragee, ointment or suppository, or in liquid form such as solution, suspension, or emulsion. If desired, there may be included in the above preparations auxiliary substances, stabilizing agents, wetting or emulsifying agents, buffers and other commonly used additives.

While the dosage of the compounds may vary from and also depend upon the age and condition of the patient, an average single dose of 50 mg., 100 mg., 250 mg., and 500 mg. of the compounds according to the present invention has proved to be effective for treating of infectious diseases caused by a number of pathogenic bacteria. In general amounts, daily dose between 1 mg/body and 1000 mg/body or even more may be administered.

Now in order to show the utility of the object compounds (I), test data on anti-microbial activity of representative compounds of the present invention are shown below.

*Test method*

One loopfool of an overnight culture of each test strain in Trypticase-soy broth ($10^8$ viable cells per ml.) was streaked on heart infusion agar (HI-agar) containing graded concentrations of antibiotics, and the minimal inhibitory concentration (MIC) was expressed in terms of $\mu$g/ml after incubation at 37°C for 20 hours.

*Test compound*

(1) 7-[2-Ethoxyimino-2-(5-amino-1,2,4-thiadiazol-3-yl)acetamido]-3-(1-pyridiniomethyl)-3-cephem-4-carboxylate (syn isomer)

(2) 7-[2-Allyloxyimino-2-(5-amino-1,2,4-thiadiazol-3-yl)acetamido]-3-(1-pyridiniomethyl)-3-cephem-4-carboxylate (syn isomer)

(3) 7-[2-(2-Propynyloxyimino)-2-(5-amino-1,2,4-thiadiazol-3-yl)acetamido]-3-(1-pyridiniomethyl)-3-cephem-4-carboxylate (syn isomer)

(4) 7-[2-Cyclopentyloxyimino-2-(5-amino-1,2,4-thiadiazol-3-yl)acetamido]-3-(1-pyridiniomethyl)-3-cephem-4-carboxylate (syn isomer)

(5) 7-[2-(2-Cyclopenten-1-yloxyimino)-2-(5-amino-1,2,4-thiadiazol-3-yl)acetamido]-3-(1-pyridiniomethyl)-3-cephem-4-carboxylate (syn isomer)

(6) 7-[2-Methoxyimino-2-(5-amino-1,2,4-thiadiazol-3-yl)acetamido]-3-(1-pyridiniomethyl)-3-cephem-4-carboxylate (syn isomer)

(7) 7-[2-Isopropoxyimino-2-(5-amino-1,2,4-thiadiazol-3-yl)acetamido]-3-(2-methyl-5-oxo-6-hydroxy-2,5-dihydro-1,2,4-triazin-3-yl)thiomethyl-3-cephem-4-carboxylic acid (syn isomer).

(8) 7-[2-Ethoxyimino-2-(5-amino-1,2,4-thiadiazol-3-yl)acetamido]-3-(4-carbamoyl-1-pyridiniomethyl)-3-cephem-4-carboxylate (syn isomer)

TEST RESULTS

| Test Bacteria | Test Compound M.I.C. (μg/ml) | | | | | | | |
|---|---|---|---|---|---|---|---|---|
| | (1) | (2) | (3) | (4) | (5) | (6) | (7) | (8) |
| E. coli NIHJ JC–2 | 0.10 | 0.10 | 0.20 | 0.78 | 1.56 | 0.39 | 0.39 | 0.05 |
| Kl. pneumoniae 12 | 0.10 | 0.78 | 0.39 | 1.56 | 1.56 | 0.20 | 0.39 | 0.20 |
| Pr. vulgaris 2 | 0.10 | 0.39 | 0.20 | 3.13 | 3.13 | 0.20 | < 0.025 | 0.20 |
| Ps. aeruginosa NCTC–10490 | 1.56 | 1.56 | 1.56 | 6.25 | 6.25 | 1.56 | 1.56 | 3.13 |
| B. subtilis ATCC 6633 | 0.78 | 0.78 | 0.78 | 0.20 | 0.39 | 0.78 | 3.13 | 0.78 |

Regarding the nomenclature of the compounds of the present invention (3-pyridiniummethyl compound), there exists some nomenclatures. For example, the following compound (A) is named as 7-[2 - methoxyimino - 2 - (5 - amino - 1,2,4 - thiadiazol - 3 - yl)acetamido] - 3 - (1 - pyridiniomethyl) - 3 - cephem - 4 - carboxylate (syn isomer) or N - [7 - {2 - methoxyimino - 2 - (5 - amino - 1,2,4 - thiadiazol - 3 - yl)acetamido} - 3 - cephem - 3 - ylmethyl]pyridinium - 4 - carboxylate (syn isomer). Further, hydrochloric acid salt of the compound (B) is named as 1 - [(7 - amino - 4 - carboxy - 3 - cephem - 3 - yl)methyl]pyridinium chloride or N - [7 - amino - 3 - cephem - 3 - ylmethyl]pyridinium - 4 - carboxylate hydrochloride.

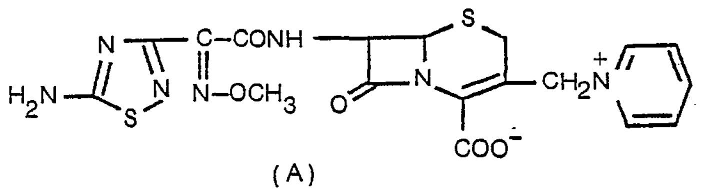

(A)

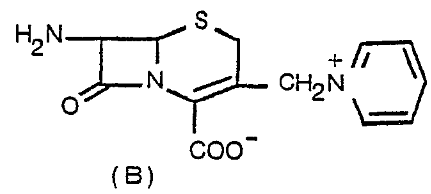

(B)

The other compounds in the present specification and claims are similarly named and they are all included in the scope of the present invention.

The following Preparations and Examples are given for the purpose of illustrating the present invention.

Preparation 1

Preparation of Methyl 5-amino-1,2,4-thiadiazole-3-carboxylate.

To a solution of 1-ethoxycarbonylformamidine hydrobromide (16.6 g.) in absolute methanol (84 ml) was added a solution of sodium (1.93 g) in absolute methanol (42 ml) at 0°C. To the mixture were added alternately bromine (12.8 g) and a solution of sodium (1.93 g) in absolute methanol (42 ml) at 0°C and then to the suspension was added potassium thiocyanate (8.1 g) in absolute methanol (100 ml). The reaction mixture was stirred for an hour at 0°C and for an additional 6 hours at ambient temperature. The mixture was filtered through cellulose powder and the filtrate was evaporated to dryness. The residue was dissolved in a mixture of ethyl acetate and water, and then the ethyl acetate layer was separated and dried over anhydrous magnesium sulfate. The solvent was evaporated and the residue was triturated with diethyl ether to give the title compound (9.0 g), m.p. 202 to 205°C.

IR (Nujol):

3400, 3250, 3100, 1710, 1610, 1540 $cm^{-1}$

NMR ($d_6$-DMSO) $\delta$:

3.85 (3H, s), 8.25 (2H, s)

Preparation 2

Preparation of Methyl 5-formamido-1,2,4-thiadiazole-3-carboxylate.

To a mixture of formic acid (33 g) and acetic anhydride (22 g) was added methyl 5-amino-1,2,4-thiadiazole-3-carboxylate (6.2 g), and then the mixture was stirred for 2 days at ambient temperature. The reaction mixture was concentrated under reduced pressure and the residue was triturated with a mixture of diethyl ether and n-hexane to give the title compound (7.2 g), m.p. 210 to 215°C.

IR (Nujol):

3100, 1720, 1680 $cm^{-1}$

NMR ($d_6$-DMSO) $\delta$:

3.90 (3H, s), 8.85 (1H, s)

Preparation 3

Preparation of 5-Formamido-3-(2-methylthio-2-methylsulfinylacetyl)-1,2,4-thiadiazole.

To a mixture of methyl 5-formamido-1,2,4-thiadiazole-3-carboxylate (9.2 g) and methyl methylthiomethyl sulfoxide (6.1 g) in N,N-dimethylformamide (100 ml) was added 50% sodium hydride (7.1 g) with cooling in an ice-bath. The mixture was stirred for an hour at ambient temperature and for an additional one hour at 40°C. After cooling to ambient temperature, methylene chloride (300 ml) was added to the reaction mixture, and the resulting precipitates were collected by filtration and washed with methylene chloride. The precipitates were added to a stirred mixture of hydrochloric acid (14.7 ml), ice-water (200 ml) and methylene chloride (200 ml). An insoluble material was filtered off and the methylene chloride layer was separated from the filtrate. The solution was dried over anhydrous magnesium sulfate, evaporated and the residue was triturated with diethyl ether to give the title compound (4.5 g), m.p. 130 to 132°C.

IR (Nujol):

3100, 1680, 1670 $cm^{-1}$

NMR ($d_6$-DMSO) $\delta$:

2.22, 2.28 } (3H, 2s)

2.68 }
2.85 } (2H, 2s)

5.70 }
5.80 } (1H, 2s)

8.86 (1H, s)

Preparation 4

Preparation of S-methyl (5-formamido-1,2,4-thiadiazol-3-yl)thioglyoxylate.

A mixture of 5-formamido-3-(2-methylthio-2-methylsulfinylacetyl)-1,2,4-thiadiazole (0.85 g) and sodium periodate (0.2 g) in glacial acetic acid (10 ml) was stirred for 45 minutes at 70°C. The reaction mixture was evaporated and the residue was dissolved in a mixture of ethyl acetate and water. The mixture was adjusted to pH 7 with an aqueous solution of sodium bicarbonate and treated with an aqueous solution of sodium thiosulfate. The organic layer was separated, dried over anhydrous magnesium sulfate and evaporated to dryness. The residue was triturated with a mixture of diethyl ether and petroleum ether to give the title compound (280 mg), m.p. 186 to 187°C.

IR (Nujol):
3100, 1680, 1660 $cm^{-1}$

NMR ($d_6$-DMSO) $\delta$:
2.55 (3H, s), 8.95 (1H, s)

Preparation 5

Preparation of 2-Methoxyimino-2-(5-formamide-1,2,4-thiadiazol-3-yl)acetic acid (syn isomer).

A mixture of S-methyl (5-formamido-1,2,4-thiadiazol-3-yl)thioglyoxylate (231 mg) in methanol (2 ml) and 1N-aqueous solution of potassium hydroxide (3.5 ml) was stirred for an hour at ambient temperature. The mixture was adjusted to pH 7.6 with 1N hydrochloric acid, followed by an addition of O-methylhydroxylamine hydrochloride (90 mg) and stirring for 30 minutes at ambient temperature.

The reaction mixture was neutralized with an aqueous solution of sodium bicarbonate and concentrated to remove methanol. The concentrated aqueous solution was adjusted to pH 4 with hydrochloric acid and washed with ethyl acetate. The aqueous layer was adjusted to pH 1 with hydrochloric acid, saturated with sodium chloride and extracted with ethyl acetate. The extract was evaporated to dryness and the residue was triturated with diethyl ether, collected by filtration and then dried to give the title compound (80 mg), m.p. 185 to 186°C.

IR (Nujol):
3150, 1720, 1690 $cm^{-1}$

NMR ($d_6$-DMSO) $\delta$:
3.98 (3H, s), 8.84 (1H, s)

Preparation 6

Preparation of 2-Methoxyimino-2-(5-formamido-1,2,4-thiadiazol-3-yl)acetic acid (syn isomer)

A mixture of 5-formamido-3-(2-methylthio-2-methylsulfinylacetyl)-1,2,4-thiadiazole (3.2 g) and sodium periodate (0.8 g) in glacial acetic acid (32 ml) was stirred for 45 minutes at 70°C. The resulting mixture was evaporated and the residue was washed with n-hexane and then thereto were added methanol (20 ml) and 1N aqueous solution of potassium hydroxide (40 ml). The solution was stirred for an hour at ambient temperature. The reaction mixture was adjusted to pH 8 with 1N hydrochloric acid, followed by an addition of O-methylhydroxylamine hydrochloride (0.96 g) and stirring for an hour at ambient temperature. The reaction mixture was neutralized with an aqueous solution of sodium bicarbonate and concentrated to remove methanol. The resulting aqueous solution was washed with ethyl acetate, adjusted to pH 1 with 10% hydrochloric acid, saturated with sodium chloride and extracted with ethyl acetate. The extract was dried over anhydrous magnesium sulfate, evaporated, and the residue was triturated with diisopropyl ether to give the title compound (1.02 g), m.p. 185 to 186°C.

Preparation 7

Preparation of 2-Methoxyimino-2-(5-amino-1,2,4-thiadiazol-3-yl)acetic acid (syn isomer).

A solution of 2-methoxyimino-2-(5-formamido-1,2,4-thiadiazol-3-yl)acetic acid (syn isomer) (1.4 g) in 1N aqueous solution of sodium hydroxide (19.1 ml) was heated at 50° to 55°C for an hour. To the solution was added conc.hydrochloric acid (1.9 ml) under cooling in an ice-bath. The mixture was saturated with sodium chloride and extracted with ethyl acetate. The extract was dried over anhydrous magnesium sulfate and evaporated to dryness. The residue was triturated with diethyl ether to give the title compound (0.9 g), m.p. 180 to 182°C (dec.).

IR (Nujol):
3450, 3250, 3100, 1715, 1610, 1530 $cm^{-1}$

NMR ($d_6$-DMSO) $\delta$:
3.90 (3H, s), 8.10 (3H, broad s)

Preparation 8

A mixture of 5-formamido-3-(2-methylthio-2-methylsulfinylacetyl)-1,2,4-thiadiazole (10 g) and sodium periodate (2.0 g) in glacial acetic acid (50 ml) was stirred for 50 minutes at 70°C. The solvent was evaporated and the residue was washed with n-hexane. To the residue was added 1N aqueous solution of sodium hydroxide (160 ml) and the mixture was stirred for an hour at ambient temperature. To the reaction mixture was added O-ethylhydroxylamine hydrochloride (3.5 g) and the solution was adjusted to pH 3 to 4 with 10% hydrochloric acid and then stirred for an hour at ambient temperature. After an insoluble material was filtered off, the filtrate was washed with ethyl acetate, adjusted to pH 1 with 10% hydrochloric acid and extracted with ethyl acetate. The extract was dried over magnesium sulfate and evaporated to dryness. The residue was triturated with a mixture of diethyl ether and diisopropyl ether to give 2-ethoxyimino-2-(5-formamido-1,2,4-thiadiazol-3-yl)acetic acid (syn isomer) (4.5 g), m.p. 165 to 168°C (dec.).

IR (Nujol):
3450, 3170, 3050, 1730, 1690, 1595, 1565 $cm^{-1}$

NMR ($d_6$-DMSO) $\delta$:
1.30 (3H, t, J=7Hz), 4.30 (2H, q, J=7Hz), 8.87 (1H, s).

Preparation 9

The following compounds were obtained according to a similar manner to that of Preparation 8.

(1) 2-Propoxyimino-2-(5-formamido-1,2,4-thiadiazol-3-yl)acetic acid (syn isomer), m.p. 168 to 170°C (dec.).

IR (Nujol):
3250, 3140, 1720, 1690, 1590, 1530 $cm^{-1}$

NMR ($d_6$-DMSO) $\delta$:
0.90 (3H, t, J=6Hz), 1.4—1.9 (2H, m), 4.17 (2H, t, J=6Hz), 8.85 (1H, s).

(2) 2-Isopropoxyimino-2-(5-formamido-1,2,4-thiadiazol-3-yl)acetic acid (syn isomer), m.p. 180 to 182°C (dec.).

IR (Nujol):
3230, 1720, 1690, 1590, 1530 $cm^{-1}$

NMR ($d_6$-DMSO) $\delta$:
1.25 (6H, d, J=6Hz), 4.2—4.7 (1H, m), 8.85 (1H, s)

Preparation 10

A mixture of 2-ethoxyimino-2-(5-formamido-1,2,4-thiadiazol-3-yl)acetic acid (syn isomer) (4.4 g) and 1N aqueous solution of sodium hydroxide (54 ml) was stirred for 2 hours at 50 to 55°C. The mixture was cooled in an ice bath, acidified with hydrochloric acid (5.4 ml) and extracted with ethyl acetate. The extract was dried over magnesium sulfate and evaporated to dryness. The residue was triturated with diethyl ether to give 2-ethoxyimino-2-(5-amino-1,2,4-thiadiazol-3-yl)acetic acid (syn isomer) (2.92 g), m.p. 168 to 170°C (dec.).

IR (Nujol):
3450, 3370, 3250, 3150, 1665, 1610, 1530 $cm^{-1}$

NMR ($d_6$-DMSO) $\delta$:
1.22 (3H, t, J=7Hz), 4.17 (2H, q, J=7Hz), 8.17 (2H, broad s)

Preparation 11

The following compounds were obtained according to a similar manner to that of Preparation 10.

(1) 2-Propoxyimino-2-(5-amino-1,2,4-thiadiazol-3-yl)acetic acid (syn isomer), m.p. 100 to 103°C (dec.).

IR (Nujol):
3620, 3520, 3330, 3120, 2600, 2500, 1720, 1620, 1550 $cm^{-1}$

NMR ($d_6$-DMSO) $\delta$:
1.00 (3H, t, J=6Hz), 1.3—2.0 (2H, m), 4.13 (2H, t, J=6Hz), 8.17 (2H, broad, s).

(2) 2-Isopropoxyimino-2-(5-amino-1,2,4-thiadiazol-3-yl)acetic acid (syn isomer), m.p. 152 to 155°C (dec.).

IR (Nujol):
3450, 3300, 3200, 1730, 1620, 1530 $cm^{-1}$

NMR ($d_6$-DMSO) $\delta$:
1.22 (6H, d, J=6Hz), 4.1—4.6 (1H, m), 8.20 (2H, broad s).

Preparation 12

(1) A mixture of N-hydroxyphthalimide (8.15 g), triethylamine (5.05 g), N,N-dimethylformamide (60

ml) and 1-bromo-2-cyclohexene (8.05 g) was stirred for 3.5 hours at room temperature. The reaction mixture was poured into water (300 ml). The precipitated crystals were collected by filtration, washed successively with water and n-hexane and then dried to give N-(2-cyclohexen-1-yloxy)phthalimide (9.8 g). m.p. 87°C.

IR (Nujol):
1770, 1720, 1610 $cm^{-1}$
NMR ($d_6$-DMSO) $\delta$:
1.50—2.17 (6H, m), 4.60—4.77 (1H, m), 5.73—6.27 (2H, m), 7.90 (4H, s)

(2) A mixture of N-hydroxyphthalimide (58.2 g), 1-chloro-2-cyclopentene (36.9 g), triethylamine (53.9 g) in acetonitrile (370 ml) was treated in similar manner to that of Preparation 12-(1) to give N-(2-cyclopenten-1-yloxy)phthalimide (56.5 g).

IR (Nujol):
1780, 1730, 1610 $cm^{-1}$
NMR ($d_6$-DMSO) $\delta$:
7.92 (4H, s), 6.28 (1H, m), 6.00 (1H, m), 5.42 (1H, m), 2.9—1.98 (4H, m).

Preparation 13

(1) A mixture of N-(2-cyclopenten-1-yloxy)phthalimide (22.9 g) and hydrazine hydrate (4.75 g) in ethanol (115 ml) was refluxed for 5 minutes. The reaction mixture was filtered. The filtrate containing (2-cyclopenten-1-yl)oxyamine was added to a solution of sodium 2-(5-formamido-1,2,4-thiadiazol-3-yl)glyoxylate (22.4 g) in water. The mixture was adjusted to pH 2 with 10% hydrochloric acid, stirred for 2 hours and then concentrated. The concentrate was adjusted to pH 1 with 10% hydrochloric acid. The precipitates were collected by filtration and dried to give 2-(2-cyclopenten-1-yl)oxyimino-2-(5-formamido-1,2,4-thiadiazol-3-yl)acetic acid (syn isomer) (20.0 g), m.p. 150°C (dec.).

IR (Nujol):
3400, 3100, 1720, 1690, 1540 $cm^{-1}$
NMR ($d_6$-DMSO) $\delta$:
1.80—2.50 (4H, m), 5.30—5.50 (1H, m), 5.83—6.30 (2H, m), 8.90 (1H, s).

(2) A mixture of N-(2-cyclohexen-1-yloxy)phthalimide (7.29 g), hydrazine hydrate (1.5 g) in ethanol (40 ml) was refluxed for 5 minutes. The reaction mixture was cooled and filtered to give the filtrate containing (2-cyclohexen-1-yl)oxyamine (filtrate A). On the other hand, a mixture of S-methyl 2-(5-formamido-1,2,4-thiadiazol-3-yl)thioglyoxylate (6.93 g) in 1N-aqueous solution of sodium hydroxide (90 ml) was stirred for 30 minutes at room temperature. The reaction mixture containing sodium 2-(5-formamido-1,2,4-thiadiazol-3-yl)glyoxylate was adjusted to pH 7 with 10% hydrochloric acid and thereto was added the filtrate A and then the pH was adjusted to 3 with 10% hydrochloric acid. The mixture was stirred for 3 hours at room temperature. The reaction mixture was concentrated and to the concentrate was added ethyl acetate. The mixture was adjusted to pH 1 with 10% hydrochloric acid. The precipitates were collected by filtration to give 2-(2-cyclohexen-1-yl)oxyimino-2-(5-formamido-1,2,4-thiadiazol-3-yl)-acetic acid (syn isomer) (2.5 g). On the other hand, the ethyl acetate layer was separated from the filtrate and evaporated. The residue was triturated with diethyl ether to give the same object compound (1.5 g). Total yield: 4.0 g, m.p. 190 to 192°C (dec.).

IR (Nujol):
3550, 3400, 3200, 2500, 1690, 1590, 1540 $cm^{-1}$
NMR ($d_6$-DMSO) $\delta$:
1.5—2.3 (6H, m), 4.73—5.0 (1H, m), 5.76—6.23 (2H, m), 8.97 (1H, s), 13.60 (1H, broad s).

(3) To a solution of sodium hydroxide (11.2 g) in water (140 ml) was added S-methyl 2-(5-formamido-1,2,4-thiadiazol-3-yl)thioglyoxylate (27 g) at 10°C and the mixture was stirred for 30 minutes at 20°C. The reaction mixture containing sodium 2-(5-formamido-1,2,4-thiadiazol-3-yl)-glyoxylate was cooled, adjusted to pH 7 with 10% hydrochloric acid and thereto was added a solution of cyclopentyloxyamine (15.3 g) in ethanol (150 ml). The mixture was adjusted to pH 3 with 10% hydrochloric acid, and stirred for 1.5 hours. The reaction mixture was adjusted to pH 7 with an aqueous solution of sodium bicarbonate and then evaporated to remove ethanol. The residue was washed with ethyl acetate. To the aqueous layer was added ethyl acetate and the mixture was adjusted to pH 1 with 10% hydrochloric acid. The precipitates were collected by filtration to give 2-cyclopentyloxyimino-2-(5-formamido-1,2,4-thiadiazol-3-yl)acetic acid (syn isomer) (3.99 g). The filtrate was extracted with ethyl acetate and the extract was dried over magnesium sulfate and then concentrated. The precipitates were collected by filtration and washed with diethyl ether to give the same object compound (8.1 g). Total yield: 12.09 g, m.p. 180 to 185°C (dec.).

IR (Nujol):
3130, 3040, 2680, 2610, 2520, 1720, 1690, 1660, 1600, 1550 $cm^{-1}$

NMR ($d_6$-DMSO) δ:

1.33—2.10 (8H, m), 4.67—5.0 (1H, m), 8.88 (1H, s), 13.50 (1H, s).

Preparation 14

A mixture of 2-(2-cyclopenten-1-yl)oxyimino-2-(5-formamido-1,2,4-thiadiazol-3-yl)acetic acid (syn isomer) (20.0 g) and 1N aqueous solution of sodium hydroxide (200 ml) was stirred for an hour at 50 to 55°C. The reaction mixture was cooled, adjusted to pH 7 with 10% hydrochloric acid and thereto was added ethyl acetate. The mixture was adjusted to pH 1 with 10% hydrochloric acid and extracted with ethyl acetate. The extract was washed with a saturated aqueous solution of sodium chloride, dried over magnesium sulfate and evaporated. The residue was pulverized with diisopropyl ether to give 2-(2-cyclopenten-1-yl)oxyimino-2-(5-amino-1,2,4-thiadiazol-3-yl)acetic acid (syn isomer), m.p. 150°C (dec.).

IR (Nujol):

3300, 3150, 1710, 1620, 1520 $cm^{-1}$

NMR ($d_6$-DMSO) δ:

1.80—2.50 (4H, m), 5.30—5.50 (1H, m), 5.83—6.30 (2H, m), 8.20 (2H, s)

Preparation 15

The following compounds were obtained according to a similar manner to that of Preparation 14.

(1) 2-(2-Cyclohexen-1-yl)oxyimino-2-(5-amino-1,2,4-thiadiazol-3-yl)acetic acid (syn isomer), m.p. 173°C.

IR (Nujol):

3400, 3300, 3200, 1720, 1620, 1600, 1520 $cm^{-1}$

NMR ($d_6$-DMSO) δ:

1.50—2.17 (6H, m), 4.53—4.83 (1H, m), 5.57—6.13 (2H, m), 8.18 (2H, s).

(2) 2-Cyclopentyloxyimino-2-(5-amino-1,2,4-thiadiazol-3-yl)acetic acid (syn isomer), m.p. 160 to 165°C (dec.).

IR (Nujol):

3470, 3290, 3200, 2400, 1715, 1615, 1600, 1520 $cm^{-1}$

NMR ($d_6$-DMSO) δ:

1.17—2.10 (8H, m), 4.60—4.97 (1H, m), 8.22 (2H, s)

Preparation 16

A mixture of 5-formamido-3-(2-methylthio-2-methylsulfinylacetyl)-1,2,4-thiadiazole (10 g) and sodium periodate (2.0 g) in glacial acetic acid (50 ml) was stirred for 50 minutes at 70°C. The solvent was evaporated and the residue was washed with n-hexane. To the residue was added 1N aqueous solution of sodium hydroxide (160 ml) and the mixture was stirred for an hour at ambient temperature. To the reaction mixture was added O-allylhydroxylamine hydrochloride (4.31 g) and the solution was adjusted to pH 3 to 4 with 10% hydrochloric acid and then stirred for an hour at ambient temperature. After an insoluble material was filtered off, the filtrate was washed with ethyl acetate, adjusted to pH 1 with 10% hydrochloric acid and extracted with ethyl acetate. The extract was dried over magnesium sulfate and evaporated to dryness. The residue was triturated with a mixture of diethyl ether and diisopropyl ether to give 2-allyloxyimino-2-(5-formamido-1,2,4-thiadiazol-3-yl)acetic acid (syn isomer) (5.6 g), m.p. 169 to 172°C (dec.).

IR (Nujol):

3130, 2500, 1720, 1690, 1590, 1550 $cm^{-1}$

NMR ($d_6$-DMSO) δ:

4.79 (2H, d, J=6Hz), 5.1—5.6 (2H, m), 5.8—6.4 (1H, m), 8.88 (1H, s).

Preparation 17

The following compounds were obtained according to a similar manner to that of Preparation 16.

(1) 2-(2-Propynyloxyimino)-2-(5-formamido-1,2,4-thiadiazol-3-yl)acetic acid (syn isomer), m.p. 150 to 155°C (dec.).

IR (Nujol):

3570, 3360, 3260, 3120, 1720, 1670, 1550, 1530 $cm^{-1}$

NMR ($d_6$-DMSO) δ:

3.55 (1H, t, J=2Hz), 4.88 (2H, d, J=2Hz), 8.85 (1H, s).

(2) 2-Hydroxyimino-2-(5-formamido-1,2,4-thiadiazol-3-yl)acetic acid (syn isomer), m.p. 240 to 241°C (dec.).

IR (Nujol):

3550, 3460, 1665, 1635, 1560 $cm^{-1}$

Preparation 18

A solution of S-methyl (5-formamido-1,2,4-thiadiazol-3-yl)thioglyoxylate (6.64 g) in 1N aqueous solution of sodium hydroxide (80 ml) was adjusted to pH 8.5 with 10% hydrochloric acid and stirred for 30 minutes at ambient temperature. On the other hand, a mixture of N-(2,2,2-trifluoroethoxy)-phthalimide (8.78 g) and hydrazine hydrate (1.7 g) in ethanol (40 ml) was refluxed for 5 minutes and then cooled in an ice bath. A resulting precipitates were filtered off and washed with ethanol. The filtrate and the washings were combined and the combined solution containing O-(2,2,2-trifluoroethyl)hydroxylamine was added to the above aqueous solution. The mixture was adjusted to pH 3 to 4 with 10% hydrochloric acid and stirred for 1.5 hours at ambient temperature. The solution was neutralized with an aqueous solution of sodium bicarbonate, concentrated to half volume in vacuo and washed with ethyl acetate. The aqueous solution was acidified with 10% hydrochloric acid and extracted with ethyl acetate. The extract was dried over magnesium sulfate, evaporated to dryness and the residue was triturated with diisopropyl ether to give 2-(2,2,2-trifluoroethoxyimino)-2-(5-formamido-1,2,4-thiadiazol-3-yl)acetic acid (syn isomer) (2.46 g), m.p. 180 to 185°C (dec.).

NMR ($d_6$-DMSO) $\delta$:

4.80 and 5.07 (2H, ABq, J=9Hz), 8.85 (1H, s).

Preparation 19

The following compound was obtained according to a similar manner to that of Preparation 18.

2-Methylthiomethoxyimino-2-(5-formamido-1,2,4-thiadiazol-3-yl)acetic acid (syn isomer), m.p. 146 to 148°C (dec.).

IR (Nujol):

3300, 2600, 2550, 1730, 1705, 1680, 1600, 1530 $cm^{-1}$

NMR ($d_6$-DMSO) $\delta$:

2.23 (3H, s), 5.40 (2H, s), 8.87 (1H, s).

Preparation 20

A mixture of S-methyl(5-formamido-1,2,4-thiadiazol-3-yl)thioglyoxylate (6 g) and an aqueous solution (50 ml) of sodium hydroxide (4.2 g) was stirred for an hour at 50 to 55°C. The mixture was cooled to ambient temperature and adjusted to pH 7 with 10% hydrochloric acid. On the other hand, a mixture of N-(ethoxycarbonylmethoxy)phthalimide (12.9 g) and hydrazine hydrate (2.08 g) in ethanol (60 ml) was refluxed for 5 minutes and cooled in an ice bath. A resulting precipitate was filtererd off and washed with ethanol. The filtrate and the washings were combined and the combined solution containing O-(ethoxycarbonylmethyl)-hydroxylamine was added to the above aqueous solution. The mixture was adjusted to pH 3 to 4 with 10% hydrochloric acid and stirred for 1.5 hours at ambient temperature. The solution was neutralized with an aqueous solution of sodium bicarbonate, concentrated to half volume in vacuo and washed with ethyl acetate. The aqueous solution was acidified with 10% hydrochloric acid and extracted with ethyl acetate. The extract was dried over magnesium sulfate, evaporated to dryness and the residue was triturated with diisopropyl ether to give 2-ethoxycarbonylmethoxyimino-2-(5-amino-1-2,4-thiadiazol-3-yl)acetic acid (syn isomer) (1.8 g), m.p. 135 to 140°C (dec.).

IR (Nujol):

3500, 3330, 3210, 2670, 2550, 1740, 1610, 1540 $cm^{-1}$

NMR ($d_6$-DMSO) $\delta$:

1.24 (3H, t, J=7Hz), 4.14 (2H, q, J=7Hz), 4.80 (2H, s), 8.15 (2H, broad s).

Preparation 21

The following compound was obtained according to a similar manner to that of Preparation 20.

2-(1-Ethoxycarbonyl-1-methylethoxyimino)-2-(5-amino-1,2,4-thiadiazol-3-yl)acetic acid (syn isomer), m.p. 165 to 168°C (dec.).

IR (Nujol):

3450, 3350, 3240, 1750, 1730, 1630, 1530 $cm^{-1}$

NMR ($d_6$-DMSO) $\delta$:

1.18 (3H, t, J=7Hz), 1.50 (6H, s), 4.15 (2H, q, J=7Hz), 8.23 (2H, broad s).

Preparation 22

The following compounds were obtained according to a similar manner to that of Preparation 14.

(1) 2-Allyloxyimino-2-(5-amino-1,2,4-thiadiazol-3-yl)acetic acid (syn isomer), m.p. 93 to 95°C (dec.).

IR (Nujol):

3430, 3100, 1710, 1615, 1525 $cm^{-1}$

NMR ($d_6$-DMSO) $\delta$:

4.72 (2H, d, J=6Hz), 5.1—5.5 (2H, m), 5.7—6.3 (1H, m), 8.17 (1H, broad s).

(2) 2-(2-Propynyloxyimino)-2-(5-amino-1,2,4-thiadiazol-3-yl)acetic acid (syn isomer), m.p. 155 to 157°C (dec.).

IR (Nujol):

3500, 3310, 3160, 2600, 2480, 1745, 1610, 1535 $cm^{-1}$

NMR ($d_6$-DMSO) $\delta$:

3.53 (1H, t, J=2Hz), 4.87 (2H, d, J=2Hz), 8.23 (2H, broad s).

(3) 2-(2,2,2-Trifluoroethoxyimino)-2-(5-amino-1,2,4-thiadiazol-3-yl)acetic acid (syn isomer), m.p. 140 to 143°C (dec.).

IR (Nujol):

3450, 3350, 3260, 1745, 1670, 1645, 1615, 1515 $cm^{-1}$

NMR ($d_6$-DMSO) $\delta$:

4.72 and 4.95 (2H, ABq, J=9Hz), 8.25 (2H, broad s).

(4) 2-Methylthiomethoxyimino-2-(5-amino-1,2,4-thiadiazol-3-yl)acetic acid (syn isomer), m.p. 140 to 143°C (dec.).

IR (Nujol):

3500, 3300, 3150, 2670, 2580, 1740, 1615, 1605, 1530 $cm^{-1}$

NMR ($d_6$-DMSO) $\delta$:

2.22 (3H, s), 5.33 (2H, s), 8.20 (2H, broad s).

(5) 2-Trityloxyimino-2-(5-amino-1,2,4-thiadiazol-3-yl)acetic acid (syn isomer), m.p. 173 to 174°C (dec.).

IR (Nujol):

3450, 1735, 1620, 1540 $cm^{-1}$

NMR ($d_6$-DMSO) $\delta$:

7.35 (15H, s), 8.22 (2H, s).

Preparation 23

To a mixture of 2-hydroxyimino-2-(5-formamido-1,2,4-thiadiazol-3-yl)acetic acid (syn isomer) (9.5 g) and dimethylformamide (80 ml) was added with stirring at ambient temperature trityl chloride (22.8 g), and triethylamine (4.1 g) was gradually added thereto after 3 minutes stirring. The resulting mixture was stirred for 10 minutes and ethyl acetate (250 ml) was added thereto. The mixture was washed three times with water and with a saturated aqueous solution of sodium chloride, dried over magnesium sulfate and concentrated. To the residue were added an aqueous solution of sodium bicarbonate (50 ml) and diisopropyl ether (100 ml). Precipitates were collected by filtration and the aqueous layer in the filtrate was separated. The collected precipitates were suspended in the separated aqueous layer and ethyl acetate was added thereto. The mixture was adjusted to pH 2 with 10% hydrochloric acid and extracted with ethyl acetate. The extract was dried over magnesium sulfate and concentrated in vacuo. The residue was washed with hexane to give 2-trityloxyimino-2-(5-formamido-1,2,4-thiadiazol-3-yl)acetic acid (syn isomer) (17.1 g), m.p. 175 to 176°C (dec.).

IR (Nujol):

3180, 3070, 1700, 1600, 1540 $cm^{-1}$

NMR ($d_6$-DMSO) $\delta$:

7.35 (15H, s), 8.83 (1H, s), 13.52 (1H, broad s).

Preparation 24

The following compounds were obtained according to similar manner to that of Preparation 12-(1).

(1) N-(1-t-Butoxycarbonylethoxy)phthalimide, m.p. 80 to 82°C.

NMR ($d_6$-DMSO, $\delta$):

1.42 (9H, s), 1.48 (3H, d, J=7Hz), 4.72 (1H, q, J=7Hz), 7.86 (4H, s).

(2) N-(1-t-Butoxycarbonyl-1-methylethoxy)phthalimide, m.p. 96 to 100°C.

NMR ($d_6$-DMSO) $\delta$:

1.42 (9H, s), 1.48 (6H, s), 7.87 (4H, s).

(3) N-(1-Benzoyloxycarbonylethoxy)phthalimide, m.p. 65 to 68°C.

IR (Nujol):

1790, 1740, 1450, 1210, 1190, 1110, 1080, 980, 880, 735, 700 $cm^{-1}$

(4) N-(2-Oxo-3-tetrahydrofuryloxy)phthalimide, m.p. 140 to 142°C.

IR (Nujol):

1785, 1760, 1720, 1605, 1215, 1185, 870, 695 $cm^{-1}$

Preparation 25

The following compounds were obtained according to a similar manner to that of Preparation 20.

(1) 2-(t-Butoxycarbonylmethoxyimino)-2-(5-amino-1,2,4-thiadiazol-3-yl)acetic acid (syn isomer), m.p. 150 to 155°C (dec.).

IR (Nujol):

3420, 3230, 3100, 1725, 1610, 1530 $cm^{-1}$

NMR ($d_6$-DMSO) $\delta$:

1.45 (9H, s), 4.70 (2H, s), 8.12 (2H, broad s)

(2) 2-(1-t-Butoxycarbonylethoxyimino)-2-(5-amino-1,2,4-thiadiazol-3-yl)acetic acid (syn isomer), m.p. 155 to 156°C (dec.).

IR (Nujol):

3400, 3300, 3200, 1720, 1710, 1620, 1520 $cm^{-1}$

NMR ($d_6$-DMSO) $\delta$:

1.2—1.7 (12H, m), 4.72 (1H, q, J=7Hz), 8.2 (2H, broad s).

(3) 2-(1-t-Butoxycarbonyl-1-methylethoxyimino)-2-(5-amino-1,2,4-thiadiazol-3-yl)acetic acid (syn isomer), m.p. 180 to 181°C (dec.).

IR (Nujol):

3400, 3300, 3200, 1745, 1715, 1630, 1530 $cm^{-1}$

NMR ($d_6$-DMSO) $\delta$:

1.38 (9H, s), 1.43 (6H, s), 8.15 (2H, broad s).

(4) 2-(1-Benzyloxycarbonylethoxyimino)-2-(5-amino-1,2,4-thiadiazol-3-yl)acetic acid (syn isomer), m.p. 129 to 133°C (dec.).

IR (Nujol):

3300, 3200, 1720, 1620, 1530 $cm^{-1}$

NMR ($d_6$-DMSO) $\delta$:

1.45 (3H, d, J=6Hz), 4.97 (1H, q, J=6Hz), 5.18 (2H, s), 7.31 (5H, s), 8.17 (2H, broad s).

Preparation 26

S-Methyl (5-formamido-1,2,4-thiadiazol-3-yl)-thioglyoxylate (64.8 g) and 1-carboxy-3-hydroxypropoxyamine, which was prepared by refluxing a mixture of N-(2-oxo-3-tetrahydrofuryloxy)-phthalimide (65.0 g), conc. hydrochloric acid (50 ml) and water (200 ml) for 1 hour, were treated according to a similar manner to that of Preparation 20 to give 2-(1-carboxy-3-hydroxypropoxyimino)-2-(5-amino-1,2,4-thiadiazol-3-yl)acetic acid (syn isomer) (33.2 g), m.p. 186 to 188°C (dec.).

IR (Nujol):

3400, 3250, 3100, 1710, 1620, 1540 $cm^{-1}$

NMR ($d_6$-DMSO) $\delta$:

1.73—2.10 (2H, m), 3.50 (2H, t, J=6Hz), 4.73 (1H, t, J=6Hz), 8.13 (2H, s).

Preparation 27

To a solution of 2-(1-carboxy-3-hydroxypropoxyimino)-2-(5-amino-1,2,4-thiadiazol-3-yl)acetic acid (syn isomer) (33.0 g) in methanol (2.8 l) were added anhydrous magnesium sulfate (120 g) and acetic anhydride (60 g). The mixture was stirred at ambient temperature for 30 minutes, filtered and the filtrate was evaporated to dryness. The residue was triturated in acetone (200 ml) and ethyl acetate (1 l) was added thereto. The mixture was stirred at ambient temperature for 1 hour and the precipitates were collected by filtration and washed with ethyl acetate.

The precipitates were dissolved in water (200 ml) and then ethyl acetate (500 ml), acetone (200 ml) and 6N hydrochloric acid (40 ml) were added thereto. An organic layer was separated out and the aqueous layer was extracted with ethyl acetate. The organic layers were combined, dried over anhydrous magnesium sulfate and evaporated to dryness. The residue was triturated in diethyl ether, filtered and washed with diisopropyl ether to give 2-(2-oxo-3-tetrahydrofuryloxyimino)-2-(5-amino-1,2,4-thiadiazol-3-yl)acetic acid (syn isomer) (26.5 g), m.p. 185—187°C (dec.).

IR (Nujol):

3400, 3300, 3200, 1775, 1730, 1640, 1605, 1535 $cm^{-1}$

NMR ($d_6$-DMSO) $\delta$:

2.27—2.70 (2H, m), 4.17—4.50 (2H, m), 5.27 (1H, t, J=8Hz), 8.22 (2H, s).

Preparation 28

The following compound was prepared according to a similar manner to that of Preparation 20.

2-Methoxycarbonylmethoxyimino-2-(5-amino-1,2,4-thiadiazol-3-yl)acetic acid (syn isomer), m.p. 190—193°C (dec.).

IR (Nujol):

3380, 3280, 3180, 1750, 1710, 1610, 1510, 1260, 1230 $cm^{-1}$

NMR ($d_6$-DMSO) $\delta$:

3.73 (3H, s), 4.87 (2H, s), 8.2 (2H, bs).

Preparation 29

The following compound was obtained according to a similar manner to that of Preparation 30.

7-[2-Cyclopentyloxyimino-2-(5-amino-1,2,4-thiadiazol-3-yl)acetamido]cephalosporanic acid (syn isomer), m.p. 140 to 145°C (dec.).

IR (Nujol):

3480, 3370, 3250, 1785, 1730, 1680, 1630, 1530 $cm^{-1}$

NMR ($d_6$-DMSO) $\delta$:

1.33—2.17 (8H, m), 2.03 (3H, s), 3.57 (2H, broad s), 4.60—4.90 (1H, m), 4.73 and 4.97 (2H, ABq, J=13Hz), 5.15 (1H, d, J=5Hz), 5.80 (1H, dd, J=5 and 8Hz), 8.10 (2H, broad s), 9.47 (1H, d, J=8Hz).

Preparation 30

To a cold solution of phosphorus pentachloride (10.4 g) in methylene chloride (250 ml) was added 2-cyclopentyloxyimino-2-(5-amino-1,2,4-thiadiazol-3-yl)acetic acid (syn isomer) (12.8 g) at —18°C and the mixture was stirred for 15 minutes at —13 to —10°C. On the other hand, a mixture of 7-amino-3-acetoacetoxymethyl-3-cephem-4-carboxylic acid (15.7 g) and trimethylsilylacetamide (50 g) in methylene chloride (250 ml) was warmed to make a clear solution and cooled to —10°C. The cold solution was added to the above activated mixture and the mixture was stirred for 25 minutes at —10°C. The reaction mixture was poured into an aqueous solution (500 ml) of sodium bicarbonate (29.5 g) and stirred for 15 minutes at room temperature. The aqueous layer was separated out, adjusted to pH 2 with 6N hydrochloric acid and extracted with ethyl acetate. The extract was washed with water, dried over anhydrous magnesium sulfate and evaporated to dryness. The residue was triturated with diethyl ether to give a powder of 7 - [2 - cyclopentyloxyimino - 2 - (5 - amino - 1,2,4 - thiadiazol - 3 - yl)acetamido] - 3 - acetoacetoxymethyl - 3 - cephem - 4 - carboxylic acid (syn isomer) (13.5 g), mp 130 to 135°C (dec.).

IR (Nujol):

3300, 1780, 1720, 1680, 1620, 1525 $cm^{-1}$

NMR (DMSO-$d_6$, $\delta$):

1.3—2.0 (8H, m), 2.15 (3H, s), 3.52 (2H, bs), 3.60 (2H, s), 4.5—4.7 (1H, m), 4.77, 5.00 (2H, ABq, J=14Hz), 5.13 (1H, d, J=4Hz), 5.80 (1H, 2d, J=4 and 8Hz), 8.10 (2H, s), 9.50 (1H, d, J=8Hz)

Preparation 31

To a solution of 7 - [2 - (1 - t - butoxycarbonylethoxyimino) - 2 - (5 - amino - 1,2,4 - thiadiazol - 3 - yl)acetamido] - 3 - acetoacetoxymethyl - 3 - cephem - 4 - carboxylic acid (syn isomer) (5.16 g) in formic acid (50 ml) was added conc. hydrochloric acid (1.7 ml) and the mixture was stirred for 30 minutes at room temperature. The solvent was distilled off under reduced pressure and the residue was pulverized with diethyl ether and collected by filtration to give a brownish powder. The powder was dissolved in a mixture of ethyl acetate and water and adjusted to pH 7 with a saturated aqueous solution of sodium bicarbonate under stirring. The aqueous layer was separated out and ethyl acetate was added thereto. The mixture was adjusted to pH 1 with 6N hydrochloric acid and extracted with ethyl acetate. The extract was dried over anhydrous magnesium sulfate and evaporated to dryness. The residue was pulverized with diethyl ether, collected by filtration, washed with the same solvent and dried over anhydrous phosphorus pentoxide to give brownish powder of 7 - [2 - (1 - carboxyethoxyimino) - 2 - (5 - amino - 1,2,4 - thiadiazol - 3 - yl)acetamido] - 3 - acetoacetoxymethyl - 3 - cephem - 4 - carboxylic acid (syn isomer) (3.7 g), mp 95 to 100°C (dec.).

IR (Nujol):

3450, 3350, 3230, 1780, 1720, 1630, 1525 $cm^{-1}$

NMR (DMSO-$d_6$, $\delta$):

1.45 (3H, d, J=7Hz), 2.20 (3H, s), 3.58 (2H, broad s), 3.67 (2H, s), 4.65—5.30 (3H, m), 5.22 (1H, d, J=5Hz), 5.77—6.10 (1H, m), 8.23 (2H, broad s), 9.40—9.68 (1H, m)

Preparation 32

The following compounds were obtained according to similar manners to those of Preparations 30 and 31.

(1) 7-[2-(2-Cyclopenten-1-yloxyimino)-2-(5-amino-1,2,4-thiadiazol-3-yl)acetamido]-3-

acetoacetoxymethyl-3-cephem-4-carboxylic acid (syn isomer), mp 135 to 140°C (dec.).

IR (Nujol):

3400, 3300, 3200, 1775, 1735, 1710, 1675, 1620, 1525 $cm^{-1}$

NMR ($DMSO-d_6$, $\delta$):

1.93—2.47 (4H, m), 2.18 (3H, s), 3.55 (2H, bs), 3.65 (2H, s), 4.80, 5.07 (2H, ABq, J=13Hz), 5.13 (1H, d, J=5Hz), 5.23—5.53 (1H, m), 5.70—6.23 (3H, m), 8.12 (2H, bs), 9.50 (1H, d, J=8Hz)

(2) 7-[2-Carboxymethoxyimino-2-(5-amino-1,2,4-thiadiazol-3-yl)acetamido]-3-acetoacetoxymethyl-3-cephem-4-carboxylic acid (syn isomer), mp 95 to 100°C (dec.).

IR (Nujol):

3400, 3290, 3190, 1770, 1720, 1615, 1520 $cm^{-1}$

NMR ($DMSO-d_6$, $\delta$):

2.17 (3H, s), 3.53 (2H, bs), 3.63 (2H, s), 4.67 (2H, s), 4.80, 5.07 (2H, ABq, J=13Hz), 5.15 (1H, d, J=5Hz), 5.87 (1H, 2d, J=5 and 8Hz), 8.15 (2H, bs), 9.53 (1H, d, J=8Hz)

(3) 7-[2-t-Butoxycarbonylmethoxyimino-2-(5-amino-1,2,4-thiadiazol-3-yl)acetamido]-3-acetoacetoxymethyl-3-cephem-4-carboxylic acid (syn isomer), mp 105 to 110°C (dec.).

IR (Nujol):

3350, 3250, 1780, 1720, 1620, 1525 $cm^{-1}$

NMR ($DMSO-d_6$, $\delta$):

1.43 (9H, s), 2.17 (3H, s), 3.53 (2H, bs), 3.63 (2H, s), 4.63 (2H, s), 4.82, 5.05 (2H, ABq, J=13Hz), 5.15 (1H, d, J=5Hz), 5.85 (1H, 2d, J=5 and 8Hz), 8.15 (2H, bs), 9.53 (1H, d, J=8Hz)

(4) 7-[2-(1-t-Butoxycarbonylethoxyimino)-2-(5-amino-1,2,4-thiadiazol-3-yl)acetamido]-3-acetoacetoxymethyl-3-cephem-4-carboxylic acid (syn isomer), brownish powder, mp 110 to 115°C (dec.).

IR (Nujol):

3400, 3300, 3200, 1780, 1720, 1620, 1525 $cm^{-1}$

NMR ($DMSO-d_6$, $\delta$):

1.40 (3H, d, J=7Hz), 1.42 (9H, s), 2.17 (3H, s), 3.57 (2H, bs), 3.63 (2H, s), 4.58—5.22 (3H, m), 5.17 (1H, d, J=5Hz), 5.73—5.97 (1H, m), 8.10 (2H, bs), 9.33—9.57 (1H, m)

(5) 7-[2-(1-Methyl-1-carboxyethoxyimino)-2-(5-amino-1,2,4-thiadiazol-3-yl)acetamido]-3-acetoacetoxymethyl-3-cephem-4-carboxylic acid (syn isomer), mp 180 to 185°C (dec.).

IR (Nujol):

3350, 3250, 1780, 1720, 1625, 1525 $cm^{-1}$

NMR ($DMSO-d_6$, $\delta$):

1.47 (6H, s), 2.17 (3H, s), 3.55 (2H, bs), 3.62 (2H, s), 4.80, 5.03 (2H, ABq, J=14Hz), 5.17 (1H, d, J=4Hz), 5.87 (1H, 2d, J=4 and 8Hz), 8.13 (2H, s), 9.47 (1H, d, J=8Hz)

(6) 7-[2-(1-Methyl-1-t-butoxycarbonylethoxyimino)-2-(5-amino-1,2,4-thiadiazol-3-yl)acetamido]-3-acetoacetoxymethyl-3-cephem-4-carboxylic acid (syn isomer), mp 140 to 145°C (dec.).

IR (Nujol):

3350, 3250, 1785, 1720, 1620, 1530 $cm^{-1}$

NMR ($DMSO-d_6$, $\delta$):

1.47 (6H, s), 1.50 (9H, s), 2.17 (3H, s), 3.57 (2H, bs), 3.63 (2H, s), 4.80, 5.07 (2H, ABq, J=14Hz), 5.17 (1H, d, J=4Hz), 5.86 (1H, 2d, J=4 and 8Hz), 8.13 (2H, s), 9.43 (1H, d, J=8Hz)

(7) Sodium 7-[2-ethoxyimino-2-(5-amino-1,2,4-thiadiazol-3-yl)acetamido]-3-acetoacetoxymethyl-3-cephem-4-carboxylic acid (syn isomer), mp 175 to 180°C (dec.).

IR (Nujol):

3450, 3300, 3100, 1790, 1720, 1670, 1640, 1610, 1550 $cm^{-1}$

NMR ($D_2O$, $\delta$):

1.38 (3H, t, J=6Hz), 2.34 (3H, s), 3.44, 3.66 (2H, ABq, J=18Hz), 4.40 (2H, q, J=6Hz), 5.05, 5.86 (2H, ABq, J=12Hz), 5.26 (1H, d, J=4Hz), 5.90 (1H, d, J=4Hz)

(8) 7-[2-Methoxyimino-2-(5-amino-1,2,4-thiadiazol-3-yl)acetamido]-3-acetoacetoxymethyl-3-cephem-4-carboxylic acid (syn isomer), mp 120 to 125°C (dec.).

IR (Nujol):

3350, 3250, 1780, 1710, 1680, 1630, 1530 $cm^{-1}$

NMR ($D_2O+NaHCO_3$, $\delta$):

2.32 (3H, s), 3.40, 3.62 (2H, ABq, J=18Hz), 4.10 (3H, s), 4.84, 5.04 (2H, ABq, J=14Hz), 5.22 (1H, d, J=4Hz), 5.86 (1H, d, J=4Hz)

(9) 7-[2-Propoxyimino-2-(5-amino-1,2,4-thiadiazol-3-yl)acetamido]-3-acetoacetoxymethyl-3-cephem-4-carboxylic acid (syn isomer), mp 125 to 130°C (dec.).

IR (Nujol):

3350, 3250, 1780, 1710, 1680, 1620, 1530 $cm^{-1}$

NMR ($D_2O+NaHCO_3$, δ):

0.94 (3H, t, J=6Hz), 1.5—1.9 (2H, m), 2.30 (3H, s), 3.40, 3.62 (2H, ABq, J=18Hz), 4.26 (2H, t, J=6Hz), 4.84, 5.04 (2H, ABq, J=12Hz), 5.22 (1H, d, J=4Hz), 5.86 (1H, d, J=4Hz)

(10) 7-[2-Isopropoxyimino-2-(5-amino-1,2,4-thiadiazol-3-yl)acetamido]-3-acetoacetoxymethyl-3-cephem-4-carboxylic acid (syn isomer), mp 95 to 100°C (dec.).

IR (Nujol):

3400, 3300, 3200, 1775, 1740, 1710, 1670, 1620, 1525 $cm^{-1}$

NMR (DMSO-$d_6$, δ):

1.28 (6H, d, J=6Hz), 2.18 (3H, s), 3.48, 3.60 (2H, ABq, J=18Hz), 3.62 (2H, s), 4.24—4.54 (1H, m), 4.78, 5.02 (2H, ABq, J=13Hz), 5.14 (1H, d, J=5Hz), 5.80 (1H, dd, J=5 and 8Hz), 8.06 (2H, broad s), 9.44 (1H, d, J=8Hz)

(11) Sodium 7-[2-allyloxyimino-2-(5-amino-1,2,4-thiadiazol-3-yl)acetamido]-3-acetoacetoxymethyl-3-cephem-4-carboxylate (syn isomer), mp 160 to 170°C (dec.).

IR (Nujol):

3450, 3300, 3100, 1790, 1720, 1670, 1550 $cm^{-1}$

NMR ($D_2O$, δ):

2.31 (3H, s), 3.33, 3.64 (2H, ABq, J=18Hz), 4.6—5.1 (4H, m), 5.1—5.5 (2H, m), 5.20 (1H, d, J=5Hz), 5.8—6.3 (1H, m), 5.84 (1H, d, J=5Hz)

(12) Sodium 7-[2-(2,2,2-trifluoroethoxyimino)-2-(5-amino-1,2,4-thiadiazol-3-yl)acetamido]-3-acetoacetoxymethyl-3-cephem-4-carboxylate (syn isomer), mp 128 to 132°C (dec.).

IR (Nujol):

3300, 1780, 1710, 1670, 1600, 1530 $cm^{-1}$

NMR ($D_2O$, δ):

2.32 (3H, s), 3.50, 3.63 (2H, ABq, J=17Hz), 4.60—5.07 (4H, m), 5.23 (1H, d, J=4Hz), 5.87 (1H, d, J=4Hz)

(13) Sodium 7-[2-methylthiomethoxyimino-2-(5-amino-1,2,4-thiadiazol-3-yl)acetamido]-3-acetoacetoxymethyl-3-cephem-4-carboxylate (syn isomer), mp 180 to 190°C (dec.).

IR (Nujol):

3500—3200, 1770, 1670, 1620, 1530 $cm^{-1}$

NMR (DMSO-$d_6$, δ):

2.18 (3H, s), 2.20 (3H, s), 3.16, 3.48 (2H, ABq, J=18Hz), 3.58 (2H, s), 4.84, 5.08 (2H, ABq, J=12Hz), 4.98 (1H, d, J=5Hz), 5.22 (2H, s), 5.62 (1H, dd, J=5 and 8Hz), 8.14 (2H, broad s), 9.48 (1H, d, J=8Hz)

(14) 7-[2-(2-Propynyloxyimino)-2-(5-amino-1,2,4-thiadiazol-3-yl)acetamido]-3-acetoacetoxymethyl-3-cephem-4-carboxylic acid (syn isomer), mp 90 to 95°C (dec.).

IR (Nujol):

3400, 3280, 3200, 2100, 1770, 1740, 1710, 1670, 1620 $cm^{-1}$

NMR (DMSO-$d_6$, δ):

2.18 (3H, s), 3.46 (1H, t, J=2Hz), 3.58 (2H, ABq, J=18Hz), 3.62 (2H, s), 4.76 (2H, d, J=2Hz), 4.78, 5.02 (2H, ABq, J=14Hz), 5.12 (1H, d, J=5Hz), 5.80 (1H, dd, J—5 and 8Hz), 8.10 (2H, broad s), 9.60 (1H, d, J=8Hz)

Preparation 33

To a mixture of sodium iodide (80 g) and pyridine (11.36 g) in water (40 ml) was added sodium 7-[D-5-carboxy-5-(3-phenylureido)valeramido]cephalosporanate (40 g) at 50°C under stirring, which was continued at 60°C for 4.5 hours. The warm reaction mixture was diluted with water (80 ml), adjusted to pH 3.5 with 6N hydrochloric acid and subjected to column chromatography on a non-ionic adsorption resin "Diaion HP—20" (Trademark, prepared by Mitsubishi Chemical Industries) (600 ml). After the column was washed with water (2.4 l), elution was carried out with 35% aqueous isopropyl alcohol, which was warmed to 45°C prior to use. To the eluate (1 l) was added N,N-dimethylformamide (100 ml) and the mixture was concentrated to 120 ml under reduced pressure. To the residue was added isopropyl alcohol (1 l) under stirring, which was continued for one hour. The resulting precipitates were collected by filtration, washed with isopropyl alcohol and dried to give 7 - [D - 5 - carboxy - 5 - (3 - phenylureido)valeramido] - 3 - (1 - pyridiniomethyl) - 3 - cephem - 4 - carboxylate (22.0 g), mp 180 to 185°C (dec.).

IR (Nujol):

3300, 1780, 1720, 1680, 1610, 1540, 1500 $cm^{-1}$

NMR ($DMSO-d_6+D_2O$, $\delta$):

1.4—1.8 (4H, m), 2.0—2.3 (2H, m), 3.14, 3.54 (2H, ABq, J=17Hz), 4.0—4.2 (1H, m), 5.04 (1H, d, J=4Hz), 5.24, 5.62 (2H, ABq, J=14Hz), 5.60 (1H, d, J=4Hz), 6.7—7.5 (5H, m), 8.0—8.2 (2H, m), 8.45—8.70 (1H, m), 9.28—9.42 (2H, m)

Preparation 34

To a mixture of 7 - [D - 5 - carboxy - 5 - (3 - phenylureido)valeramido] - 3 - (1 - pyridiniomethyl) - 3 - cephem - 4 - carboxylate (2.77 g) and N,N-dimethylaniline (4.2 g) in methylene chloride (30 ml) was dropped trimethylsilyl chloride (3.3 g) at ambient temperature under stirring, which was continued for 30 minutes. The mixture was cooled to –30°C and phosphorus pentachloride (2.1 g) was added thereto under stirring, which was continued for one hour at –30°C to –25°C. The reaction mixture was added to a solution of 1,3-butandiol (4.5 g) in methylene chloride (30 ml) at –20°C under stirring, which was continued for 1.5 hours at ambient temperature. The resulting precipitates were collected by filtration, washed with methylene chloride and dried to give a crude product (2.1 g) of 1 - [(7 - amino - 4 - carboxy - 3 - cephem - 3 - yl)methyl]pyridinium chloride hydrochloride dihydrate. To the crude product was added 1H hydrochloric acid (8 ml) and the mixture was stirred for 30 minutes at ambient temperature. An insoluble material was filtered off and the filtrate was cooled in an ice-bath, followed by an addition of isopropyl alcohol (20 ml) under stirring. To the mixture was added isopropyl alcohol (25 ml) and the resulting precipitates were filtered, washed with the same solvent and acetone and dried to give a pure product (1.15 g), mp 140 to 145°C (dec.).

NMR ($D_2O$, $\delta$):

3.53, 3.80 (2H, ABq, J=18Hz), 5.30 (1H, d, J=4Hz), 5.45 (1H, d, J=4Hz), 5.53, 5.83 (2H, ABq, J=14Hz), 8.00—8.33 (2H, m), 8.50—8.33 (1H, m), 8.90—9.13 (2H, m)

Preparation 35

The following compound was obtained according to a similar manner to that of Preparation 30.

Sodium 7-[2-ethoxyimino-2-(5-amino-1,2,4-thiadiazol-3-yl)acetamido]cephalosporanate (syn isomer), mp 180 to 185°C (dec.).

IR (Nujol):

3480, 3430, 3250, 1780, 1730, 1665, 1635, 1610, 1540, 1515, 1400, 1280, 1240, 1040 $cm^{-1}$

Example 1

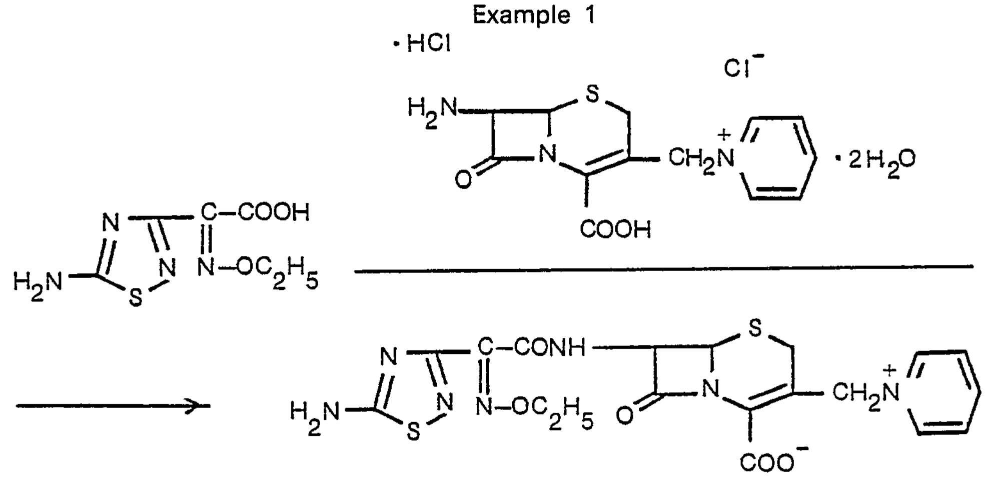

To a cold solution of phosphorus pentachloride (2.64 g) in methylene chloride (25 ml) was added 2-ethoxyimino-2-(5-amino-1,2,4-thiadiazol-3-yl)acetic acid (syn isomer) (2.48 g) at –20°C and the mixture was stirred for 35 minutes at –20 to –14°C. To the mixture was added cold diisopropyl ether (75 ml) below –10°C under stirring, which was continued until the mixture was warmed to ambient temperature. The resulting precipitates were collected by filtration, washed with diisopropyl ether and then kept in a desiccator for several minutes. On the other hand, a mixture of 1-[(7-amino-4-carboxy-3-cephem-3-yl)methyl]pyridinium chloride hydrochloride dihydrate (3.27 g) and trimethylsilylacetamide (16 g) in methylene chloride (50 ml) was warmed at 35°C to make a solution, which was cooled to –20°C. To the cold solution were added the precipitates prepared above and the mixture was stirred for 25 minutes at –18 to –12°C and for an additional 20 minutes at –12 to –3°C. A solution of sodium bicarbonate (4 g) in water (30 ml) was added to the reaction mixture and the aqueous layer was separated out, adjusted to pH 1 with 6N hydrochloric acid, washed with ethyl acetate and then readjusted to pH 4 with an aqueous solution of sodium bicarbonate. The aqueous solution was passed

through a column packed with alumina (16 g) and then subjected to column chromatography on a non-ionic adsorption resin Diaion HP—20 (trademark: prepared by Mitsubishi Chemical Industries) (100 ml). After the column was washed with water, the elution was carried out with 20% aqueous methanol. The eluates containing an object compound were collected, evaporated to remove methanol under reduced pressure and lyophilized to give white powder of 7-[2-ethoxyimino-2-(5-amino-1,2,4-thiadiazol-3-yl)acetamido]-3-(1-pyridiniomethyl)-3-cephem-4-carboxylate (syn isomer) (2.39 g) mp. 155 to 165°C (dec.).

IR (Nujol):

3400—3150, 1770, 1660, 1610, 1530 $cm^{-1}$

NMR (DMSO-$d_6$, $\delta$):

1.21 (3H, t, J=7Hz), 2.9—3.7 (2H, m), 4.12 (2H, q, J=7Hz), 5.05 (1H, d, J=5Hz), 5.19, 5.68 (2H, ABq, J=14Hz), 5.7 (1H, m), 8.1 (4H, m), 8.6 (1H, m), 9.4 (3H, m)

Example 2

To a cold solution of phosphorus pentachloride (1.25 g) in methylene chloride (30 ml) was added 2-(2-cyclopenten-1-yloxyimino)-2-(5-amino-1,2,4-thiadiazol-3-yl) acetic acid (syn isomer) (1.5 g) at —15°C and the mixture was stirred for 30 minutes at —13 to —10°C. On the other hand, a mixture of N-[7-amino-3-cephem-3-ylmethyl]pyridinium-4-carboxylate dihydrochloride (1.82 g) and trimethylsilylacetamide (10 g) in methylene chloride (50 ml) was stirred for 10 minutes at room temperature and cooled to —10°C. The cooled solution was added to the above activated mixture and the mixture was stirred for 15 minutes at —10°C. The reaction mixture was poured into an aqueous solution (100 ml) of sodium bicarbonate (3.6 g) and stirred for 15 minutes at room temperature. The aqueous layer was separated out, adjusted to pH 2 with 10% hydrochloric acid and washed with ethyl acetate. The aqueous solution was subjected to column chromatography on a non ionic adsorption resin, Diaion HP—20 (Trademark, prepared by Mitsubishi Chemical Industries) (100 ml). After the column was washed with water, the elution was carried out with 40% aqueous methanol. The eluates containing an object compound were collected, evaporated to remove methanol under reduced pressure and lyophilized to give white powder of N - [7 - {2 - (2 - cyclopenten - 1 - yloxyimino) - 2 - (5 - amino - 1,2,4 - thiadiazol - 3 - yl)acetamido} - 3 - cephem - 3 - ylmethyl]pyridinium - 4 - carboxylate (syn isomer) (1.5 g), mp. 190 to 195°C (dec.).

IR (Nujol):

3350, 3200, 1780, 1660, 1620, 1530 $cm^{-1}$

NMR ($D_2O$+$NaHCO_3$, $\delta$):

1.9—2.5 (4H, m), 3.23, 3.60 (2H, ABq, J=16Hz), 5.2—6.1 (7H, m), 7.9—9.1 (5H, m)

Example 3

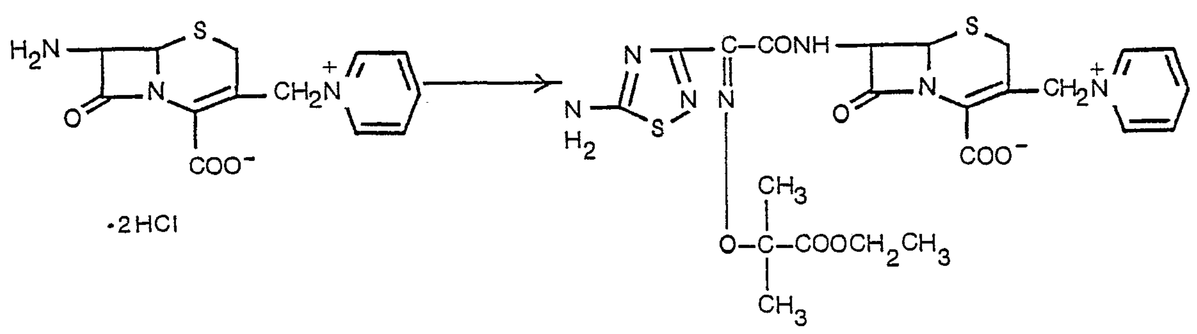

To a cold solution of phosphorous pentachloride (1.46 g) in methylene chloride (30 ml) was added 2 - (1 - methyl - 1 - ethoxycarbonylethoxyimino) - 2 - (5 - amino - 1,2,4 - thiadiazol - 3 - yl)acetic acid (syn isomer) (2.11 g) at —18°C and the mixture was stirred for 30 minutes at —14 to —11°C. To the reaction mixture was added dry n-hexane (90 ml) below —10°C and the mixture was stirred for several minutes and the solvent was removed by decantation. The residue was triturated with n-hexane and collected by filtration to obtain a powder of the activated acid. On the other hand, a mixture of N - [7 - amino - 3 - cephem - 3 - ylmethyl]pyridinium - 4 - carboxylate dihydrochloride (2 g) and trimethylsilylacetamide (10 g) in methylene chloride was stirred for 10 minutes at room temperature and cooled to —18°C. To the cold solution was added the powder obtained above and the mixture was stirred for 30 minutes at —13 to —10°C and for 30 minutes at —5 to 0°C. The reaction mixture was poured into an aqueous solution (100 ml) of sodium bicarbonate (3.6 g) and stirred for 15 minutes at room temperature and then adjusted to pH 1 with 6N hydrochloric acid. The aqueous layer was separated out, washed with ethyl acetate and subjected to column chromatography on a non ionic adsorption resin, Diaion HP—20 (100 ml). After the column was washed with water, 5% aqueous ethanol and 10% aqueous ethanol successively, the elution was carried out with 20% aqueous ethanol. The eluates containing an object compound were collected, evaporated to

remove ethanol under reduced pressure and lyophilized to give N-[7-{2-(1-methyl-1-ethoxycarbonylethoxyimino) - 2 - (5 - amino - 1,2,4 - thiadiazol - 3 - yl) - acetamido} - 3 - cephem - 3 - ylmethyl]-pyridinium - 4 - carboxylate (syn isomer) (1.30 g), white powder, mp. 164 to 168°C (dec.).

IR (Nujol):
3350—3150, 1770, 1720, 1670, 1620, 1520 $cm^{-1}$

NMR (DMSO-$d_6$, $\delta$):
1.15 (3H, t, J=7Hz), 1.45 (6H, s), 3.03 and 3.55 (2H, ABq, J=18Hz), 4.10 (2H, q, J=7Hz), 5.11 (1H, d, J=Hz), 5.20 and 5.67 (2H, ABq, J=13Hz), 5.75 (1H, 2d, J=5 and 8Hz), 8.20 (4H, m), 8.57 (1H, m), 9.47 (3H, m)

Example 4

The following compounds were obtained according to similar manners to those of Examples 1 to 3.

(1) 7-[2-Propoxyimino-2-(5-amino-1,2,4-thiadiazol-3-yl)acetamido]-3-(1-pyridiniomethyl)-3-cephem-4-carboxylic (syn isomer) mp. 230 to 240°C (dec.).

IR (Nujol):
3400—3200, 1770, 1670—1600, 1530 $cm^{-1}$

NMR (DMSO-$d_6$, $\delta$):
0.85 (3H, t, J=7Hz), 1.6 (2H, m), 3.06, 3.55 (2H, ABq, J=18Hz), 4.04 (2H, t, J=6Hz), 5.06 (1H, d, J=5Hz), 5.18, 5.70 (2H, ABq, J=14Hz), 5.74 (1H, dd, J=5 and 8Hz), 8.2 (4H, m), 8.6 (1H, m), 9.5 (3H, m)

(2) 7-[2-Methoxyimino-2-(5-amino-1,2,4-thiadiazol-3-yl)acetamido]-3-(1-pyridiniomethyl)-3-cephem-4-carboxylate (syn isomer), mp. 250 to 260°C (dec.).

IR (Nujol):
3400—3100, 1770, 1650, 1610, 1520 $cm^{-1}$

NMR (DMSO-$d_6$, $\delta$):
3.07, 3.57 (2H, ABq, J=18Hz), 3.86 (3H, s), 5.06 (1H, d, J=5Hz), 5.19, 5.69 (2H, ABq, J=14Hz), 5.73 (1H, dd, J=5, 8Hz), 8.0—8.3 (4H, m), 8.4—8.7 (1H, m), 9.3—9.6 (3H, m).

(3) 7-[2-Isopropoxyimino-2-(5-amino-1,2,4-thiadiazol-3-yl)acetamido]-3-(1-pyridiniomethyl)-3-cephem-4-carboxylate (syn isomer), mp. 160 to 165°C (dec.).

IR (Nujol):
3270, 3180, 1770, 1660, 1610, 1525 $cm^{-1}$

NMR (DMSO-$d_6$+$D_2O$, $\delta$):
1.22 (6H, d, J=6Hz), 3.15, 3.57 (2H, ABq, J=18Hz), 4.17—4.60 (1H, m), 5.12 (1H, d, J=5Hz), 5.33, 5.70 (2H, ABq, J=14Hz), 5.78 (1H, d, J=5Hz), 8.0—8.4 (2H, m), 8.47—8.83 (1H, m), 9.33—9.67 (2H, m)

(4) N-[7-{2-(t-Butoxycarbonylmethoxyimino)-2-(5-amino-1,2,4-thiadiazol-3-yl)acetamido]-3-cephem-3-ylmethyl]pyridinium-4-carboxylate (syn isomer), mp. 150 to 155°C (dec.).

IR (Nujol):
3300, 3200, 1770, 1680, 1620, 1530 $cm^{-1}$

(5) N-[7-{2-Carboxymethoxyimino-2-(5-amino-1,2,4-thiadiazol-3-yl)acetamido}-3-cephem-3-ylmethyl]pyridinium-4-carboxylate (syn isomer), mp. 150 to 155°C (dec.).

IR (Nujol):
3350, 3200, 1780, 1680, 1530 $cm^{-1}$

(6) N-[7-{2-Cyclopentyloxyimino-2-(5-amino-1,2,4-thiadiazol-3-yl)acetamido}-3-cephem-3-ylmethyl]pyridinium-4-carboxylate (syn isomer), mp. 180 to 185°C (dec.).

IR (Nujol):
3300, 3200, 1780, 1670, 1620, 1530 $cm^{-1}$

(7) N-[7-{2(1-Carboxyethoxyimino)-2-(5-amino-1,2,4-thiadiazol-3-yl)acetamido}-3-cephem-3-ylmethyl]-4'-carbamoylpyridinium-4-carboxylate (syn isomer), mp. 170 to 175°C (dec.).

IR (Nujol):
3300, 3160, 1770, 1680, 1610, 1560, 1520 $cm^{-1}$

(8) N-[7-{2-(1-t-Butoxycarbonylethoxyimino)-2-(5-amino-1,2,4-thiadiazol-3-yl)acetamido}-3-cephem-3-ylmethyl]pyridinium-4-carboxylate (syn isomer), mp. 160 to 165°C (dec.).

IR (Nujol):
3290, 3160, 1770, 1725, 1670, 1620, 1525 $cm^{-1}$

NMR ($CD_3OD+D_2O$, δ):
1.2—1.6 (12H, m), 3.20 and 3.67 (2H, ABq, J=18Hz), 4.40—4.90 (1H, m), 5.20 (1H, d, J=5Hz), 5.33—5.80 (2H, m), 5.92 (1H, d, J=5Hz), 7.9—9.4 (5H, m).

(9) N-[7-{2-(1-Carboxyethoxyimino)-2-(5-amino-1,2,4-thiadiazol-3-yl)acetamido}-3-cephem-3-ylmethyl]pyridinium-4-carboxylate (syn isomer), mp. 175 to 180°C (dec.).
IR (Nujol):
3300, 3200, 1775, 1670, 1620, 1520 $cm^{-1}$

(10) N-[7-{2-(1-Benzyloxycarbonylethoxyimino)-2-(5-amino-1,2,4-thiadiazol-3-yl)acetamido}-3-cephem-3-ylmethyl]pyridinium-4-carboxylate (syn isomer), mp. 178 to 182°C (dec.).
IR (Nujol):
3250, 3150, 1770, 1670, 1620, 1520 $cm^{-1}$
NMR ($DMSO-d_6+D_2O$, δ):
1.45 (3H, d, J=7Hz), 3.10 and 3.60 (2H, ABq, J=16Hz), 4.87 (1H, q, J=7Hz), 5.20 (2H, s), 4.97—5.10 (2H, m), 5.25 (1H, d, J=5Hz), 5.83 (1H, d, J=5Hz), 7.43 (5H, s), 8.27 (2H, m), 8.63 (1H, m), 9.38 (2H, m)

(11) N-[7-{2-Ethoxycarbonylmethoxyimino-2-(5-amino-1,2,4-thiadiazol-3-yl)acetamido}-3-cephem-3-ylmethyl]pyridinium-4-carboxylate (syn isomer), mp. 184 to 188°C (dec.).
IR (Nujol):
3400—3100, 1770, 1670, 1610, 1520 $cm^{-1}$
NMR ($DMSO-d_6$, δ):
1.17 (3H, t, J=7Hz), 3.05 and 3.53 (2H, ABq, J=18Hz), 4.13 (2H, q, J=7Hz), 4.70 (2H, s), 5.08 (1H, d, J=5Hz), 5.17 and 5.70 (2H, ABq, J=13Hz), 5.72 (1H, dd, J=5 and 8hz), 8.16 (4H, m), 8.62(1H, m), 9.50 (3H, m)

(12) N-[7-{2-(2-Cyclohexen-1-yloxyimino)-2-(5-amino-1,2,4-thiadiazol-3-yl)acetamido}-3-cephem-3-ylmethyl]pyridinium-4-carboxylate (syn isomer), mp. 150 to 155°C (dec.).
IR (Nujol):
3300, 3200, 1775, 1660, 1610, 1520 $cm^{-1}$
NMR ($DMSO-d_6$, δ):
1.5—2.0 (6H, m), 3.13, 3.57 (2H, ABq, J=17Hz), 4.6—4.7 (1H, m), 5.07 (1H, d, J=4Hz), 5.27, 5.60 (2H, ABq, J=14Hz), 5.80 (1H, 2d, J=4 and 8Hz), 5.77—6.0 (2H, m), 8.17 (2H, s), 8.0—8.4 (2H, m), 8.43—8.80 (1H, m), 9.4—9.5 (2H, m), 9.55 (1H, d, J=8Hz)

(13) N-[7-{2-Carboxymethoxyimino-2-(5-amino-1,2,4-thiadiazol-3-yl)acetamido}-3-cephem-3-ylmethyl]-4'-carbamoylpyridinium-4-carboxylate (syn isomer), mp. 175 to 180°C (dec.).
IR (Nujol):
3350, 3200, 1775, 1680, 1615, 1565, 1525 $cm^{-1}$

(14) N-[7-{2-Methoxycarbonylmethoxyimino-2-(5-amino-1,2,4-thiadiazol-3-yl)acetamido}-3-cephem-3-ylmethyl]pyridinium-4-carboxylate (syn isomer), mp. 165 to 170°C (dec.).
IR (Nujol):
3300—3150, 1760, 1670, 1620, 1520 $cm^{-1}$
NMR ($D_2O$, δ):
3.17, 3.70 (2H, ABq, J=18Hz), 3.80 (3H, s), 4.93 (2H, s), 5.30 (1H, d, J=5Hz), 5.44, 5.73 (2H, ABq, J=14Hz), 5.93 (1H, d, J=5Hz), 8.10 (2H, m), 8.60 (1H, m), 8.98 (2H, m)

(15) N-[7-{2-(1-Methyl-1-carboxyethoxyimino)-2-(5-amino-1,2,4-thiadiazol-3-yl)acetamido}-3-cephem-3-ylmethyl]pyridinium-4-carboxylate (syn isomer), white powder, mp. 176 to 180°C (dec.).
IR (Nujol):
3400—3150, 1770, 1670, 1620, 1520 $cm^{-1}$

(16) N-[7-{2-(1-Methyl-1-t-butoxycarbonylethoxyimino)-2-(5-amino-1,2,4-thiadiazol-3-yl)acetamido}-3-cephem-3-ylmethyl]pyridinium-4-carboxylate (syn isomer), mp. 176 to 180°C (dec.).
IR (Nujol):
3300, 3200, 1780, 1730, 1680, 1620, 1520 $cm^{-1}$
NMR ($DMSO-d_6—D_2O$, δ):
1.40 (15H, bs), 3.08, 3.42 (2H, ABq, J=18Hz), 5.13 (1H, d, J=5Hz), 5.40 (2H, m), 5.80 (1H, d, J=5Hz), 8.17 (2H, m), 8.65 (1H, m), 9.37 (2H, m)

(17) N-[7-[2-Cyclopentyloxyimino-2-(5-amino-1,2,4-thiadiazol-3-yl)acetamido]-3-cephem-3-ylmethyl]-4'-carbamoylpyridinium-4-carboxylate (syn isomer), mp 230 to 235°C (dec.).
IR (Nujol):
3300, 3200, 1770, 1680, 1610, 1560, 1520, 1510 $cm^{-1}$

(18) N-(7-(2-(2-Cylcopenten-1-yl-oxyimino)-2-(5-amino-1,2,4-thiadiazol-3-yl)acetamido)-3-cephem-3-ylmethyl)-4'-carbamoyl-pyridinium-4-carboxylate (syn isomer). mp 155 to 160°C (DEC.).
IR (Nujol):
3300, 3150, 1770, 1675, 1610, 1560, 1520 $cm^{-1}$
NMR ($DMSO-d_6$, $\delta$):
2.0—2.4 (4H, m), 3.17—3.67 (2H, m), 5.08 (1H, d, J=5Hz), 5.23—6.30 (6H, m), 8.27 (2H, broad s), 8.57 (2H, d, J=7Hz), 9.53 (1H, d, J=8Hz), 9.70 (2H, d, J=7Hz)

(19) N-(7-(2-(1-Methyl-1-carboxyethoxyimino)-2-(5-amino-1,2,4-thiadiazol-3-yl)acetamido)-3-cephem-3-ylmethyl)-4'-carbamoyl-pyridinium-4-carboxylate (syn isomer), mp 180 to 185°C (DEC.).
IR (Nujol):
3300, 1770, 1680, 1620, 1560, 1520 $cm^{-1}$
NMR ($DMSO-d_6$, $\delta$):
1.40 (6H, s), 3.0—3.6 (2H, m), 5.10 (1H, d, J=4Hz), 5.3—5.7 (2H, m), 5.80 (2H, dd, J=4 and 8Hz)), 9.18 (2H, d, J=7Hz), 9.50 (1H, d, J=8Hz), 9.63 (2H, d, J=7Hz)

(20) 7-[2-Allyloxyimino-2-(5-amino-1,2,4-thiadiazol-3-yl)acetamido]-3-(1-pyridiniomethyl)-3-cephem-4-carboxylate (syn isomer), mp 160 to 165°C (dec.).
IR (Nujol):
3290, 3180, 1770, 1660, 1610, 1525 $cm^{-1}$
NMR ($DMSO-d_6+D_2O$, $\delta$):
3.12, 3.50 (2H, ABq, J=18Hz), 4.44—4.76 (2H, m), 5.10 (1H, d, J=5Hz), 5.0—6.1 (6H, m), 8.0—8.4 (2H, m), 8.44—8.76 (1H, m), 9.32—9.68 (2H, m)

(21) 7-[2-(2-Propynyloxyimino)-2-(5-amino-1,2,4-thiadiazol-3-yl)acetamido]-3-(1-pyridiniomethyl)-3-cephem-4-carboxylate (syn isomer), mp 145 to 150°C (dec.).
IR (Nujol):
3250, 2100, 1770, 1660, 1630, 1610, 1525 $cm^{-1}$
NMR ($DMSO-d_6$, $\delta$):
3.10, 3.55 (2H, ABq, J=18Hz), 3.47 (1H, t, J=2Hz), 4.73 (2H, d, J=2Hz), 5.08 (1H, d, J=5Hz), 5.25, 5.65 (2H, ABq, J=14Hz), 5.60—5.93 (1H, m), 8.0—8.4 (4H, m), 8.4—8.8 (1H, m), 9.3—9.7 (3H, m)

(22) 7-[2-Hydroxyimino-2-(5-amino-1,2,4-thiadiazol-3-yl)acetamido]-3-(1-pyridiniomethyl)-3-cephem-4-carboxylate (syn isomer), mp 170 to 175°C (dec.).
IR (Nujol):
3350, 3200, 1780, 1620, 1530, 1490 $cm^{-1}$

(23) 7-[2-Methylthiomethoxyimino-2-(5-amino-1,2,4-thiadiazol-3-yl)acetamido]-3-(1-pyridiniomethyl)-3-cephem-4-carboxylate (syn isomer), mp 195 to 205°C (dec.).
IR (Nujol):
3350—3150, 1770, 1670, 1620, 1520, 1150 $cm^{-1}$
NMR ($DMSO-d_6+D_2O$, $\delta$):
2.17 (3H, s), 3.00, 3.62 (2H, ABq, J=18Hz), 5.10 (1H, d, J=5Hz), 5.22 (2H, s), 5.73 (1H, d, J=5Hz), 5.00—5.83 (2H, m), 8.13 (2H, m), 8.53 (1H, m), 9.33 (2H, m)

(24) 7-[2-Trityloxyimino-2-(5-amino-1,2,4-thiadiazol-3-yl)acetamido]-3-(1-pyridiniomethyl)-3-cephem-4-carboxylate (syn isomer), mp 165 to 170°C (dec.).
IR (Nujol):
3450, 1780, 1670, 1620, 1530, 1490 $cm^{-1}$
NMR ($DMSO-d_6$, $\delta$):
3.18, 3.64 (2H, ABq, J=18Hz), 5.18 (1H, d, J=5Hz), 5.34, 5.74 (2H, ABq, J=12Hz), 5.92 (1H, dd, J=5 and 8Hz), 7.28 (15H, s), 7.94—8.30 (4H, m), 8.42—8.66 (1H, m), 9.22—9.54 (2H, m), 9.78 (1H, d, J=8Hz)

(25) 7-[2-(2,2,2-Trifluoroethoxyimino)-2-(5-amino-1,2,4-thiadiazol-3-yl)acetamido]-3-(1-pyridiniomethyl)-3-cephem-4-carboxylate (syn isomer), mp 150 to 155°C (dec.).
IR (Nujol):
3300, 1780, 1675, 1630, 1530 $cm^{-1}$

NMR ($DMSO-d_6+D_2O$, $\delta$):

3.23, 3.50 (2H, ABq, J=18Hz), 4.63, 4.93 (2H, ABq, J=9Hz), 5.17 (1H, d, J=5Hz), 5.37, 5.73 (2H, ABq, J=14Hz), 5.83 (1H, d, J=5Hz), 8.1—8.4 (2H, m), 8.5—8.8 (1H, m), 9.3—9.6 (2H, m)

(26) 7-[2-Ethoxyimino-2-(5-amino-1,2,4-thiadiazol-3-yl)acetamido]-3-(4-carbamoyl-1-pyridiniomethyl)-3-cephem-4-carboxylate (syn isomer), mp 160 to 165°C (dec.).

IR (Nujol):

3300, 3200, 1780, 1680, 1620, 1570, 1530 $cm^{-1}$

(27) 7-[2-(2-Oxotetrahydrofuran-3-yloxyimino)-2-(5-amino-1,2,4-thiadiazol-3-yl)acetamido]-3-(1-pyridiniomethyl)-3-cephem-4-carboxylate (syn isomer), mp 140 to 145°C (dec.).

IR (Nujol):

3350, 1780, 1670, 1620, 1530, 1490 $cm^{-1}$

NMR ($DMSO-d_6+D_2O$, $\delta$):

2.43—2.83 (2H, m), 3.27, 3.63 (2H, ABq, J=18Hz), 4.23—4.67 (2H, m), 5.17—5.37 (1H, m), 5.20 (1H, d, J=5Hz), 5.38, 5.73 (2H, ABq, J=13Hz), 5.87 (1H, d, J=5Hz), 8.07—8.43 (2H, m), 8.53—8.80 (1H, m), 9.23—9.50 (2H, m)

(28) 7-[2-Methoxyimino-2-(5-amino-1,2,4-thiadiazol-3-yl)acetamido]-3-(4-carbamoyl-1-pyridiniomethyl)-3-cephem-4-carboxylate (syn isomer), mp 165 to 170°C (dec.).

IR (Nujol):

3350, 3200, 1780, 1690, 1610, 1570, 1530 $cm^{-1}$

(29) 7-[2-Propoxyimino-2-(5-amino-1,2,4-thiadiazol-3-yl)acetamido]-3-(4-carbamoyl-1-pyridiniomethyl)-3-cephem-4-carboxylate (syn isomer), mp 170 to 175°C (dec.).

IR (Nujol):

3350, 3200, 1780, 1690, 1610, 1570, 1530 $cm^{-1}$

(30) 7-[2-Isopropoxyimino-2-(5-amino-1,2,4-thiadiazol-3-yl)acetamido]-3-(4-carbamoyl-1-pyridiniomethyl)-3-cephem-4-carboxylate (syn isomer), mp 155 to 160°C (dec.).

IR (Nujol):

3350, 3220, 1780, 1680, 1615, 1570, 1530 $cm^{-1}$

(31) 7-[2-Allyloxyimino-2-(5-amino-1,2,4-thiadiazol-3-yl)acetamido]-3-(4-carbamoyl-1-pyridiniomethyl)-3-cephem-4-carboxylate (syn isomer), mp 161 to 165°C (dec.).

IR (Nujol):

3400—3150, 1770, 1670, 1610, 1560, 1520 $cm^{-1}$

(32) 7-[2-(2,2,2-Trifluoroethoxyimino)-2-(5-amino-1,2,4-thiadiazol-3-yl)acetamido]-3-(4-carbamoyl-1-pyridiniomethyl)-3-cephem-4-carboxylate (syn isomer), mp 160 to 165°C (dec.).

IR (Nujol):

3300, 3150, 1780, 1680, 1610, 1580, 1520 $cm^{-1}$

(33) 7-[2-Methylthiomethoxyimino-2-(5-amino-1,2,4-thiadiazol-3-yl)acetamido]-3-(4-carbamoyl-1-pyridiniomethyl)-3-cephem-4-carboxylate (syn isomer), mp 160 to 165°C (dec.).

IR (Nujol):

3300, 3150, 1770, 1680, 1610, 1560, 1520 $cm^{-1}$

(34) 7-[2-(2-Propynyloxyimino)-2-(5-amino-1,2,4-thiadiazol-3-yl)acetamido]-3-(4-carbamoyl-1-pyridiniomethyl)-3-cephem-4-carboxylate (syn isomer), mp 155 to 160°C (dec.).

IR (Nujol):

3400, 3250, 3150, 2120, 1770, 1685, 1610, 1560, 1525 $cm^{-1}$

(35) Sodium 7-[2-cyclopentyloxyimino-2-(5-amino-1,2,4-thiadiazol-3-yl)acetamido]-3-(2-methyl-5-oxo-6-hydroxy-2,5-dihydro-1,2,4-triazin-3-yl)thiomethyl-3-cephem-4-carboxylate (syn isomer), mp 169 to 174°C (dec.).

IR (Nujol):

3600—3100, 1760, 1690, 1665, 1640, 1610, 1520, 1005 $cm^{-1}$

(36) Disodium 7-[2-methoxyimino-2-(5-amino-1,2,4-thiadiazol-3-yl)acetamido]-3-(2-methyl-5-oxo-6-oxide-2,5-dihydro-1,2,4-triazin-3-yl)thiomethyl-3-cephem-4-carboxylate (syn isomer), mp 220 to 225°C (dec.).

IR (Nujol):

3400—3150, 1760, 1660, 1640—1560, 1520, 1040 $cm^{-1}$

(37) Disodium 7-[2-ethoxyimino-2-(5-amino-1,2,4-thiadiazol-3-yl)acetamido]-3-(2-methyl-5-oxo-6-oxido-2,5-dihydro-1,2,4-triazin-3-yl)thiomethyl-3-cephem-4-carboxylate (syn isomer), mp 255 to 265°C (dec.).

IR (Nujol):

3400—3150, 1760, 1660, 1600, 1500, 1400, 1030 $cm^{-1}$

(38) 7-[2-(2-Cyclopenten-1-yloxyimino)-2-(5-amino-1,2,4-thiadiazol-3-yl)acetamido]-3-(2-methyl-5-oxo-6-hydroxy-2,5-dihydro-1,2,4-triazin-3-yl)thiomethyl-3-cephem-4-carboxylate (syn isomer), mp 158 to 164°C (dec.).

IR (Nujol):

3450—3150, 1770, 1680, 1630, 1510, 1260, 1180, 1100, 1030, 1010 $cm^{-1}$

NMR (DMSO-$d_6$, $\delta$):

2.0—2.6 (4H, m), 3.2—4.0 (2H, m), 3.62 (3H, s), 4.13, 4.45 (2H, ABq, J=13Hz), 5.13 (1H, d, J=5Hz), 5.2—5.5 (1H, m), 5.7—6.0 (2H, m), 6.0—6.2 (1H, m), 8.20 (2H, broad s), 9.50 (1H, d, J=8Hz)

(39) 7-[2-Isopropoxyimino-2-(5-amino-1,2,4-thiadiazol-3-yl)acetamido]-3-(2-methyl-5-oxo-6-hydroxy-2,5-dihydro-1,2,4-triazin-3-yl)thiomethyl-3-cephem-4-carboxylate (syn isomer), mp 160 to 167°C (dec.).

IR (Nujol):

3400, 3280, 3180, 1780, 1770, 1630, 1515, 1410, 1240, 1009 $cm^{-1}$

NMR (DMSO-$d_6$+$D_2O$, $\delta$):

1.27 (6H, d, J=6Hz), 3.62 (3H, s), 3.5—3.9 (2H, m), 4.13, 4.41 (2H, ABq, J=14Hz), 4.40 (1H, t, J=6Hz), 5.17 (1H, d, J=5Hz), 5.83 (1H, d, J=5Hz)

(40) 7-[2-(2-Propynyloxyimino)-2-(5-amino-1,2,4-thiadiazol-3-yl)acetamido]-3-(2-methyl-5-oxo-6-hydroxy-2,5-dihydro-1,2,4-triazin-3-yl)thiomethyl-3-cephem-4-carboxylic acid (syn isomer), mp 161 to 166°C (dec.).

IR (Nujol):

3260, 3180, 1770, 1670, 1620, 1520, 1335 $cm^{-1}$

NMR (DMSO-$d_6$+$D_2O$, $\delta$):

3.48 (1H, s), 3.61 (3H, s), 3.3—3.9 (2H, m), 4.10, 4.38 (2H, ABq, J=14Hz), 4.77 (2H, s), 5.12 (1H, d, J=5Hz), 5.78 (1H, d, J=5Hz)

(41) 7-[2-Allyloxyimino-2-(5-amino-1,2,4-thiadiazol-3-yl)acetamido]-3-(2-methyl-5-oxo-6-hydroxy-2,5-dihydro-1,2,4-triazin-3-yl)thiomethyl-3-cephem-4-carboxylic acid (syn isomer), mp 169 to 173°C (dec.).

IR (Nujol):

3360, 3210, 1775, 1670, 1625, 1560, 1520, 1250, 1175, 1100, 1020 $cm^{-1}$

NMR (DMSO-$d_6$, $\delta$):

3.3—4.0 (2H, m), 3.58 (3H, s), 4.0—4.6 (2H, m), 4.5—4.8 (2H, m), 5.13 (1H, d, J=5Hz), 5.0—5.6 (3H, m), 5.81 (1H, dd, J=5 and 9Hz), 8.18 (2H, broad s), 9.53 (1H, d, J=9Hz)

Example 5

A mixture of 7 - [2 - cyclopentyloxyimino - 2 - (5 - amino - 1,2,4 - thiadiazol - 3 - yl)-acetamido]cephalosporanic acid (syn isomer (5.1 g), sodium bicarbonate (840 mg), water (50 ml), potassium thiocyanate (24.3 g) and isonicotinamide (1.83 g) was stirred for 22 hours at 50 to 55°C. The reaction mixture was cooled and added to ethyl acetate. The mixture was adjusted to pH 2 with 10% hydrochloric acid and filtered. The aqueous layer was separated from the filtrate, washed with ethyl acetate and evaporated. The residue was subjected to column chromatography (non-ionic adsorption resin, Diaion HP20 prepared by Mitsubishi Chemical Industries) and the column was washed with water (0.7 l) and then eluted with 30% aqueous methanol (0.7 l). The eluates containing the object compound were collected, washed with ethyl acetate and then evaporated. The residue was lyophilized to give N - [7 - {2 - cyclopentyloxyimino - 2 - (5 - amino - 1,2,4 - thiadiazol - 3 - yl)-acetamido} - 3 - cephem - 3 - ylmethyl] - 4' - carbamoylpyridinium - 4 - carboxylate (syn isomer) (1.0 g), mp 230 to 235°C (dec.).

IR (Nujol):

3300, 3200, 1770, 1680, 1610, 1560, 1520, 1510 $cm^{-1}$

NMR ($d_6$-DMSO, $\delta$):

1.30—1.95 (8H, m), 3.15 and 3.50 (2H, ABq, J=18Hz), 5.60—5.75 (1H, m), 5.06 (1H, d, J=4Hz), 5.30 and 5.65 (2H, ABq, J=14Hz), 5.70 (1H, dd, J=4 and 8Hz), 8.12 (2H, s), 8.45 (2H, d, J=6Hz), 9.42 (2H, d, J=6Hz), 9.50 (1H, d, J=8Hz)

Example 6

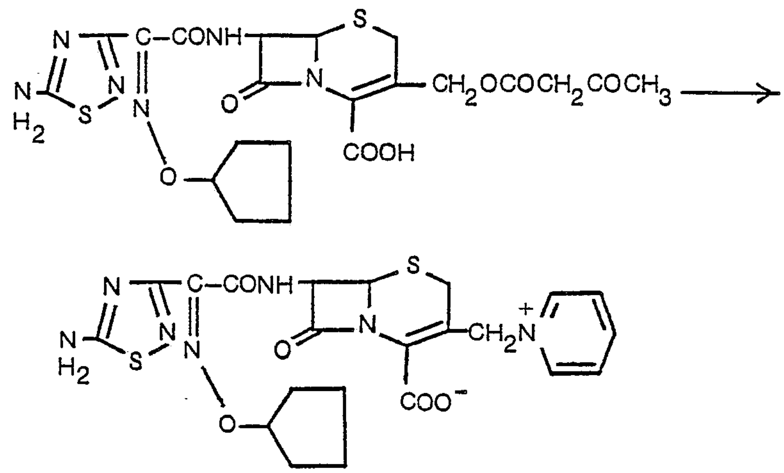

A mixture of 7 - [2 - cyclopentyloxyimino - 2 - (5 - amino - 1,2,4 - thiadiazol - 3 - yl)-acetamido] - 3 - acetoacetoxymethyl - 3 - cephem - 4 - carboxylic acid (syn isomer) (2.8 g), sodium bicarbonate (420 mg), potassium iodide (28 g) and pyridine (590 mg) in water (28 ml) was stirred for one hour at 55°C. After cooling, ethyl acetate (20 ml), 1N hydrochloric acid (5.5 ml) and acetone (10 ml) were added thereto under stirring. The aqueous layer was separated out, washed with ethyl acetate and concentrated to 30 ml under reduced pressure. An insoluble substance was filtered off and the filtrate was subjected to column chromatography on a non ionic adsorption resin, Diaion HP20 (100 ml). After the column was washed with water (500 ml), the elution was carried out with 30% aqueous methanol. The eluates containing a object compound were collected, evaporated to remove methanol under reduced pressure and lyophilized to give N - [7 - {2 - cyclopentyloxyimino - 2 - (5 - amino - 1,2,4 - thiadiazol - 3 - yl)acetamido} - 3 - cephem - 3 - ylmethyl]pyridinium - 4 - carboxylate (syn isomer) (620 mg), white powder, mp. 180 to 185°C (dec.).

IR (Nujol):

3300, 3200, 1780, 1670, 1620, 1530 $cm^{-1}$

NMR (DMSO-$d_6$, $\delta$):

1.4—2.0 (8H, m), 3.17 3.53 (2H, ABq, J=18Hz), 4.60—4.83 (1H, m), 5.10 (1H, d, J=4Hz), 5.30, 5.83 (2H, ABq, J=14Hz), 5.87 (1H, 2d, J=4 and 8Hz), 8.17 (2H, s), 9.50 (1H, d, J=8Hz), 8.0—9.7 (5H, m)

Example 7

The following compounds were obtained according to similar manners to those of Examples 5 and 6.

(1) N-[7-{2-(2-Cyclopenten-1-yloxyimino)-2-(5-amino-1,2,4-thiadiazol-3-yl)acetamido}-3-cephem-3-ylmethyl]pyridinium-4-carboxylate (syn isomer), mp. 190 to 195°C (dec.).

IR (Nujol):

3350, 3200, 1780, 1660, 1620, 1530 $cm^{-1}$

(2) N-[7-{2-(t-Butoxycarbonylmethoxyimino)-2-(5-amino-1,2,4-thiadiazol-3-yl)acetamido}-3-cephem-3-ylmethyl]pyridinium-4-carboxylate (syn isomer), mp. 150 to 155°C (dec.).

IR (Nujol):

3300, 3200, 1770, 1680, 1620, 1530 $cm^{-1}$

(3) N-[7-{2-Carboxymethoxyimino-2-(5-amino-1,2,4-thiadiazol-3-yl)acetamido}-3-cephem-3-ylmethyl]pyridinium-4-carboxylate (syn isomer), mp. 150 to 155°C (dec.).

IR (Nujol):

3350, 3200, 1780, 1680, 1530 $cm^{-1}$

(4) N-[7-{2-Cyclopentyloxyimino-2-(5-amino-1,2,4-thiadiazol-3-yl)acetamido}-3-cephem-3-ylmethyl]pyridinium-4-carboxylate (syn isomer), mp. 180 to 185°C (dec.).

IR (Nujol):

3300, 3200, 1780, 1670, 1620, 1530 $cm^{-1}$

NMR (DMSO-$d_6$, $\delta$):

1.4—2.0 (8H, m), 3.17, 3.53 (2H, ABq, J=18Hz), 4.60—4.83 (1H, m), 5.10 (1H, d, J=4Hz),

5.30 and 5.83 (2H, ABq, J=14Hz), 5.87 (1H, dd, J=4 and 8Hz), 8.17 (2H, s), 9.50 (1H, d, J=8Hz), 8.0—9.7 (5H, m)

(5) N-[7-{2-(1-Carboxyethoxyimino)-2-(5-amino-1,2,4-thiadiazol-3-yl)acetamido}-3-cephem-3-ylmethyl]-4'-carbamoylpyridinium-4-carboxylate (syn isomer), mp. 170 to 175°C (dec.).

IR (Nujol):

3300, 3160, 1770, 1680, 1610, 1560, 1520 $cm^{-1}$

NMR (DMSO-$d_6$, δ):

1.38 (3H, d, J=7Hz), 3.10—3.60 (2H, m), 4.40—4.83 (1H, m), 5.10 (1H, d, J=5Hz), 5.28—6.00 (3H, m), 8.22 (2H, broad s), 8.48 (2H, d, J=6Hz), 9.48 (2H, d, J=6Hz), 9.32—9.65 (1H, m)

(6) N-[7-{2-(1-t-Butoxycarbonylethoxyimino)-2-(5-amino-1,2,4-thiadiazol-3-yl)acetamido}-3-cephem-3-ylmethyl]pyridinium-4-carboxylate (syn isomer), mp. 160 to 165°C (dec.).

IR (Nujol):

3290, 3160, 1770, 1725. 1670, 1620, 1525 $cm^{-1}$

(7) N-[7-{2-(1-Carboxyethoxyimino)-2-(5-amino-1,2,4-thiadiazol-3-yl)acetamido}-3-cephen-3-ylmethyl]pyridinium-4-carboxylate (syn isomer), mp. 175 to 180°C (dec.).

IR (Nujol):

3300, 3200, 1775, 1670, 1620, 1520 $cm^{-1}$

(8) N-[7-{2-(1-Benzyloxycarbonylethoxyimino)-2-(5-amino-1,2,4-thiadiazol-3-yl)acetamido}-3-cephem-3-ylmethyl]pyridinium-4-carboxylate (syn isomer), mp. 178 to 182°C (dec.).

IR (Nujol):

3250, 3150, 1770, 1670, 1620, 1520 $cm^{-1}$

(9) N-[7-{2-Ethoxycarbonylmethoxyimino-2-(5-amino-1,2,4-thiadiazol-3-yl)acetamido}-3-cephem-3-ylmethyl]pyridinium-4-carboxylate (syn isomer), mp. 184 to 188°C (dec.).

IR (Nujol):

3400—3100, 1770, 1670, 1610, 1520 $cm^{-1}$

(10) N-[7-{2-(2-Cyclohexen-1-yloxyimino)-2-(5-amino-1,2,4-thiadiazol-3-yl)acetamido}-3-cephem 3-ylmethyl]pyridinium-4-carboxylate (syn isomer), mp. 150 to 155°C (dec.).

IR (Nujol):

3300, 3200, 1775, 1660, 1610, 1520 $cm^{-1}$

(11) N-[7-{2-Carboxymethoxyimino-2-(5-amino-1,2,4-thiadiazol-3-yl)acetamido}-3-cephem-3-ylmethyl]-4'-carbamoylpyridinium-4-carboxylate (syn isomer), mp. 175 to 180°C (dec.).

IR (Nujol):

3350, 3200, 1775, 1680, 1615, 1565, 1525 $cm^{-1}$

NMR (DMSO-$d_6$-$D_2O$, δ):

3.23, 3.55 (2H, ABq, J=18Hz), 4.67 (2H, s), 5.10 (1H, d, J=5Hz), 5.35, 5.72 (2H, ABq, J=15Hz), 5.80 (1H, d, J=5Hz), 8.43 (2H, d, J=6Hz), 9.38 (2H, d, J=6Hz)

(12) N-[7-{2-Methoxycarbonylmethoxyimino-2-(5-amino-1,2,4-thiadiazol-3-yl)acetamido}-3-cephem-3-ylmethyl]pyridinium-4-carboxylate (syn isomer), mp. 165 to 170°C (dec.).

IR (Nujol):

3300—3150, 1760, 1670, 1620, 1520 $cm^{-1}$

(13) N-[7-{2-(1-Methyl-1-carboxyethoxyimino)-2-(5-amino-1,2,4-thiadiazol-3-yl)acetamido}-3-cephem-3-ylmethyl]pyridinium-4-carboxylate (syn isomer), white powder, mp. 176 to 180°C (dec.).

IR (Nujol):

3400—3150, 1770, 1670, 1620, 1520 $cm^{-1}$

(14) N-[7-{2-(1-Methyl-1-ethoxycarbonylethoxyimino)-2-(5-amino-1,2,4-thiadiazol-3-yl)acetamido}-3-cephem-3-ylmethyl]pyridinium-4-carboxylate (syn isomer), white powder, mp. 164 to 168°C (dec.).

IR (Nujol):

3350—3150, 1770, 1720, 1670, 1620, 1520 $cm^{-1}$

(15) N-[7-{2-(1-Methyl-1-t-butoxycarbonylethoxyimino)-2-(5-amino-1,2,4-thiadiazol-3-yl)acetamido}-3-cephem-3-ylmethyl]pyridinium-4-carboxylate (syn isomer), mp. 176 to 180°C (dec.).

IR (Nujol):

3300, 3200, 1780, 1730, 1680, 1620, 1520 $cm^{-1}$

(16) N-(7-(2-(2-Cyclopenten-1-yl-oxyimino)-2-(5-amino-1,2,4-thiadiazol-3-yl)acetamido)-3-cephem-3-ylmethyl)-4'-carbamoyl-pyridinium-4-carboxylate (syn isomer), mp. 155 to 160°C (DEC.).

IR (Nujol):

3300, 3150, 1770, 1675, 1610, 1560, 1520 $cm^{-1}$

NMR ($DMSO-d_6$, $\delta$):

2.0—2.4 (4H, m), 3.17—3.67 (2H, m), 5.08 (1H, d, J=5Hz), 5.23—6.30 (6H, m), 8.27 (2H, broad s), 8.57 (2H, d, J=7Hz), 9.53 (1H, d, J=8Hz), 9.70 (2H, d, J=7Hz)

(17) N-(7-(2-(1-Methyl-1-carboxyethoxyimino)-2-(5-amino-1,2,4-thiadiazol-3-yl)acetamido)-3-cephem-3-ylmethyl)-4'-carbamoyl-pyridinium-4-carboxylate (syn isomer), mp. 180 to 185°C (DEC.).

IR (Nujol):

3300, 1770, 1680, 1620, 1560, 1520 $cm^{-1}$

(18) 7-[2-Ethoxyimino-2-(5-amino-1,2,4-thiadiazol-3-yl)acetamido]-3-(1-pyridiniomethyl)-3-cephem-4-carboxylate (syn isomer), mp. 155 to 165°C (dec.).

IR (Nujol):

3400—3150, 1770, 1660, 1610, 1530 $cm^{-1}$

(19) 7-[2-Propoxyimino-2-(5-amino-1,2,4-thiadiazol-3-yl)acetamido]-3-(1-pyridiniomethyl)-3-cephem-4-carboxylate (syn isomer), mp. 230 to 240°C (dec.).

IR (Nujol):

3400—3200, 1770, 1670—1600, 1530 $cm^{-1}$

(20) 7-[2-Methoxyimino-2-(5-amino-1,2,4-thiadiazol-3-yl)acetamido]-3-(1-pyridiniomethyl)-3-cephem-4-carboxylate (syn isomer), mp. 250 to 260°C (dec.).

IR (Nujol):

3400—3100, 1770, 1650, 1610, 1520 $cm^{-1}$

(21) 7-[2-Isopropoxyimino-2-(5-amino-1,2,4-thiadiazol-3-yl)acetamido]-3-(1-pyridiniomethyl)-3-cephem-4-carboxylate (syn isomer), mp. 160 to 165°C (dec.).

IR (Nujol):

3270, 3180, 1770, 1660, 1610, 1525 $cm^{-1}$

(22) 7-[2-Allyloxyimino-2-(5-amino-1,2,4-thiadiazol-3-yl)acetamido]-3-(1-pyridiniomethyl)-3-cephem-4-carboxylate (syn isomer), mp 160 to 165°C (dec.).

IR (Nujol):

3290, 3180, 1770, 1660, 1610, 1525 $cm^{-1}$

(23) 7-[2-(2-Propynyloxyimino)-2-(5-amino-1,2,4-thiadiazol-3-yl)acetamido]-3-(1-pyridiniomethyl)-3-cephem-4-carboxylate (syn isomer), mp 145 to 150°C (dec.).

IR (Nujol):

3250, 2100, 1770, 1660, 1630, 1610, 1525 $cm^{-1}$

(24) 7-[2-Hydroxyimino-2-(5-amino-1,2,4-thiadiazol-3-yl)acetamido]-3-(1-pyridiniomethyl)-3-cephem-4-carboxylate (syn isomer), mp 170 to 175°C (dec.).

IR (Nujol):

3350, 3200, 1780, 1620, 1530, 1490 $cm^{-1}$

(25) 7-[2-Methylthiomethoxyimino-2-(5-amino-1,2,4-thiadiazol-3-yl)acetamido]-3-(1-pyridiniomethyl)-3-cephem-4-carboxylate (syn isomer), mp 195 to 205°C (dec.).

IR (Nujol):

3350—3150, 1770, 1670, 1620, 1520, 1150 $cm^{-1}$

(26)7-[2-Trityloxyimino-2-(5-amino-1,2,4-thiadiazol-3-yl)acetamido]-3-(1-pyridiniomethyl)-3-cephem-4-carboxylate (syn isomer), mp 165 to 170°C (dec.).

IR (Nujol):

3450, 1780, 1670, 1620, 1530, 1490 $cm^{-1}$

(27) 7-[2-(2,2,2-Trifluoroethoxyimino-2-(5-amino-1,2,4-thiadiazol-3-yl)acetamido]-3-(1-pyridiniomethyl)-3-cephem-4-carboxylate (syn isomer), mp 150 to 155°C (dec.).

IR (Nujol):

3300, 1780, 1675, 1630, 1530 $cm^{-1}$

(28) 7-[2-Ethoxyimino-2-(5-amino-1,2,4-thiadiazol-3-yl)acetamido]-3-4-carbamoyl-1-pyridiniomethyl)-3-cephem-4-carboxylate (syn isomer), mp 160 to 165°C (dec.).

IR (Nujol):

3300, 3200, 1780, 1680, 1620, 1570, 1530 $cm^{-1}$

NMR ($DMSO-d_6+D_2O$, $\delta$):

1.33 (3H, t, J=7Hz), 3.33, 3.67 (2H, ABq, J=18Hz), 4.35 (2H, q, J=7Hz), 5.30 (1H, d, J=4Hz), 5.47, 5.67 (2H, ABq, J=14Hz), 5.90 (1H, d, J=4Hz), 8.40 (2H, d, J=7Hz), 9.17 (2H, d, J=7Hz)

(29) 7-[2-(2-Oxotetrahydrofuran-3-yloxyimino)-2-(5-amino-1,2,4-thiadiazol-3-yl)acetamido]-3-(1-pyridiniomethyl)-3-cephem-4-carboxylate (syn isomer), mp 140 to 145°C (dec.).

IR (Nujol):

3350, 1780, 1670, 1620, 1530, 1490 $cm^{-1}$

(30) 7-[2-Methoxyimino-2-(5-amino-1,2,4-thiadiazol-3-yl)acetamido]-3-(4-carbamoyl-1-pyridiniomethyl)-3-cephem-4-carboxylate (syn isomer), mp 165 to 170°C (dec.).

IR (Nujol):

3350, 3200, 1780, 1690, 1610, 1570, 1530 $cm^{-1}$

NMR ($D_2O$, $\delta$):

3.33, 3.67 (2H, ABq, J=18Hz), 4.07 (3H, s), 5.30 (1H, d, J=4Hz), 5.47, 5.67 (2H, ABq, J=14Hz), 5.90 (1H, d, J=4Hz), 8.40 (2H, d, J=7Hz), 9.17 (2H, d, J=7Hz)

(31) 7-[2-Propoxyimino-2-(5-amino-1,2,4-thiadiazol-3-yl)acetamido]-3-(4-carbamoyl-1-pyridiniomethyl)-3-cephem-4-carboxylate (syn isomer), mp 170 to 175°C (dec.).

IR (Nujol):

3350, 3200, 1780, 1690, 1610, 1570, 1530 $cm^{-1}$

NMR ($D_2O$, $\delta$):

0.95 (3H, t, J=7Hz), 1.5—2.0 (2H, m), 3.33, 3.68 (2H, ABq, J=17Hz), 4.28 (2H, t, J=7Hz), 5.33 (1H, d, J=4Hz), 5.47, 5.70 (2H, ABq, J=14Hz), 5.92 (1H, d, J=4Hz), 8.42 (2H, d, J=7Hz), 9.17 (2H, d, J=7Hz)

(32) 7-[2-Isopropoxyimino-2-(5-amino-1,2,4-thiadiazol-3-yl)acetamido]-3-(4-carbamoyl-1-pyridiniomethyl)-3-cephem-4-carboxylate (syn isomer), mp 155 to 160°C (dec.).

IR (Nujol):

3350, 3220, 1780, 1680, 1615, 1570, 1530 $cm^{-1}$

NMR ($DMSO-d_6+D_2O$, $\delta$):

1.22 (6H, d, J=6Hz), 3.17, 3.48 (2H, ABq, J=18Hz), 4.1—4.6 (1H, m), 5.03 (1H, d, J=5Hz), 5.25, 5.63 (2H, ABq, J=14Hz), 5.70 (1H, d, J=5Hz), 8.40 (2H, d, J=6Hz), 9.45 (2H, d, J=6Hz)

(33) 7-[2-Allyloxyimino-2-(5-amino-1,2,4-thiadiazol-3-yl)acetamido]-3-(4-carbamoyl-1-pyridiniomethyl)-3-cephem-4-carboxylate (syn isomer), mp 161 to 165°C (dec.).

IR (Nujol):

3400—3150, 1770, 1670, 1610, 1560, 1520 $cm^{-1}$

NMR ($DMSO-d_6+D_2O$, $\delta$):

3.09, 3.50 (2H, ABq, J=18Hz), 4.5—4.7 (2H, m), 4.9—5.4 (4H, m), 5.06 (1H, d, J=5Hz), 5.6—6.1 (1H, m), 5.71 (1H, d, J=5Hz), 8.43 (2H, d, J=6Hz), 9.50 (2H, d, J=6Hz)

(34) 7-[2-(2,2,2-Trifluoroethoxyimino-2-(5-amino-1,2,4-thiadiazol-3-yl)acetamido]-3-(4-carbamoyl-1-pyridiniomethyl)-3-cephem-4-carboxylate (syn isomer), mp 160 to 165°C (dec.).

IR (Nujol):

3300, 3150, 1780, 1680, 1610, 1580, 1520 $cm^{-1}$

NMR ($D_2O$, $\delta$):

3.30, 3.67 (2H, ABq, J=17Hz), 4.73, 4.97 (2H, ABq, J=8Hz), 5.30 (1H, d, J=4Hz), 5.47, 5.67 (2H, ABq, J=14Hz), 5.92 (1H, d, J=4Hz), 8.40 (2H, d, J=7Hz), 9.20 (2H, d, J=7Hz)

(35) 7-[2-Methylthiomethoxyimino-2-(5-amino-1,2,4-thiadiazol-3-yl)acetamido]-3-(4-carbamoyl-1-pyridiniomethyl)-3-cephem-4-carboxylate (syn isomer), mp 160 to 165°C (dec.).

IR (Nujol):

3300, 3150, 1770, 1680, 1610, 1560, 1520 $cm^{-1}$

NMR ($DMSO-d_6+D_2O$, $\delta$):

2.23 (3H, s), 3.15, 3.67 (2H, ABq, J=18Hz), 5.17 (1H, d, J=5Hz), 5.32 (2H, s), 5.00—5.57 (2H, m), 5.80 (1H, d, J=5Hz), 8.68 (2H, d, J=6Hz), 9.50 (2H, dl J=6Hz)

(36) 7-[2-(2-Propynyloxyimino)-2-(5-amino-1,2,4-thiadiazol-3-yl)acetamido]-3-(4-carbamoyl-1-pyridiniomethyl)-3-cephem-4-carboxylate (syn isomer), mp 155 to 160°C (dec.).

IR (Nujol):

3400, 3250, 3150, 2120, 1770, 1685, 1610, 1560, 1525 $cm^{-1}$

NMR ($DMSO-d_6+D_2O$, $\delta$):

3.23, 3.58 (2H, ABq, J=18Hz), 3.45 (1H, t, J=2Hz), 4.80 (2H, d, J=2Hz), 5.13 (1H, d, J=5Hz), 5.35, 5.72 (2H, ABq, J=14Hz), 5.78 (1H, d, J=5Hz), 8.47 (2H, d, J=7Hz), 9.50 (2H, d, J=7Hz)

Example 8

7 - [2 - Cyclopentyloxyimino - 2 - (5 - amino - 1,2,4 - thiadiazol - 3 - yl)acetamido] - 3 - acetoacetoxymethyl - 3 - cephem - 4 - carboxylic acid (syn isomer) was reacted with 2 - methyl - 5 - oxo - 6 - hydroxy - 2,5 - dihydro - 1,2,4 - triazine - 3 - thiol according to similar manners to those of Examples 5 and 6 to give sodium 7- [2 - cyclopentyloxyimino - 2 - (5 - amino - 1,2,4 - thiadiazol - 3 - yl)acetamido] - 3 - (2 - methyl - 5 - oxo - 6 - hydroxy - 2,5 - dihydro - 1,2,4 - triazin - 3 - yl)thiomethyl - 3 - cephem - 4 - carboxylate (syn isomer), mp 169 to 174°C (dec.).

IR (Nujol):

3600—3100, 1760, 1690, 1665, 1640, 1610, 1520, 1005 $cm^{-1}$

NMR ($D_2O+NaHCO_3$, $\delta$):

1.3—2.1 (8H, m), 3.63 (3H, s), 3.4—3.9 (2H, m), 4.08, 4.40 (2H, ABq, J=14Hz), 4.7—5.1 (1H, m), 5.22 (1H, d, J=5Hz), 5.80 (1H, d, J=5Hz)

Example 9

The following compounds were obtained according to similar manners to those of Examples 5, 6 and 8.

(1) Disodium 7-[2-methoxyimino-2-(5-amino-1,2,4-thiadiazol-3-yl)acetamido]-3-(2-methyl-5-oxo-6-oxido-2,5-dihydro-1,2,4-triazin-3-yl)thiomethyl-3-cephem-4-carboxylate (syn isomer), mp 220 to 225°C (dec.).

IR (Nujol):

3400—3150, 1760, 1660, 1640—1560, 1520, 1040 $cm^{-1}$

NMR ($D_2O$, $\delta$):

3.64 (3H, s), 3.48, 3.78 (2H, ABq, J=18Hz), 4.08 (3H, s), 4.00—4.56 (2H, m), 5.20 (1H, d, J=5Hz), 5.82 (1H, d, J=5Hz)

(2) Disodium 7-[2-ethoxyimino-2-(5-amino-1,2,4-thiadiazol-3-yl)acetamido]-3-(2-methyl-5-oxo-6-oxido-2,5-dihydro-1,2,4-triazin-3-yl)thiomethyl-3-cephem-4-carboxylate (syn isomer), mp 255 to 265°C (dec.).

IR (Nujol):

3400—3150, 1760, 1660, 1600, 1500, 1400, 1030 $cm^{-1}$

NMR ($D_2O$, $\delta$):

1.35 (3H, t, J=7Hz), 3.42, 3.80 (2H, ABq, J=18Hz), 3.65 (3H, s), 4.07, 4.43 (2H, ABq, J=13Hz), 4.38 (2H, q, J=7Hz), 5.22 (1H, d, J=5Hz), 5.83 (1H, d, J=5Hz)

(3) 7-[2-(2-Cyclopenten-1-yloxyimino)-2-(5-amino-1,2,4-thiadiazol-3-yl)acetamido]-3-(2-methyl-5-oxo-6-hydroxy-2,5-dihydro-1,2,4-triazin-3-yl)thiomethyl-3-cephem-4-carboxylic acid (syn isomer), mp 158 to 164°C (dec.).

IR (Nujol):

3450—3150, 1770, 1680, 1630, 1510, 1260, 1180, 1100, 1030, 1010 $cm^{-1}$

(4) 7-[2-Isopropoxyimino-2-(5-amino-1,2,4-thiadiazol-3-yl)acetamido]-3-(2-methyl-5-oxo-6-hydroxy-2,5-dihydro-1,2,4-triazin-3-yl)thiomethyl-3-cephem-4-carboxylic acid (syn isomer), mp 160 to 167°C (dec.).

IR (Nujol):

3400, 3280, 3180, 1780, 1770, 1630, 1515, 1410, 1240, 1009 $cm^{-1}$

(5) 7-[2-(2-Propynyloxyimino-2-(5-amino-1,2,4-thiadiazol-3-yl)acetamido]-3-(2-methyl-5-oxo-6-hydroxy-2,5-dihydro-1,2,4-triazin-3-yl)thiomethyl-3-cephem-4-carboxylic acid (syn isomer), mp 161 to 166°C (dec.).

IR (Nujol):

3260, 3180, 1770, 1670, 1620, 1520, 1335 $cm^{-1}$

(6) 7-[2-Allyloxyimino-2-(5-amino-1,2,4-thiadiazol-3-yl)acetamido]-3-(2-methyl-5-oxo-6-hydroxy-2,5-dihydro-1,2,4-triazin-3-yl)thiomethyl-3-cephem-4-carboxylic acid (syn isomer), mp 169 to 173°C (dec.).

IR (Nujol):
3360, 3210, 1775, 1670, 1625, 1560, 1520, 1250, 1175, 1100, 1020 $cm^{-1}$

Example 10

To a solution of N - [7 - {2 - t - butoxycarbonylmethoxyimino - 2 - (5 - amino - 1,2,4 - thiadiazol - 3 - yl)acetamido} - 3 - cephem - 3 - ylmethyl]pyridinium - 4 - carboxylate (syn isomer) (1.8 g in formic acid (18 ml) was added conc. hydrochloric acid (0.5 ml) and the mixture was stirred for one hour at room temperature. The solvent was distilled off under reduced pressure and the residue was pulverized with acetone, collected by filtration, washed with acetone and diisopropyl ether to give a powder. The powder was dissolved in water (5 ml) and subjected to column chromatography on a non ionic adsorption resin Diaion HP 20 (Trademark, prepared by Mitsubishi Chemical Industries (50 ml). After the column was washed with water (500 ml), the elution was carried out with 40% aqueous methanol. The eluates containing an object compound were collected, evaporated to remove methanol under reduced pressure and lyophilized to give white powder of N - [7 - {2 - carboxymethoxyimino - 2 - (5 - amino - 1,2,4 - thiadiazol - 3 - yl)acetamido} - 3 - cephem - 3 - ylmethyl] - pyridinium - 4 - carboxylate (syn isomer) (800 mg), mp. 150 to 155°C (dec.).

IR (Nujol):
3350, 3200, 1780, 1680, 1530 $cm^{-1}$

NMR ($D_2O+NaHCO_3$, $\delta$):
3.27 and 3.63 (2H, ABq, J=18Hz), 4.70 (2H, s), 5.30 (1H, d, J=4Hz), 5.40 and 5.60 (2H, ABq, J=14Hz), 5.93 (1H, d, J=4Hz), 8.0—9.1 (5H, m)

Example 11

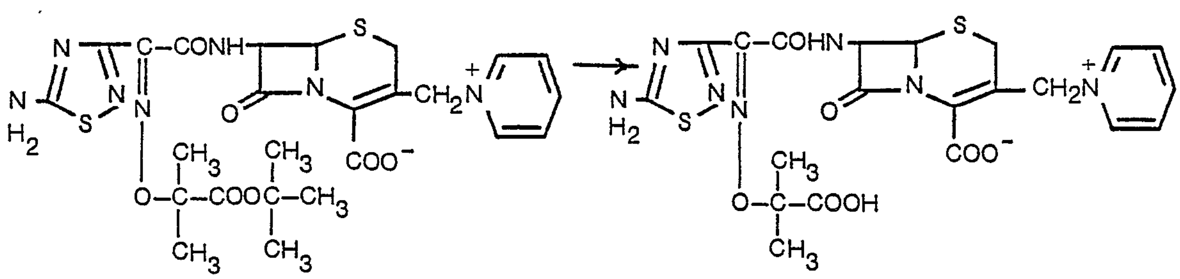

To a cold mixture of trifluoroacetic acid (22 ml) and anisole (4.4 ml) was added N - [7 - {2 - (1 - methyl - 1 - t - butoxycarbonylethoxyimino) - 2 - (5 - amino - 1,2,4 - thiadiazol - 3 - yl)-acetamido} - 3 - cephem - 3 - ylmethyl]pyridinium - 4 - carboxylate (3.18 g) and the mixture was stirred for 40 minutes at room temperature. The mixture was evaporated to remove trifluoroacetic acid and the residue was triturated with isopropyl ether to give a yellowish powder. The powder was dissolved in an aqueous sodium bicarbonate, adjusted to pH 1 with 6N hydrochloric acid and washed with ethyl acetate. The aqueous solution was subjected to a column chromatography on a non ionic adsorption resin, Diaion HP—20 (140 ml). After the column was washed with water, the elution was carried out with 5% and 10% aqueous isopropyl alcohol. The eluates containing an object compound were collected, evaporated to remove isopropyl alcohol under reduced pressure and lyophilized to give N - [7 - {2 - (1 - methyl - 1 - carboxyethoxyimino) - 2 - (5 - amino - 1,2,4 - thiadiazol - 3 - yl)acetamido} - 3 - cephem - 3 - ylmethyl]pyridinium - 4 - carboxylate (syn isomer) (2.20 g), white powder, mp. 176 to 180°C (dec.).

IR (Nujol):
3400—3150, 1770, 1670, 1620, 1520 $cm^{-1}$

NMR ($DMSO-d_6+D_2O$, $\delta$):
1.48 (6H, s), 3.10, 3.62 (2H, ABq, J=18Hz), 5.12 (1H, d, J=5Hz), 5.45 (2H, m), 5.78 (1H, d, J=5Hz), 8.13 (2H, m), 8.58 (1H, m), 9.38 (2H, m)

Example 12

The following compounds were prepared according to the similar manners to those of Examples 10 and 11.

(1) N-[7-{2-(1-Carboxyethoxyimino)-2-(5-amino-1,2,4-thiadiazol-3-yl)acetamido}-3-cephem-3-ylmethyl]-4'-carbamoylpyridinium-4-carboxylate (syn isomer), mp.170 to 175°C (dec.).

IR (Nujol):
3300, 3160, 1770, 1680, 1610, 1560, 1520 $cm^{-1}$

(2) N-[7-{2-(1-Carboxyethoxyimino)-2-(5-amino-1,2,4-thiadiazol-3-yl)acetamido}-3-cephem-3-ylmethyl]pyridinium-4-carboxylate (syn isomer), mp. 175 to 180°C (dec.).

IR (Nujol):
3300, 3200, 1775, 1670, 1620, 1520 $cm^{-1}$
NMR ($D_2O$+$NaHCO_3$, $\delta$):
1.50 (3H, d, J=7Hz), 3.25 and 3.67 (2H, ABq, J=18Hz), 4.40—4.90 (1H, m), 5.32 (1H, d, J=5Hz), 5.42 and 5.60 (2H, ABq, J=15Hz), 5.83—6.00 (1H, m), 7.9—9.1 (5H, m)

(3) N-[7-{2-Carboxymethoxyimino-2-(5-amino-1,2,4-thiadiazol-3-yl)acetamido}-3-cephem-3-ylmethyl]4'-carbamoylpyridinium-4-carboxylate (syn isomer), mp. 175 to 180°C (dec.).
IR (Nujol):
3350, 3200, 1775, 1680, 1615, 1565, 1525 $cm^{-1}$

(4) N-[7-{2-(1-Methyl-1-carboxyethoxyimino)-2-(5-amino-1,2,4-thiadiazol-3-yl)acetamido}-3-cephem-3-ylmethyl]-4'-carbamoylpyridinium-4-carboxylate (syn isomer), mp. 180 to 185°C (dec.).
IR (Nujol):
3300, 1770, 1680, 1620, 560, 1520 $cm^{-1}$

Example 13

A mixture of 7 - [2 - trityloxyimino - 2 - (5 - amino - 1,2,4 - thiadiazol - 3 - yl)-acetamido] - 3 - (1 - pyridiniomethyl) - 3 - cephem - 4 - carboxylate (syn isomer) (5.55 g) and concentrated hydrochloric acid (2.3 ml) in formic acid (60 ml) was stirred for two hours at ambient temperature. After an insoluble material was filtered off, the filtrate was evaporated to dryness and the residue was pulverized with acetone and collected by filtration. The powder was dissolved in water (13 ml) and subjected to column chromatography on a non-ionic adsorption resin Diaion HP20 (Trademark, prepared by Mitsubishi Chemical Industries) (100 ml), using water as an eluent. The eluates containing an object compound were collected and lyophilized to give yellowish white powder of 7 - [2 - hydroxyimino - 2 - (5 - amino - 1,2,4 - thiadiazol - 3 - yl)acetamido] - 3 - (1 - pyridiniomethyl)-3 - cephem - 4 - carboxylate (syn isomer) (675 mg), mp 170 to 175°C (dec.).
IR (Nujol):
3350, 3200, 1780, 1620, 1530, 1490 $cm^{-1}$
NMR (DMSO-$d_6$+$D_2O$, $\delta$):
3.14, 3.54 (2H, ABq, J=18Hz), 5.08 (1H, d, J=5Hz), 5.28, 5.62 (2H, ABq, J=12Hz), 5.86 (1H, d, J=5Hz), 7.96—8.24 (2H, m), 8.40—8.68 (1H, m), 9.16—9.42 (2H, m)

Example 14

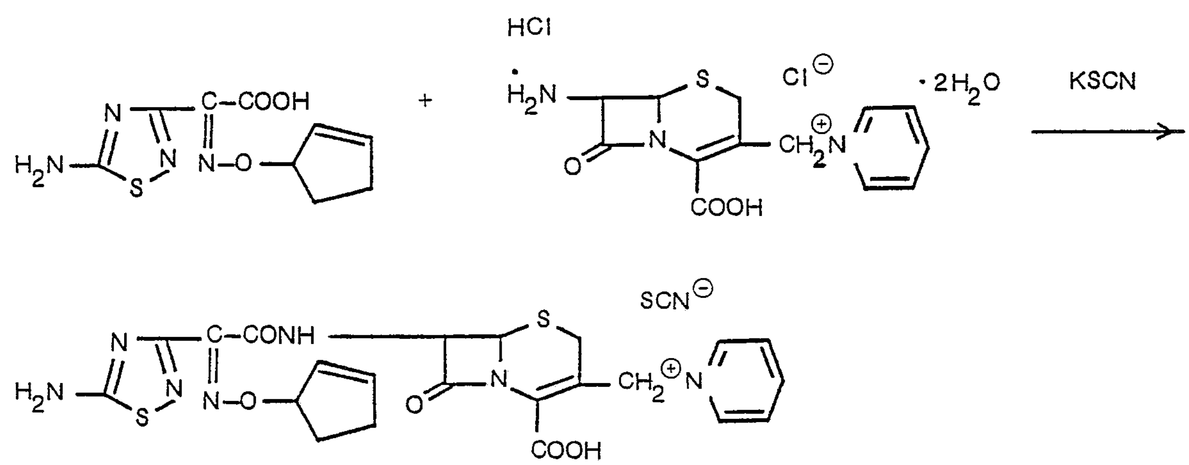


To a cold solution of phosphorus pentachloride (20.8 g) in methylene chloride (375 ml) was added 2 - (2 - cyclopenten - 1 - yloxyimino) - 2 - (5 - amino - 1,2,4 - thiadiazol - 3 - yl)acetic acid (syn isomer) (25.4 g) at —18°C and the mixture was stirred for 40 minutes at —12 to —10°C. To the reaction mixture was added diisopropyl ether (1.2 l) below —10°C under stirring, which was continued until the mixture was warmed to ambient temperature. The resulting precipitates were collected by filtration, washed with diisopropyl ether and then kept in a desiccator for several minutes. On the other hand, a mixture of 1 - [(7 - amino - 4 - carboxy - 3 - cephem - 3 - yl)methyl]-pyridinium chloride hydrochloride dihydrate (30.77 g) and trimethylsilylacetamide (154.5 g) in methylene chloride (800 ml) was warmed at 35°C to make a solution, which was cooled to —18°C. To the cold solution were added the precipitates prepared above and the mixture was stirred for 30 minutes at —12 to —10°C. A solution of sodium bicarbonate (26 g) in water (400 ml) was added to the reaction mixture and the aqueous layer was separated out, adjusted to pH 1.5 with 6N hydrochloric acid, and washed with ethyl acetate. The aqueous solution was adjusted to pH 4 with an aqueous

solution of sodium bicarbonate and passed through a column packed with acidic alumina (117 g). To the eluate (1.2 l) were added potassium thiocyanate (56.2 g) and sodium chloride (171.5 g) and then the mixture was adjusted to pH 2.6 with 1N hydrochloric acid under cooling in an ice-bath. After an insoluble material was filtered off, sodium chloride (171.5 g) was added to the filtrate and the solution was adjusted to pH 1.6 with 1N hydrochloric acid under stirring and cooling in an ice-bath. The resulting precipitates were filtered washed with cold water (2 x 150 ml) and dried to give 1 - [[7 - {2 - (2 - cyclopenten - 1 - yloxyimino) - 2 - (5 - amino - 1,2,4 - thiadiazol - 3 - yl)acetamidô5K - 4 - carboxy - 3 - cephem - 3 - yl]methyl]pyridinium thiocyanate (syn isomer) (28.1 g) mp 151 to 156°C (dec.).

IR (Nujol):
2050, 1780, 1670, 1630, 1610, 1530 $cm^{-1}$

NMR ($DMSO-d_6+D_2O$, $\delta$):
1.6—2.7 (4H, m), 3.33, 3.66 (2H, ABq, J=18Hz), 5.20 (1H, d, J=5Hz), 4.9—6.3 (5H, m), 5.86 (1H, d, J=5Hz), 8.2 (2H, m), 8.7 (1H, m), 9.15 (2H, m)

### Example 15

1 - [[7 - {2 - (2 - Cyclopenten - 1 - yloxyimino) - 2 - (5 - amino - 1,2,4 - thiadiazol - 3 - yl)acetamido} - 4 - carboxy - 3 - cephem - 3 - yl]methyl]pyridinium iodide (syn isomer) was obtained according to a similar manner to that of Example 14 by using sodium iodide instead of potassium thiocyanate.

IR (Nujol):
3400—3100, 1775, 1670, 1620, 1520 $cm^{-1}$

NMR ($DMSO-d_6+D_2O$, $\delta$):
1.6—2.8 (4H, m), 3.35, 3.80 (2H, ABq, J=19Hz), 5.31 (1H, d, J=5Hz), 5.93 (1H, d, J=5Hz), 5.0—6.4 (5H, m), 8.28 (2H, m), 8.74 (1H, m), 9.15 (2H, m)

### Example 16

To a solution of 1 - [[7 - {2 - (2 - cyclopenten - 1 - yloxyimino) - 2 - (5 - amino - 1,2,4 - thiadiazol - 3 - yl)acetamido} - 4 - carboxy - 3 - cephem - 3 - yl]methyl]pyridinium thiocyanate (syn isomer) (11.7 g) in dimethylformamide (30 ml) was added a solution of lithium chloride (1.7 g) in methanol (20 ml) under stirring, which was continued for 10 minutes at ambient temperature. An insoluble material was filtered, washed with dimethylformamide (6 ml) and then the filtrate and the washings were combined. The combined solution was added to acetone (300 ml) under stirring, which was continued for five minutes at ambient temperature. The resulting precipitates were filtered, washed with acetone (40 ml x 3) and dried in vacuo to give 1 - [[7 - {2 - (2 - cyclopenten -1 - yloxyimino) - 2 - (5 - amino - 1,2,4 - thiadiazol - 3 - yl)acetamido} - 4 - carboxy - 3 - cephem - 3 - yl]-methyl]pyridinium chloride (syn isomer) (11.0 g).

IR (Nujol):
3400—3100, 1780, 1660, 1630, 1530 $cm^{-1}$

NMR ($DMSO-d_6$, $\delta$):
1.6—2.6 (4H, m), 3.39, 3.61 (2H, ABq, J=18Hz), 5.19 (1H, d, J=5Hz), 4.9—5.6 (2H, m), 5.64 (1H, broad s), 5.81 (1H, dd, J=5 and 8Hz), 5.7—6.2 (2H, m), 8.20 (2H, m), 8.64 (1H, m), 9.22 (2H, m), 9.48 (1H, d, J=8Hz)

### Example 17

To a solution of sodium iodide (10 g) and pyridine (1.28 g) in formamide (8 ml) was added sodium 7 - [2 - ethoxyimino - 2 - (5 - amino - 1,2,4 - thiadiazol - 3 - yl)acetamido] - cephalosporanate (syn isomer) (4.0 g) at 75°C under stirring, which was continued for 1.5 hours at 80 to 85°C. The mixture was cooled to ambient temperature and poured into ethanol (100 ml). A resulting precipitate was collected by filtration and an additional one was obtained from the filtrate by an addition of diisopropyl ether (100 ml). These precipitates were dissolved in water (50 ml) and the solution was adjusted to pH 3 with 6N hydrochloric acid and washed with ethyl acetate. The aqueous solution was subjected to column chromatography on a non-ionic adsorption resin Diaion HP—20 (Trademark, prepared by Mitsubishi Chemical Industries) (160 ml). After the column was washed with water, the elution was carried out with 30% aqueous methanol. The eluates containing an object compound were collected, evaporated to remove methanol under reduced pressure and lyophilized to give white powder of 7 - [2 - ethoxyimino - 2 - (5 - amino - 1,2,4 - thiadiazol - 3 - yl)acetamido] - 3 - (1 - pyridiniomethyl)-3 - cephem - 4 - carboxylate (syn isomer) (1.52 g), mp. 155 to 165°C (dec).

IR (Nujol):
3400—3150, 1770, 1660, 1610, 1530 $cm^{-1}$.

**Claims for the Contracting States: BE CH DE FR GB IT LU NL SE**

1. New cephem compounds of the formula:

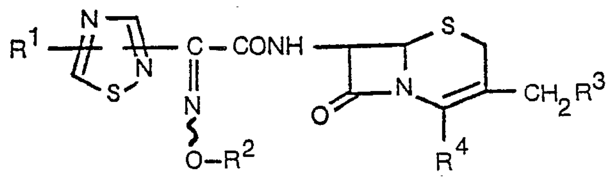

(I)

wherein

$R^1$ is amino or protected amino;

$R^2$ is hydrogen, ($C_1$—$C_6$) alkyl which may be substituted with phenyl, carboxy, esterified carboxy, ($C_1$—$C_6$) alkylthio or halogen, ($C_2$—$C_6$) alkenyl, ($C_2$—$C_6$) alkynyl, cyclo($C_3$—$C_6$)alkyl, cyclo($C_3$—$C_6$)-alkenyl, or tetrahydrofuryl substituted with an oxo group;

$R_3$ is a group of the formula:

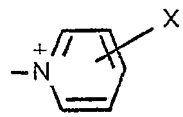

wherein X is hydrogen or carbamoyl; and

$R^4$ is $COO^-$; or

$R^3$ is 2-($C_1$—$C_6$) alkyl-5-oxo-6-hydroxy-2,5-dihydro-1,2,4-triazinylthio; and

$R^4$ is carboxy or esterified carboxy, and pharmaceutically acceptable salts thereof.

2. Syn isomer of a compound of claim 1.

3. A compound of claims 1 or 2, wherein

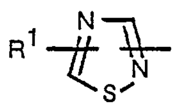 group is 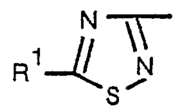 .

4. A compound of any of claims 1 to 3, wherein $R^1$ is amino; $R^2$ is hydrogen, ($C_1$—$C_6$) alkyl which may be substituted with 1 to 3 substituent(s) selected from the group consisting of halogen, ($C_1$—$C_6$) alkylthio, carboxy, esterified carboxy and aryl, ($C_2$—$C_6$) alkenyl, ($C_2$—$C_6$) alkynyl, cyclo($C_3C_6$)alkyl, cyclo($C_3$—$C_6$)alkenyl, or tetrahydrofuryl substituted with an oxo group.

5. A compound of any of claims 1 to 4, wherein $R^2$ is hydrogen, methyl, ethyl, propyl, isopropyl, 2,2,2-trifluoroethyl, methylthiomethyl, carboxymethyl, 1-methyl-1-carboxyethyl, 1-carboxyethyl, methoxycarbonylmethyl, ethoxycarbonylmethyl, 1-methyl-1-ethoxycarbonylethyl, t-butoxycarbonylmethyl, 1-t-butoxycarbonylethyl, 1-methyl-1-t-butoxycarbonylethyl, 1-benzyloxycarbonylethyl, trityl, allyl, 2-propynyl, cyclopentyl, 2-cyclopenten-1-yl, 2-cyclohexen-1-yl or 2-oxotetrahydrofuran-3-yl.

6. A compound of any of claims 1 to 5, wherein $R^3$ is a group of the formula:

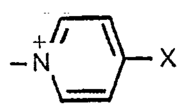

wherein X is hydrogen or carbamoyl and $R^4$ is $—COO^-$.

7. A compound of any of claims 1 to 5, wherein $R^3$ is 2-methyl-5-oxo-6-hydroxy-2,5-dihydro-1,2,4-triazinylthio and $R^4$ is carboxy.

8. A compound of any of claims 1 to 4, wherein $R^2$ is carboxy($C_{1-6}$)alkyl,(C7) alkoxycarbonyl-($C_{1-6}$)alkyl or ($C_{2-8}$)alkenyl; $R^3$ is a group of the formula:

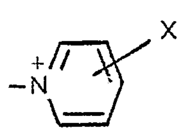

wherein X is hydrogen; and $R^4$ is $—COO^-$.

9. A compound of any of claims 1 to 4 and 8, wherein $R^2$ is carboxymethyl, t-butoxycarbonylmethyl or allyl.

10. A compound of claim 9, which is 7-[2-carboxymethoxyimino-2-(5-amino-1,2,4-thiadiazol-3-yl)-acetamido]-3-(1-pyridiniomethyl)-3-cephem-4-carboxylate (syn isomer).

11. A compound of claim 9, which is 7-[2-allyloxyimino-2-(5-amino-1,2,4-thiadiazol-3-yl)acetamido]-3-(1-pyridiniomethyl)-3-cephem-4-carboxylate (syn isomer).

12. A process for preparing new cephem compounds of the formula:

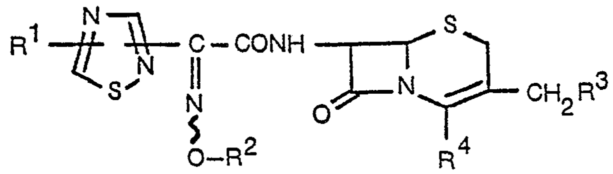

(I)

wherein

$R^1$ is amino or protected amino;

$R^2$ is hydrogen, ($C_1$—$C_6$) alkyl which may be substituted with phenyl, carboxy, esterified carboxy, ($C_1$—$C_6$) alkylthio or halogen, ($C_2$—$C_6$) alkenyl, ($C_2$—$C_6$) alkynyl, cyclo($C_3$—$C_6$)alkyl, cyclo($C_3$—$C_6$)-alkenyl, or tetrahydrofuryl substituted with an oxo group;

$R_3$ is a group of the tormula:

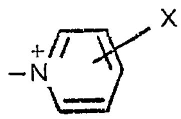

wherein X is hydrogen or carbamoyl; and

$R^4$ is $COO^-$; or

$R^3$ is 2-($C_1$—$C_6$)alkyl-5-oxo-6-hydroxy-2,5-dihydro-1,2,4-triazinylthio; and

$R^4$ is carboxy or esterified carboxy, or pharmaceutically acceptable salts thereof, which comprises reacting a compound of the formula:

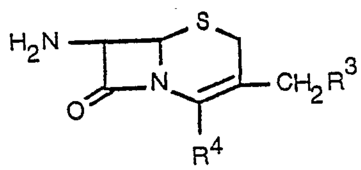

wherein $R^3$ and $R^4$ are each as defined above, or its reactive derivative at the amino group or a salt thereof, with a compound of the formula

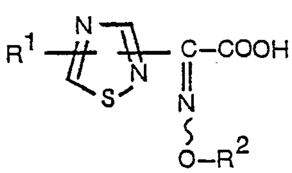

wherein $R^1$ and $R^2$ are each defined above, or its reactive derivative at the carboxy group or a salt thereof.

13. A process for preparing new cephem compounds of the formula:

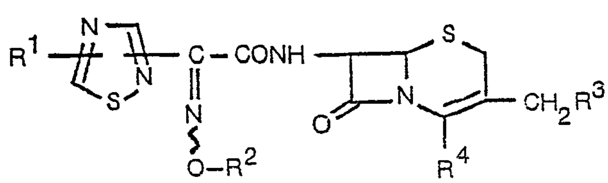

wherein

$R^1$ is amino or protected amino;

$R^2$ is hydrogen, ($C_{1-6}$) alkyl which may be substituted with phenyl, carboxy, esterified carboxy, ($C_{1-6}$) alkylthio or halogen, ($C_{2-6}$) alkenyl, ($C_{2-6}$) alkynyl, cyclo($C_{3-6}$)alkyl, cyclo($C_{1-6}$)alkenyl, or tetrahydrofuryl substituted with an oxo group;

$R_3$ is a group of the formula:

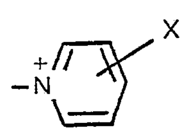

wherein X is hydrogen or carbamoyl; and

$R^4$ is $COO^-$; or

$R^3$ is 2-($C_{1-6}$)alkyl-5-oxo-6-hydroxy-2,5-dihydro-1,2,4-triazinylthio; and

$R^4$ is carboxy or esterified carboxy, or pharmaceutically acceptable salts thereof, which comprises reacting a compound of the formula:

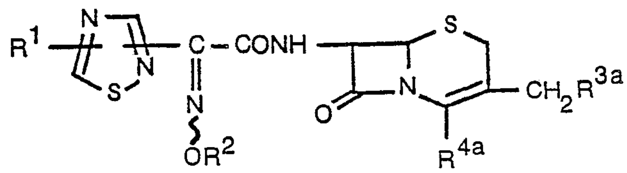

wherein $R^1$ and $R^2$ are each as defined above; $R^{3a}$ is a group which can be substituted with a group of the formula: $R^3$ wherein $R^3$ is as defined above; and $R^{4a}$ is a carboxy when $R^{3b}$ is a compound of the formula:

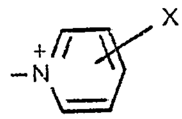

wherein X is as defined above; or $R^{4a}$ is carboxy or esterified carboxy when $R^{3b}$ is a compound of the formula: $R^{3c}$-H wherein $R^{3c}$ is 2-($C_{1-6}$)alkyl-5-oxo-6-hydroxy-2,5-dihydro-1,2,4-triazinylthio; or a salt thereof, with a compound of the formula:

$R^{3b}$

wherein $R^{3b}$ is as defined above, or its reactive derivative.

14. A process for preparing a compound of the formula:

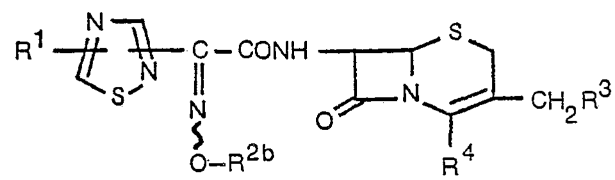

wherein

$R^1$ is amino or protected amino;

$R^{2b}$ is a carboxy($C_{1-6}$)alkyl;

$R^3$ is a group of the formula:

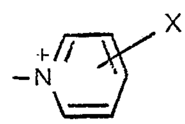

wherein X is hydrogen or carbamoyl; and

$R^4$ is —$COO^-$; or

$R^3$ is 2-($C_{1-6}$)alkyl-5-;oxo-6-hydroxy-2,5-dihydro-1,2,4-triazinylthio; and

$R^4$ is carboxy or esterified carboxy; or pharmaceutically acceptable salts thereof; which comprises subjecting a compound of the formula:

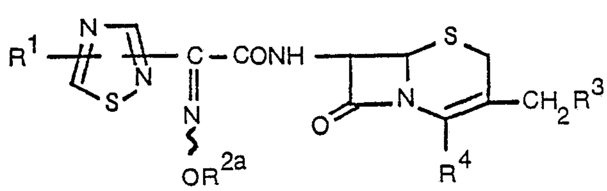

wherein $R^1$, $R^3$ and $R^4$ are each as defined above and $R^{2a}$ is an esterified carboxy($C_{1-6}$)alkyl, or a salt thereof to elimination reaction of the protective group of carboxy.

15. A process for preparing a compound of the formula:

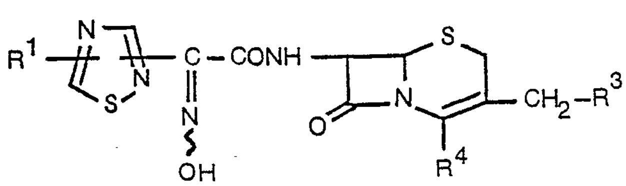

wherein

$R^1$ is amino or protected amino;

$R_3$ is a group of the formula:

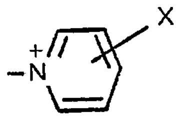

wherein X is hydrogen or carbamoyl; and

$R^4$ is $COO^-$; or

$R^3$ is 2-($C_1$—$C_6$)alkyl-5-oxo-6-hydroxy-2,5-dihydro-1,2,4-triazinylthio; and

$R^4$ is carboxy or esterified carboxy, or pharmaceutically acceptable salts thereof, which comprises subjecting a compound of the formula:

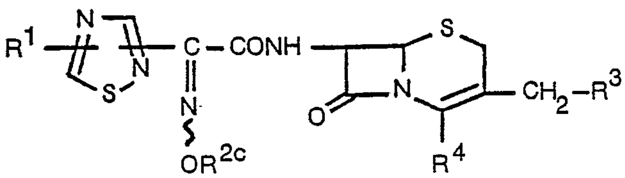

wherein $R^1$, $R^3$ and $R^4$ are each as defined above and $R^{2c}$ is a protective group of hydroxy, or a salt thereof, to elimination reaction of the protective group of hydroxy.

16. A compound of the formula:

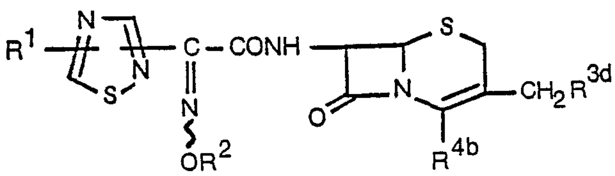

wherein

$R^1$ is amino or protected amino;

$R^2$ is hydrogen, $(C_{1-6})$ alkyl which may be substituted with phenyl, carboxy, esterified carboxy, $(C_{1-6})$ alkylthio or halogen, $(C_{2-6})$ alkenyl, $(C_{2-6})$ alkynyl, cyclo$(C_{3-6})$alkyl, cyclo$(C_{3-6})$alkenyl, or tetrahydrofuryl substituted with an oxo group;

$R^{3d}$ is lower alkanoyl$(C_{1-6})$alkanoyloxy, and $R^{4b}$ is carboxy or esterified carboxy, and a salt thereof.

17. A process for preparing a compound of the formula:

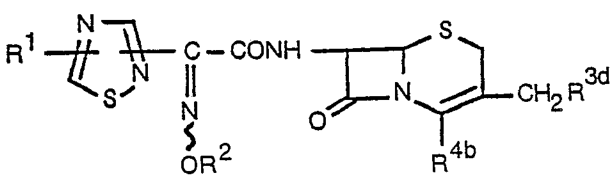

wherein

$R^1$ is amino or protected amino;

$R^2$ is hydrogen, $(C_{1-6})$ alkyl which may be substituted with phenyl, carboxy, esterified carboxy, $(C_{1-6})$ alkylthio or halogen, $(C_{2-6})$ alkenyl, $(C_{2-6})$ alkynyl, cyclo$(C_{3-6})$alkyl, cyclo$(C_{3-6})$alkenyl, or tetrahydrofuryl substituted with an oxo group;

$R^{3d}$ is $(C_{1-6})$ alkanoyl $(C_{1-6})$ alkanoyloxy; and

$R^{4b}$ is carboxy or esterified carboxy; or a salt thereof, which comprises a) reacting a compound of the formula:

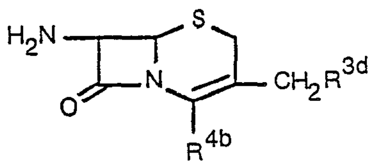

wherein $R^{3d}$ and $R^{4b}$ are each as defined above, or its reactive derivative at the amino group or a salt thereof, with a compound of the formula:

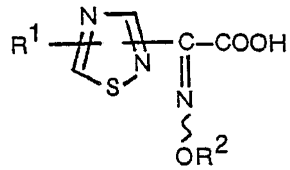

wherein $R^1$ and $R^2$ are each as defined above, or its reactive derivative at the carboxy group or a salt thereof, or b) subjecting a compound of the formula

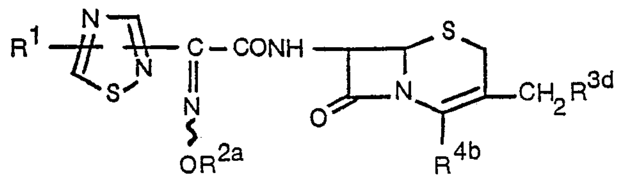

wherein $R^1$, $R^{3d}$ and $R^{4b}$ are each as defined above and $R^{2a}$ is a protected carboxy($C_{1-6}$)alkyl, or a salt thereof, to elimination reaction of the protective group of carboxy, to give a compound of the formula:

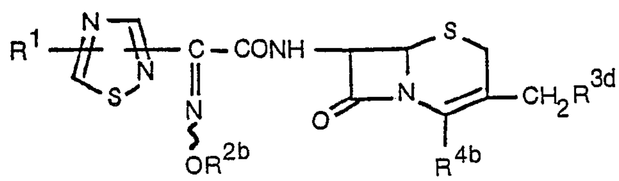

wherein $R^1$, $R^{3d}$ and $R^{4b}$ are each as defined above and $R^{2b}$ is a carboxy($C_{1-6}$)alkyl, or a salt thereof.

18. A pharmaceutical antibacterial composition comprising a compound of claim 1 in association with a pharmaceutically acceptable, substantially non-toxic carrier or excipient.

19. A compound of formula I for use as a medicament.

20. A compound of formula I for use in treating infectious diseases.

**Claims for the Contracting State: AT**

1. A process for preparing new cephem compounds of the formula:

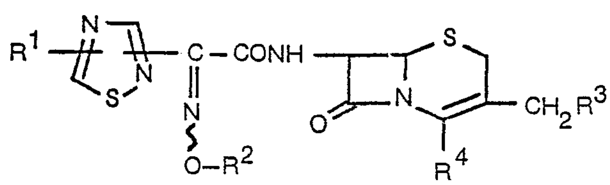

wherein

$R^1$ is amino or protected amino;

$R^2$ is hydrogen, ($C_1$—$C_6$) alkyl which may be substituted with phenyl, carboxy, esterified carboxy, ($C_1$—$C_6$) alkylthio or halogen, ($C_2$—$C_6$) alkenyl, ($C_2$—$C_6$) alkynyl, cyclo($C_3$—$C_6$)alkyl, cyclo($C_3$—$C_6$)-alkenyl, or tetrahydrofuryl substituted with an oxo group;

$R_3$ is a group of the formula:

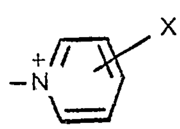

wherein X is hydrogen or carbamoyl; and

$R^4$ is $COO^-$; or

$R^3$ is 2-($C_1$—$C_6$)alkyl-5-oxo-6-hydroxy-2,5-dihydro-1,2,4-triazinylthio; and

$R^4$ is carboxy or esterified carboxy, or pharmaceutically acceptable salts thereof, which comprises reacting a compound of the formula:

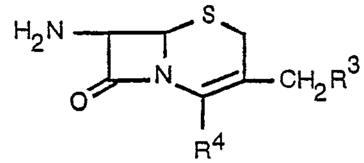

wherein $R^3$ and $R^4$ are each as defined above, or its reactive derivative at the amino group or a salt thereof, with a compound of the formula:

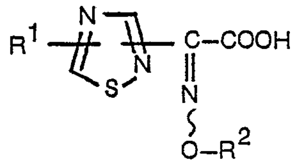

wherein $R^1$ and $R^2$ are each as defined above, or its reactive derivative at the carboxy group or a salt thereof.

2. A process for preparing new cephem compounds of the formula:

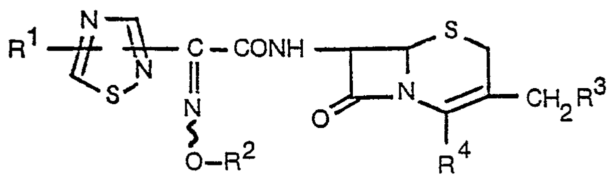

wherein

$R^1$ is amino or protected amino;

$R^2$ is hydrogen, ($C_1$—$C_6$) alkyl which may be substituted with phenyl, carboxy, esterified carboxy, ($C_1$—$C_6$) alkylthio or halogen, ($C_2$—$C_6$) alkenyl, ($C_2$—$C_6$) alkynyl, cyclo($C_3$—$C_6$)alkyl, cyclo($C_3$—$C_6$)-alkenyl, or tetrahydrofuryl substituted with an oxo group;

$R_3$ is a group of the formula:

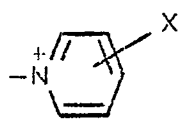

wherein X is hydrogen or carbamoyl; and

$R^4$ is $COO^-$; or

$R^3$ is 2-($C_1$—$C_6$)alkyl-5-oxo-6-hydroxy-2,5-dihydro-1,2,4-triazinylthio; and

$R^4$ is carboxy or esterified carboxy, or pharmaceutically acceptable salts thereof, which comprises reacting a compound of the formula:

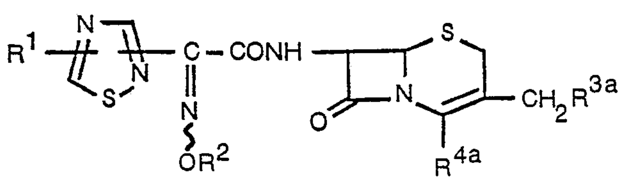

wherein $R^1$ and $R^2$ are each as defined above; $R^{3a}$ is a group which can be substituted with a group of the formula: $R^3$ wherein $R^3$ is as defined above; and $R^{4a}$ is a carboxy when $R^{3b}$ is a compound of the formula:

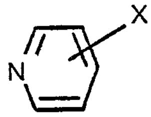

wherein X is as defined above; or $R^{4a}$ is carboxy or esterified carboxy when $R^{3b}$ is a compound of the formula: $R^{3c}$-H wherein $R^{3c}$ is 2-($C_1$—$C_6$)alkyl-5-oxo-6-hydroxy-2,5-dihydro-1,2,4-triazinylthio; or a salt thereof, with a compound of the formula:

$R^{3b}$

wherein $R^{3b}$ is as defined above, or its reactive derivative.

3. A process for preparing the syn isomer of a compound of claims 1 or 2.

4. A process for preparing a compound of any of claims 1 to 3, wherein

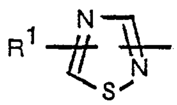 group is 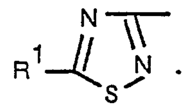 .

5. A process for preparing a compound of any of claims 1 to 4, wherein $R^1$ is amino, $R^2$ is hydrogen, ($C_1$—$C_6$) alkyl which may be substituted with 1 to 3 substituent(s) selected from the group consisting of halogen, ($C_1$—$C_6$) alkylthio, carboxy, esterified carboxy and aryl, ($C_2$—$C_6$) alkenyl, ($C_2$—$C_6$) alkynyl, cyclo($C_3$—$C_6$)alkyl, cyclo($C_3$—$C_6$)alkenyl, or tetrahydrofuryl substituted with an oxo group.

6. A process for preparing a compound of any of claims 1 to 5, wherein $R^2$ is hydrogen, methyl, ethyl, propyl, isopropyl, 2,2,2-trifluoroethyl, methylthiomethyl, carboxymethyl, 1-methyl-1-carboxyethyl, 1-carboxyethyl, methoxycarbonylmethyl, ethoxycarbonylmethyl, 1-methyl-1-ethoxycarbonylethyl, t-butoxycarbonylmethyl, 1-t-butoxycarbonylethyl, 1-methyl-1-t-butoxycarbonylethyl, 1-benzyloxycarbonylethyl, trityl, allyl, 2-propynyl, cyclopentyl, 2-cyclopenten-1-yl, 2-cyclohexen-1-yl or 2-oxotetrahydrofuran-3-yl.

7. A process for preparing a compound of any of claims 1 to 6, wherein $R^3$ is a group of the formula:

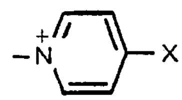

wherein X is hydrogen or carbamoyl and $R^4$ is —$COO^-$.

8. A process for preparing a compound of any of claims 1 to 6, wherein $R^3$ is 2-methyl-5-oxo-6-hydroxy-2,5-dihydro-1,2,4-triazinylthio and $R^4$ is carboxy.

9. A process for preparing a compound of any of claims 1 to 5, wherein $R^2$ is carboxy($C_{1-6}$)alkyl, ($C_{2-7}$) alkoxycarbonyl($C_{1-6}$)alkyl or ($C_{2-6}$) alkenyl; $R^3$ is a group of the formula:

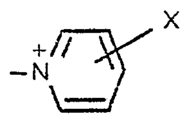

wherein X is hydrogen; and $R^4$ is —$COO^-$.

10. A process for preparing a compound of any of claims 1 to 5 and 9, wherein $R^2$ is carboxymethyl, t-butoxycarbonylmethyl or allyl.

11. A process for preparing a compound of claim 10, which is 7-[2-carboxymethoxyimino-2-(5-amino-1,2,4-thiadiazol-3-yl)-acetamido]-3-(1-pyridiniomethyl)-3-cephem-4-carboxylate (syn isomer).

12. A process for preparing a compound of claim 10, which is 7-[2-allyloxyimino-2-(5-amino-1,2,4-thiadiazol-3-yl)acetamido]-3-(1-pyridiniomethyl)-3-cephem-4-carboxylate (syn isomer).

13. A process for preparing a compound of the formula:

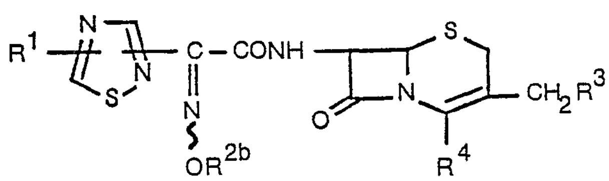

wherein

$R^1$ is amino or protected amino;

$R^{2b}$ is a carboxy($C_{1-6}$)alkyl;

$R^3$ is a group of the formula:

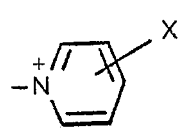

wherein X is hydrogen or carbamoyl; and

$R^4$ is —$COO^-$; or

$R^3$ is 2-($C_{1-6}$)alkyl-5-oxo-6-hydroxy-2,5-dihydro-1,2,4-triazinylthio; and

$R^4$ is carboxy or esterified carboxy; or pharmaceutically acceptable salts thereof; which comprises subjecting a compound of the formula:

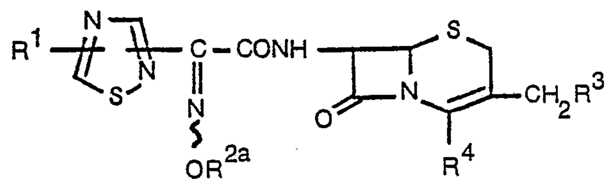

wherein $R^1$, $R^3$ and $R^4$ are each as defined above and $R^{2a}$ is an esterified carboxy$(C_{1-6})$alkyl, or a salt thereof, to elimiation reaction of the protective group of carboxy.

14. A process for preparing a compound of the formula:

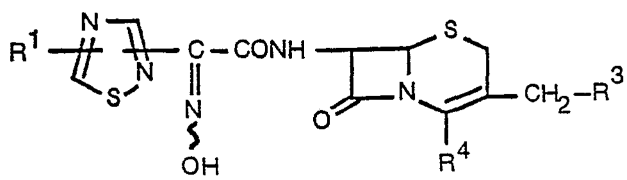

wherein

$R^1$ is amino or protected amino;

$R_3$ is a group of the formula:

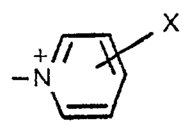

wherein X is hydrogen or carbamoyl; and

$R^4$ is $COO^-$; or

$R^3$ is 2-$(C_{1-6})$alkyl-5-oxo-6-hydroxy-2,5-dihydro-1,2,4-triazinylthio; and

$R^4$ is carboxy or esterified carboxy, or pharmaceutically acceptable salts thereof, which comprises reacting a compound of the formula:

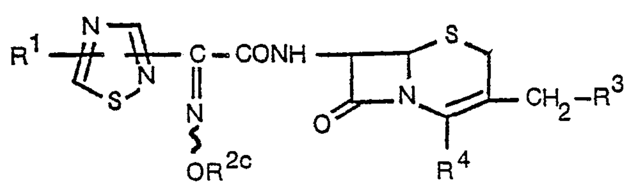

wherein $R^1$, $R^3$ and $R^4$ are each as defined above and $R^{2c}$ is a protective group of hydroxy, or a salt thereof, to elimination reaction of the protective group of hydroxy.

15. A process for preparing a compound of the formula:

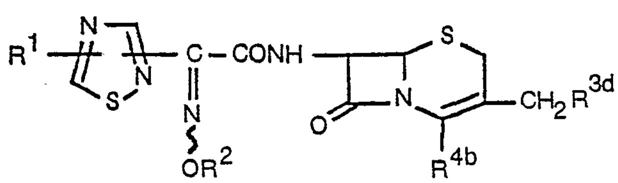

wherein

$R^1$ is amino or protected amino;

$R^2$ is hydrogen, $(C_{1-6})$ alkyl which may be substituted with phenyl, carboxy, esterified carboxy, $(C_{1-6})$ alkylthio or halogen, $(C_{2-6})$ alkenyl, $(C_{2-6})$ alkynyl cyclo$(C_{3-6})$alkyl, cyclo$(C_{3-6})$alkenyl, or tetrahydrofuryl substituted with an oxo group;

$R^{3d}$ is $(C_{1-6})$ alkanoyl $(C_{1-6})$ alkanoyloxy, and $R^{4b}$ is carboxy or esterified carboxy; or a salt thereof, which comprises a) reacting a compound of the formula:

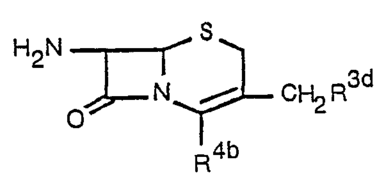

wherein $R^{3d}$ and $R^{4b}$ are each as defined above, or its reactive derivative at the amino group or a salt thereof, with a compound of the formula:

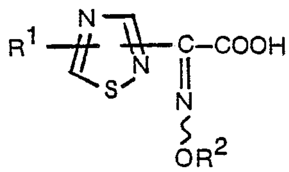

wherein $R^1$ and $R^2$ are each as defined above, or its reactive derivative at the carboxy group or a salt thereof, or b) subjecting a compound of the formula

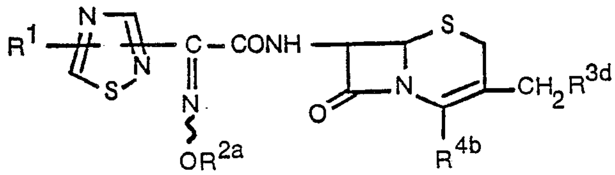

whereub $R^1$, $R^{3d}$ and $R^{4b}$ are each as defined above and $R^{2a}$ is a protected carboxy($C_{1-6}$)alkyl, or a salt thereof, to elimination reaction of the protective group of carboxy, to give a compound of the formula:

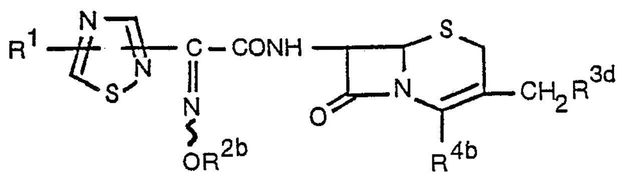

wherein $R^1$, $R^{3d}$ and $R^{4b}$ are each as defined above and $R^{2b}$ is a carboxy($C_{1-6}$)alkyl, or a salt thereof.

**Revendications pour les Etats contractants: BE CH DE FR GB IT LU NL SE**

1. Nouveaux composés de céphem ayant la formule:

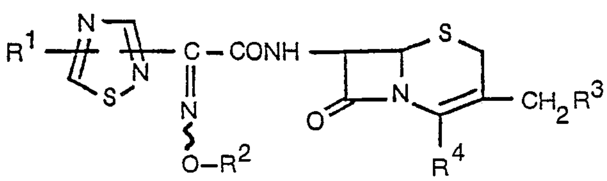

(I)

où

$R^1$ est le groupe amino ou un groupe amino protégé;

$R^2$ est l'hydrogène, un groupe alkyle en $C_1$—$C_6$ qui peut être à substitution phényle, carboxy, carboxy estérifé alkyl en $C_1$—$C_6$ thio ou halogène, alkényle en $C_2$—$C_6$, alkynyle en $C_2C_6$, cycloalkyle en $C_3$—$C_6$, cycloalkényle en $C_3$—$C_6$, ou tétrahydrofuryle substitué par un groupe oxo;

$R^3$ est un groupe de formule:

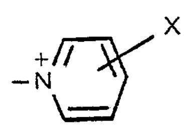

où X est l'hydrogène ou le groupe carbamoyle, et

$R^4$ est $COO^-$; ou

$R^3$ est un groupe 2-alkyl en $C_1$—$C_6$-5-oxo-6-hydroxy-2,5-dihydro-1,2,4-triazinylthio, et

$R^4$ est le groupe carboxy ou un groupe carboxy estérifié, et leurs sels pharmaceutiquement acceptables.

2. Isomère syn d'un composé de la revendication 1.

3. Composé de la revendication 1 ou de la revendication 2, dans lequel:

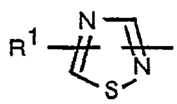

est

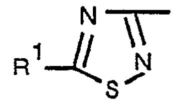 .

4. Composé selon l'une quelconque des revendications 1 à 3, dans lequel $R^1$ est le groupe amino; $R^2$ est l'hydrogène, un groupe alkyle en $C_1$—$C_6$ qui peut être substitué par 1 à 3 substituants choisis dans le groupe se composant d'halogène, de groupe alkyl en $C_1$—$C_6$ thio, carboxy, carboxy estérifié et aryle, alkényle en $C_2$—$C_6$, alkynyle en $C_2$—$C_6$, cycloalkyle en $C_3$—$C_6$, cycloalkényle en $C_3$—$C_6$ ou tétrahydrofuryle substitué par un groupe oxo.

5. Composé selon l'une quelconque des revendications 1 à 4, dans lequel: $R^2$ est l'hydrogène, le groupe méthyle, éthyle, propyle, isopropyle, 2,2,2-trifluoroéthyle, méthylthiométhyle, carboxyméthyle, 1-méthyl-1-carboxyéthyle, 1-carboxyéthyle, méthoxycarbonylméthyle, éthoxycarbonylméthyle, 1-méthyl-1-éthoxycarbonyléthyle, t-butoxycarbonylméthyle, 1-t-butoxycarbonyléthyle, 1-méthyl-1-t-butoxycarbonyléthyle, 1-benzyloxycarbonyléthyle, trityle, allyle, 2-propynyle, cyclopentyl, 2-cyclopentén-1-yle, 2-cyclohexen-1-yle ou 2-oxotétrahydrofuran-3yle.

6. Composé selon l'une quelconque des revendications 1 à 5, dans lequel $R^3$ est un groupe de formule:

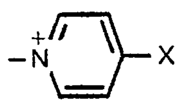

où X est l'hydrogène, ou le groupe carbamoyle et $R^4$ est —$COO^-$.

7. Composé selon l'une quelconque des revendications 1 à 5, dans lequel $R^3$ est le groupe 2-méthyl-5-oxo-6-hydroxy-2,5-dihydro-1,2,4-triazinylthio et $R^4$ est le groupe carboxy.

8. Composé selon l'une quelconque des revendications 1 à 4, dans lequel $R^2$ est un groupe carboxy-alkyle en $C_1$—$C_6$ alcoxy en $C_2$—$C_7$ carbonylalkyle en $C_1$—$C_6$ ou alkényle en $C_2$—$C_6$; $R^3$ est un groupe de formule:

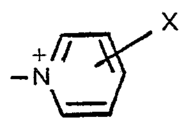

où X est l'hydrogène; et $r^4$ est —$COO^-$.

9. Composé selon l'une quelconque des revendications 1 à 4 et 8, dans lequel $R^2$ est le groupe carboxyméthyle, t-butoxycarbonylméthyle ou allyle.

10. Composé selon la revendication 9, qui est le 7-[2-carboxyméthoxyimino-2-(5-amino-1,2,4-thiadiazol-3-yl)acétamido]-3-(1-pyridinométhyl)-3-céphem-4-carboxylate (isomère syn).

11. Composé selon la revendication 9, qui est le 7-[2-allyloxyimino-2-(5-amino-1,2,4-thiadiazol-3-yl)acétamido]-3-(1-pyridiniométhyl)-3-céphem-4-carboxylate (isomère syn).

12. Procédé de préparation de nouveaux composés de céphem ayant la formule:

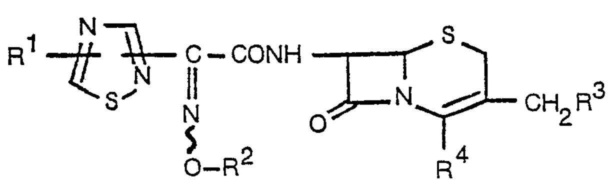

où

$R^1$ est le groupe amino ou un groupe amino protégé;

$R^2$ est l'hydrogène, un groupe alkyle en $C_1$—$C_6$, qui peut être à substitution phényle, carboxy, carboxy estérifié, alkyl en $C_1$—$C_6$ thio ou halogène, alkényle en $C_2$—$C_6$, alkynyle en $C_2$—$C_6$, cycloalkyle en $C_3$—$C_6$, cycloalkényle en $C_3$—$C_6$ ou tétrahydrofuryle substitué par un groupe oxo;

$R^3$ est un groupe de formule:

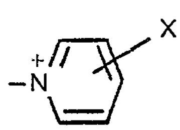

où X est l'hydrogène ou le groupe carbamoyle, et

$R^4$ est $COO^-$; ou

$R^3$ est un groupe 2-alkyl en $C_1$—$C_6$-5-oxo-6-hydroxy-2,5-dihydro-1,2,4-triazinylthio; et

$R^4$ est le groupe carboxy ou un groupe carboxy estérifié, ou de leurs sels pharmaceutiquement acceptables, qui consiste à faire réagir un composé de formule:

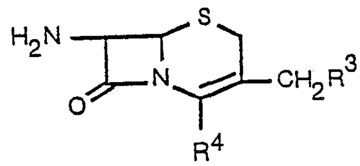

où $R^3$ et $R^4$ sont chacun comme définis ci-dessus, ou son dérivé réactif sur le groupe amino ou son sel, avec un composé de formule:

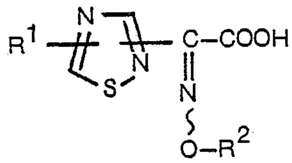

où $R^1$ et $R^2$ sont chacun comme définis ci-dessus, ou son dérivé réactif sur le groupe carboxy ou son sel.

13. Procédé de préparation de nouveaux composés de céphem ayant la formule:

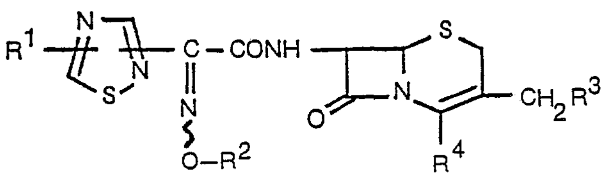

où

$R^1$ est le groupe amino ou un groupe amino protégé;

$R^2$ est l'hydrogène, un groupe alkyle en $C_1$—$C_6$ qui peut être à substitution phényle, carboxy, carboxy estérifié, alkyl en $C_1$—$C_6$ thio ou halogène, alkényle en $C_2$—$C_6$ alkynyle en $C_2$—$C_6$, cycloalkyle en $C_3$—$C_6$, cycloalkényle en $C_3$—$C_6$ ou tétrahydrofuryle substitué par un groupe oxo;

$R^3$ est un groupe de formule:

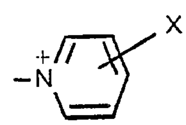

où X est l'hydrogène ou le groupe carbamoyle, et

$R^4$ est $COO^-$; ou bien

$R^3$ est un groupe 2-alkyl en $C_1$—$C_6$-5-oxo-6-hydroxy-2,5-dihydro-1,2,4-triazinylthio; et

$R^4$ est le groupe carboxy ou un groupe carboxy estérifié, ou de leurs pharmaceutiquement acceptables, qui consiste à faire réagir un composé ayant la formule:

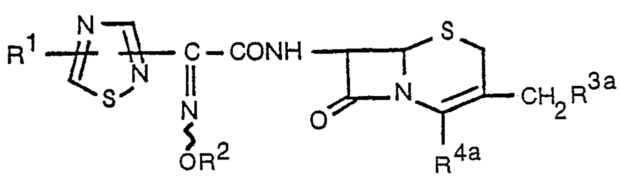

où $R^1$ et $R^2$ sont chacun comme définis ci-dessus, $R^{3a}$ est un groupe qui peut être substitué par un groupe de formule: $R^3$ où $R^3$ est défini ci-dessus; et $R^{4a}$ est le groupe carboxy quand $R^{3b}$ est un composé de formule:

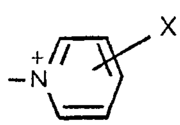

où X est comme défini ci-dessus; ou bien $R^{4a}$ est le groupe carboxy ou un groupe carboxy estérifié quand $R^{3b}$ est un composé de formule: $R^{3c}$-H où $R^{3c}$ est un groupe 2-alkyl en $C_{1-6}$-5-oxo-6-hydroxy-2,5-dihydro-1,2,4-triazinylthio; ou son sel, avec un composé de formule:

$R^{3b}$

où $R^{3b}$ est comme défini ci-dessus, ou son dérivé réactif.

14. Procédé de préparation d'un composé ayant la formule:

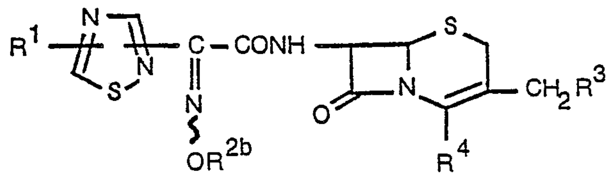

où

$R^1$ est le groupe amino ou un groupe amino protégé;

$R^{2b}$ est un groupe carboxyalkyle en $C_1$—$C_6$;

$R^3$ est un groupe de formule:

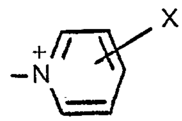

où X est l'hydrogène ou le groupe carbamoyle, et

$R^4$ est $COO^-$; ou

$R^3$ est un groupe 2-alkyl en $C_1$—$C_6$-5-oxo-6-hydroxy-2,5-dihydro-1,2,4-triazinylthio; et

$R^4$ est le groupe carboxy ou un groupe carboxy estérifié, ou de ses sels pharmaceutiquement acceptables, qui consiste à soumettre un composé ayant la formule:

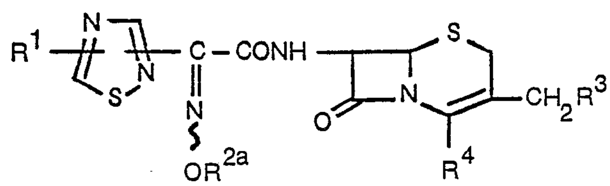

où $R^1$, $R^3$ et $R^4$ sont chacun comme définis ci-dessus et $R^{2a}$ est un groupe carboxy estérifié alkyle en $C_1$—$C_6$, ou son sel, à une réaction d'élimination du groupe de protection du groupe carboxy.

15. Procédé de préparation d'un composé de formule:

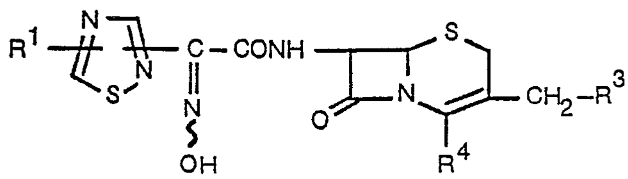

où

$R^1$ est le groupe amino ou un groupe amino protégé;

$R^3$ est un groupe de formule:

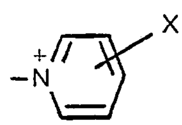

où X est l'hydrogène ou le groupe carbamoyle, et

$R^4$ est —$COO^-$; ou bien

$R^3$ est un groupe 2-alkyl en $C_1$—$C_6$-5-oxo-6-hydroxy-2,5-dihydro-1,2,4-triazinylthio; et

$R^4$ est le groupe carboxy ou un groupe carboxy estérifié, ou ses sels pharmaceutiquement acceptables, qui consiste à soumettre un composé ayant de formule:

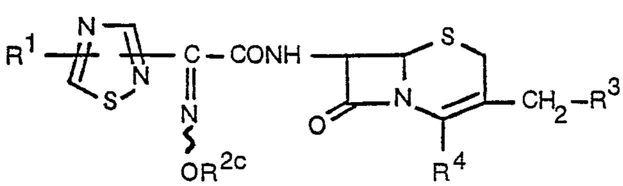

où $R^1$, $R^3$ et $R^4$ sont chacun définis ci-dessus, et $R^{2c}$ est un groupe de protection du radical hydroxy, ou son sel, à une réaction d'élimination du groupe de protection du groupe hydroxy.

16. Composé de formule:

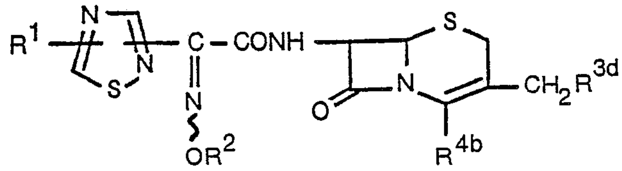

où

$R^1$ est le groupe amino ou un groupe amino protégé;

$R^2$ est l'hydrogène, un groupe alkyle en $C_1$—$C_6$ qui peut être à substitution phényle, carboxy, carboxy estérifié, alkyl en $C_1$—$C_6$ thio ou halogène, alkényle en $C_2$—$C_6$, alkynyle en $C_2$—$C_6$, cycloalkyle en $C_3$—$C_6$, cycloalkényle en $C_3$—$C_6$ ou tétrahydrofuryle substitué par un groupe oxo;

$R^{3d}$ est un groupe alcanoyle inférieur alcanoyl en $C_1$—$C_6$ oxy et

$R^{4b}$ est le groupe carboxy ou un groupe carboxy estérifié ou son sel.

17. Procédé de préparation d'un composé de formule:

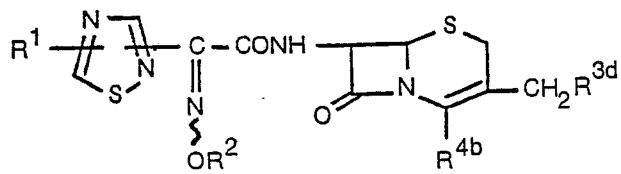

où

$R^1$ est le groupe amino ou un groupe amino protégé;

$R^2$ est l'hydrogène, un groupe alkyle en $C_1$—$C_6$ qui peut être à substitution phényle, carboxy, carboxy estérifié, alkyl en $C_1$—$C_6$ thio ou un halogène, alkényle en $C_2$—$C_6$, alkynyle en $C_2$—$C_6$, cycloalkyle en $C_3$—$C_6$, cycloalkényle en $C_3$—$C_6$ ou tétrahydrofuryle substitué par un groupe oxo;

$R^{3d}$ est un groupe alcanoyl en $C_1$—$C_6$ alcanoyl en $C_1$—$C_6$ oxy, et

$R^{4b}$ est le groupe carboxy ou un groupe carboxy estérifié ou son sel, qui consiste:

a) à faire réagir un composé ayant de formule:

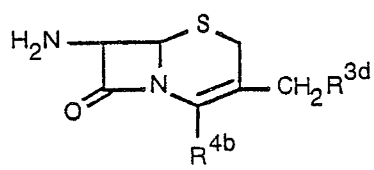

où $R^{3d}$ et $R^{4b}$ sont chacun comme définis ci-dessus, ou son dérive réactif sur le groupe amino ou son sel, avec un composé de formule:

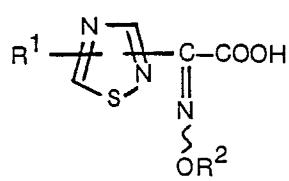

où $R^1$ et $R^2$ sont chacun comme définis ci-dessus, ou son dérivé réactif sur le groupe carboxy ou son sel, ou

b) à soumettre un composé de formule:

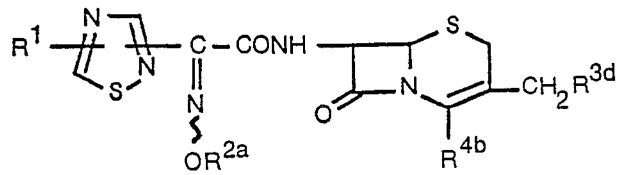

où $R^1$, $R^{3d}$ et $R^{4b}$ sont chacun définis ci-dessus et $R^{2a}$ est un groupe carboxy protégé alkyle en

$C_1$—$C_6$ ou son sel, à une réaction d'élimination du groupe de protection du groupe carboxy, pour donner un composé de formule:

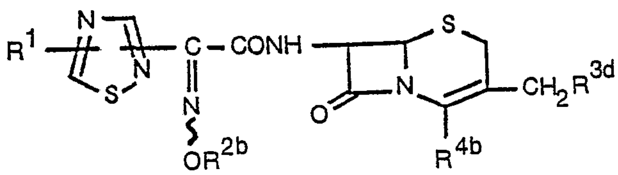

où $R^1$, $R^{3d}$ et $R^{4b}$ sont chacun comme définis ci-dessus et $R^{2b}$ est un groupe carboxy-alkyle en $C_1$—$C_6$ ou son sel.

18. Composition antibactérienne pharmaceutique comprenant un composé de la revendication 1 en association avec un support ou un excipient pharmaceutiquement acceptable, sensiblement non toxique.

19. Composé de formule I pour l'utilisation comme médicament.

20. Composé de formule I pour l'utilisation dans le traitement de maladies infectieuses.

## Revendications pour l'Etat contractant: AT

1. Procédé pour préparer de nouveaux composés de céphem ayant la formule:

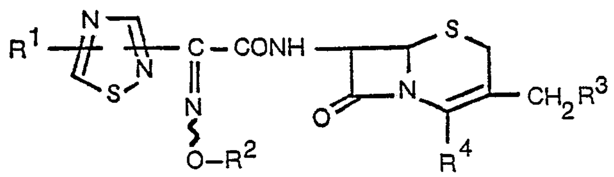

où

$R^1$ est le groupe amino ou un groupe amino protégé;

$R^2$ est l'hydrogène, un groupe alkyle en $C_1$—$C_6$ qui peut être à substitution phényle, carboxy, carboxy estérifié, alkyl en $C_1$—$C_6$ thio ou halogène, alkényle en $C_2$—$C_6$, alkynyle en $C_2$—$C_6$, cycloakyle en $C_3$—$C_6$, cycloalkényle en $C_3$—$C_6$ ou tétrahydrofuryle substitué par un groupe oxo;

$R^3$ est un groupe le formule;

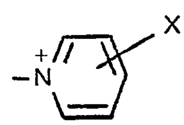

où X est l'hydrogène ou le groupe carbamoyle, et

$R^4$ est $COO^-$; ou bien

$R^3$ est un groupe 2-alkyl en $C_1$—$C_6$-5-oxo-6-hydroxy-2,5-dihydro-1,2,4-triazinylthio, et

$R^4$ est le groupe carboxy ou un groupe carboxy estérifié, ou ses sels pharmaceutiquement acceptables, qui consiste à faire réagir un composé de formule:

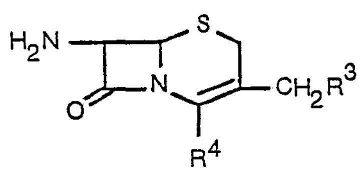

où $R^3$ et $R^4$ sont chacun comme définis ci-dessus, ou son dérivé réactif sur le groupe amino ou son sel avec un composé de formule:

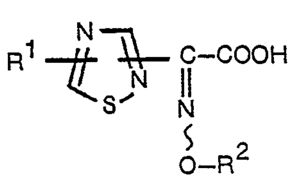

où $R^1$ et $R^2$ sont chacun comme définis ci-dessus, ou son dérivé réactif sur le groupe carboxy ou son sel.

2. Procédé de préparation de nouveaux composés de céphem ayant la formule:

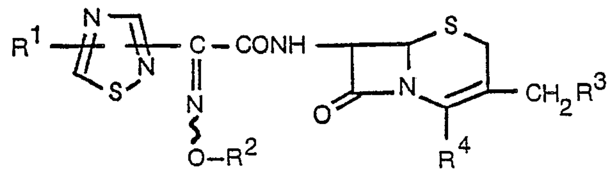

où

$R^1$ est le groupe amino ou un groupe amino protégé;

$R^2$ est l'hydrogène, un groupe alkyle en $C_1$—$C_6$ qui peut être à substitution phényle, carboxy, carboxy estérifié, alkyl en $C_1$—$C_6$ thio ou halogène, alkényle en $C_2$—$C_6$, alkynyle en $C_2$—$C_6$, cycloalkyle en $C_3$—$C_6$, cycloalkényle en $C_3$—$C_6$ ou tétrahydrofuryle substitué par un groupe oxo;

$R^3$ est un groupe le formule;

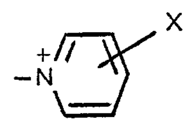

où X est l'hydrogène ou le groupe carbamoyle, et

$R^4$ est $COO^-$; ou bien

$R^3$ est un groupe 2-alkyl en $C_1$—$C_6$-5-oxo-6-hydroxy-2,5-dihydro-1,2,4-triazinylthio; et

$R^4$ est le groupe carboxy ou un groupe carboxy estérifié, ou ses sels pharmaceutiquement acceptables, qui consiste à faire réagir un composé ayant la formula:

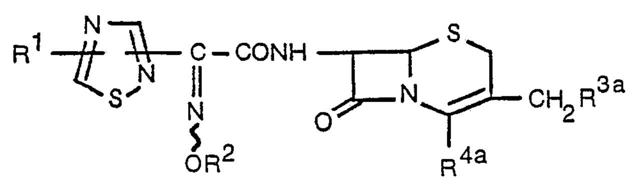

où $R^1$ et $R^2$ sont chacun comme définis ci-dessus, $R^{3a}$ est un groupe qui peut être substitué par un groupe de formule: $R^3$ où $R^3$ est comme défini ci-dessus; et $R^{4a}$ est le groupe carboxy quand $R^{3b}$ est un groupe de formule:

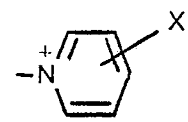

où X est comme défini ci-dessus; ou bien $R^{4a}$ est le groupe carboxy ou un groupe carboxy estérifié quand $R^{3b}$ est un composé de formule: $R^{3c}$-H où $R^{3c}$ est un groupe 2-alkyl en $C_{1-6}$-5-oxo-6-hydroxy-2,5-dihydro-1,2,4-triazinylthio; ou son sel, avec un composé de formule:

$R^{3b}$

où $R^{3b}$ est comme défini ci-dessus, ou son dérivé réactif.

3. Procédé de préparation de l'isomère syn d'un composé des revendications 1 ou 2.

4. Procédé de préparation d'un composé selon l'une quelconque des revendications 1 à 3, dans lequel

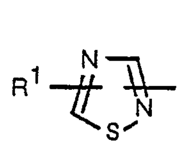 est 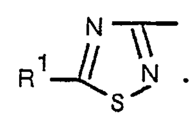 .

5. Procédé de préparation d'un composé selon l'une quelconque des revendications 1 à 4, dans lequel $R^1$ est le groupe amino; $R^2$ est l'hydrogène, un groupe alkyle en $C_1$—$C_6$ qui peut être substitué par 1 à 3 substituants choisis dans le groupe se composant d'halogène, de groupe alkyl en $C_1$—$C_6$ thio, carboxy, carboxy estérifié et aryle, alkényle en $C_2$—$C_6$, alkynyle en $C_2$—$C_6$, cycloalkyle en $C_3$—$C_6$, cycloalkényle en $C_3$—$C_6$ ou tétrahydrofuryle substitué par un groupe oxo.

6. Procédé pour préparer un composé selon l'une quelconque des revendications 1 à 5, dans lequel $R^2$ est l'hydrogène, le groupe méthyle, éthyle, propyle, isopropyle, 2,2,2-trifluoroéthyle, méthylthiométhyle, carboxyméthyle, 1-méthyl-1-carboxyéthyle, 1-carboxyéthyle, méthoxycarbonylméthyle, éthoxycarbonylméthyle, l-méthyl-1-éthoxycarbonyléthyle, t-butoxycarbonylméthyle, 1-t-butoxycarbonyléthyle, 1-méthyl-1-t-butoxycarbonyl-éthyle, 1-benzyloxycarbonyléthyle, trityle, allyle, 2-propynyle, cyclopentyle, 2-cyclopentén-1-yle, 2-cyclohexén-1-yle ou 2-oxotétrahydrofuran-3-yle.

7. Procédé de préparation d'un composé selon l'une quelconque des revendications 1 à 6, dans lequel $R^3$ est un groupe de formule:

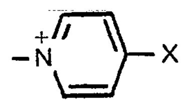

où X est l'hydrogène, ou le groupe carbamoyle et $R^4$ est $—COO^-$.

8. Procédé de préparation d'un composé selon l'une quelconque des revendications 1 à 6, dans lequel $R^3$ est le groupe 2-méthyl-5-oxo-6-hydroxy-2,5-dihydro-1,2,4-triazinylthio et $R^4$ est le groupe carboxy.

9. Procédé de préparation d'un composé selon l'une quelconque des revendications 1 à 5, dans lequel $R^2$ est un groupe carboxy-alkyle en $C_1$—$C_6$ alcoxy en $C_2$—$C_7$ carbonyl-alkyle en $C_1$—$C_6$ ou alkényle en $C_2$—$C_6$; $R^3$ est un groupe de formule:

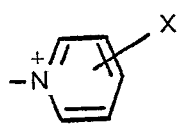

où X est l'hydrogène; et

$R^4$ est $—COO^-$.

10. Procédé de préparation d'un composé selon l'une quelconque des revendications 1 à 5 et 9, dans lequel

$R^2$ est le groupe carboxyméthyle, t-butoxycarbonylméthyle ou allyle.

méthoxyimino - 2 - (5 - amino - 1,2,4 - thiadiazol - 3 - yl)acétamido] - 3 - (1 - pyridinométhyl) - 3-céphem - 4 - carboxylate (isomère syn).

11. Procédé de préparation d'un composé de la revendication 10, qui est le 7-[2-carboxy-mèthoxyimino-2-(5-amino-1,2,4-thiadiazol-3-yl)acétamido]-3-(1-pyridniométhyl)-3-céphem-4-carboxylate (isomère syn).

12. Procédé de préparation d'un composé de la revendication 10, qui est le 7-[2-allyloxyimino-2-(5-amino-1,2,4-thiadiazol-3-yl)acétamido]-3-(1-pyridiniométhyl)-3-céphem-4-carboxylate (isomère syn).

13. Procédé de préparation d'un composé ayant la formule

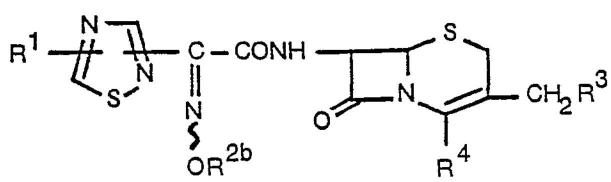

où

$R^1$ est le groupe amino ou un groupe amino protégé;

$R^{2b}$ est un groupe carboxy-alkyle en $C_1$—$C_6$;

$R^3$ est un groupe le formule;

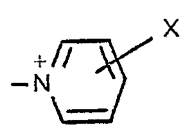

où X est l'hydrogène ou le groupe carbamoyle, et

$R^4$ est $—COO^-$; ou bien

$R^3$ est un groupe 2-alkyl en $C_1$—$C_6$-5-oxo-6-hydroxy-2,5-dihydro-1,2,4-triazinylthio; et

$R^4$ est le groupe carboxy ou un groupe carboxy estérifié; ou de ses sels pharmaceutiquement acceptables, qui consiste à soumettre un composé de formule:

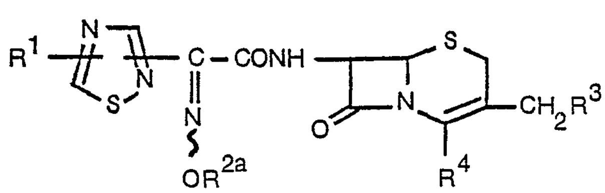

où $R^1$, $R^3$ et $R^4$ sont chacun comme définis ci-dessus et $R^{2a}$ est un groupe carboxy estérifié alkyle en

$C_1$—$C_6$, ou son sel, à une réaction d'élimination du groupe de protection du groupe carboxy.

14. Procédé de préparation d'un composé ayant la formule:

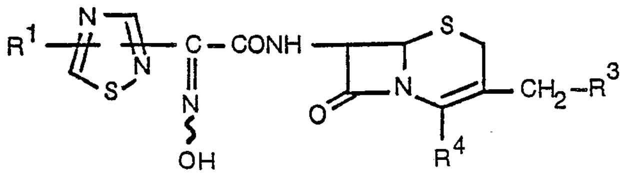

où

$R^1$ est le groupe amino ou un groupe amino protégé;

$R^3$ est un groupe de formule:

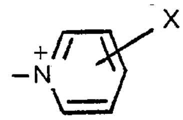

où X est l'hydrogène ou le groupe carbamoyle; et

$R^4$ est —$COO^-$; ou bien

$R^3$ est un groupe 2-alkyl en $C_1$—$C_6$-5-oxo-6-hydroxy-2,5-dihydro-1,2,4-triazinylthio; et

$R^4$ est le groupe carboxy ou un groupe carboxy estérifié, ou de ses sels pharmaceutiquement acceptables, qui consiste à soumettre un composé ayant la formule:

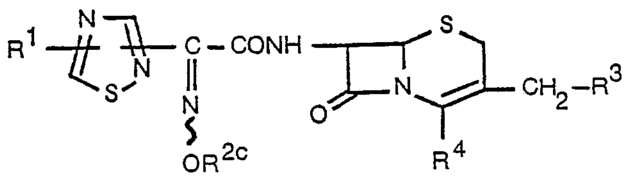

où $R^1$, $R^3$ et $R^4$ sont chacun comme définis ci-dessus, et $R^{2c}$ est un groupe de protection du groupe hydroxy, ou son sel, à une réaction d'élimination du groupe de protection du groupe hydroxy.

15. Procédé de préparation d'un composé ayant la formule:

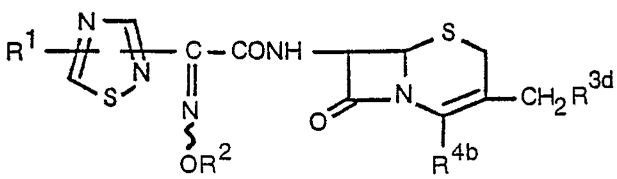

où

$R^1$ est le groupe amino ou un groupe amino protégé;

$R^2$ est l'hydrogène, un groupe alkyle en $C_1$_6 qui peut être à substitution phényle, carboxy, carboxy estérifié, alkyl en $C_1$—$C_6$ thio ou halogène, alkényle en $C_{2-6}$, alkynyle en $C_2$—$C_6$, cycloalkyle en $C_3$—$C_6$, cycloalkényle en $C_3$—$C_6$ ou tétrahydrofuryle substitué par un groupe oxo;

$R^{3d}$ est un groupe alcanoyl en $C_1$—$C_6$ alcanoyl en $C_1$—$C_6$ oxy et

$R^{4b}$ est le groupe carboxy ou un groupe carboxy estérifié, ou son sel, qui consiste:

a) à faire réagir un composé ayant la formule:

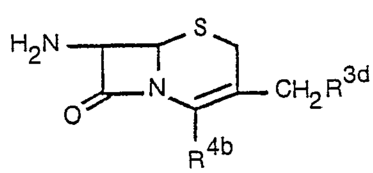

où $R^{3d}$ et $R^{4b}$ sont chacun comme définis, ou son dérivé réactif sur le groupe amino ou son sel, avec un composé de formule:

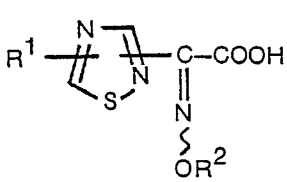

où $R^1$ et $R^2$ sont chacun comme définis ci-dessus, ou son dérivé réactif sur le groupe carboxy ou son sel, ou

b) à soumettre un composé ayant la formule:

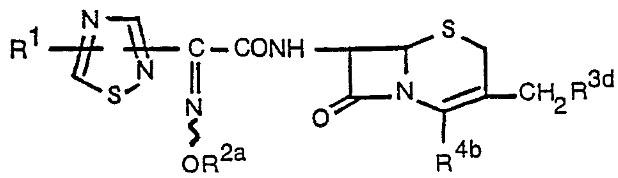

où $R^1$, $R^{3d}$ et $R^{4b}$ sont chacun comme définis ci-dessus et $R^{2a}$ est un groupe carboxy protégé alkyl en $C_1$—$C_6$ ou son sel, à une réaction d'élimination du groupe de protection du groupe carboxy, pour donner un composé ayant la formule:

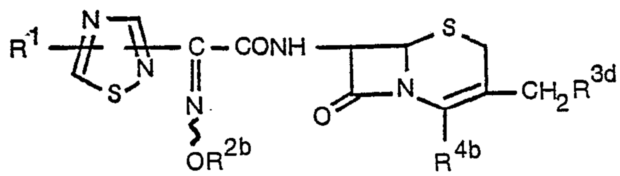

où $R^1$, $R^{3d}$ et $R^{4b}$ sont chacun comme définis ci-dessus et $R^{2b}$ est un groupe carboxy-alkyle en $C_1$—$C_6$ ou son sel.

**Patentansprüche für der Vertragsstaaten: BE CH DE FR GB IT LU NL SE**

1. Neue Cephemverbindungen der Formel

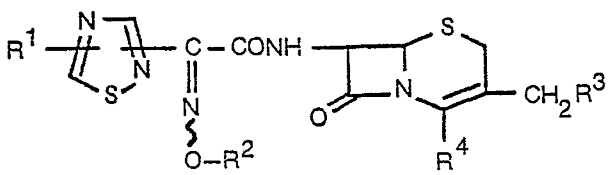

(I)

worin

$R^1$ Amino oder geschütztes Amino ist;

$R^2$ Wasserstoff, ($C_1$—$C_6$)-Alkyl, das mit Phenyl, Carboxy, verestertem Carboxy, ($C_1$—$C_6$)-Alkylthio oder Halogen substituiert sein kann, ($C_2$—$C_6$)-Alkenyl, ($C_2$—$C_6$)-Alkinyl, Cyclo-($C_3$—$C_6$)-alkyl, Cyclo-($C_3$—$C_6$)-alkenyl oder Tetrahydrofuryl substituiert mit einer Oxogruppe ist;

$R^3$ eine Gruppe der Formel

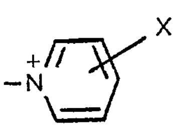

ist, worin X Wasserstoff oder Carbamoyl ist; und

$R^4$ —$COO^-$ ist; oder

$R^3$ 2-($C_1$—$C_6$)-Alkyl-5-oxo-6-hydroxy-2,5-dihydro-1,2,4-triazinylthio ist; und

$R^4$ Carboxy oder verestertes Carboxy ist, und pharmazeutisch brauchbare Salze davon.

2. Syn-Isomeres einer Verbindung nach Anspruch 1.

3. Verbindung nach Anspruch 1 oder 2, worin die Gruppe

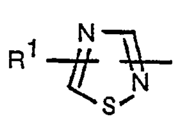 folgende Gruppe 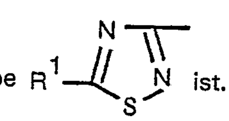 ist.

4. Verbindung nach einem der Ansprüche 1 bis 3, worin $R^1$ Amino ist; $R^2$ Wasserstoff, ($C_1$—$C_6$-Alkyl, das mit 1 bis 3 Substituenten, ausgewählt aus der Gruppe von Halogen, ($C_1$—$C_6$)-Alkylthio, Carboxy, verestertem Carboxy und Aryl substituiert sein kann, ($C_2$—$C_6$)-Alkenyl, ($C_2$—$C_6$)-Alkinyl, Cyclo-($C_3$—$C_6$)-alkyl, Cyclo-($C_3$—$C_6$)-alkenyl oder Tetrahydrofuryl substituiert mit einer Oxogruppe, ist.

5. Verbindung nach einem der Ansprüche 1 bis 4, worin $R^2$ Wasserstoff, Methyl, Ethyl, Propyl, Isopropyl, 2,2,2-Trifluoroethyl, Methylthiomethyl, Carboxymethyl, 1-Methyl-1-carboxyethyl, 1-Carboxyethyl, Methoxycarbonylmethyl, Ethoxycarbonylmethyl, 1-Methyl-1-ethoxycarbonylethyl, t-Butoxycarbonylmethyl, 1-t-Butoxycarbonylethyl, 1-Methyl-1-t-butoxycarbonylethyl, 1-Benzyloxycarbonylethyl, Trityl, Allyl, 2-Propinyl, Cyclopentyl, 2-Cyclopenten-1-yl, 2-Cyclohexen-1-yl oder 2-Oxotetrahydrofuran-3-yl ist.

6. Verbindung nach einem der Anspüche 1 bis 5, worin $R^3$ eine Gruppe der Formel

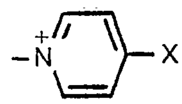

ist, worin X Wasserstoff oder Carbamoyl ist und $R^4$ —$COO^-$ ist.

7. Verbindung nach einem der Ansprüche 1 bis 5, worin $R^3$ 2-Methyl-5-oxo-6-hydroxy-2,5-dihydro-1,2,4-triazinylthio und $R^4$ Carboxy ist.

8. Verbindung nach einem der Ansprüche 1 bis 4, worin $R^2$ Carboxy-$(C_{1-6})$-alkyl, $(C_{2-7})$-Alkoxycarbonyl-$(C_{1-6})$-alkyl oder $(_2$—$C_6)$-Alkenyl ist; $R^3$ eine Gruppe der Formel

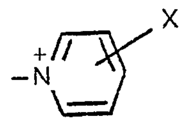

ist, worin X Wasserstoff ist; und $R^4$ —$COO^-$ ist.

9. Verbindung nach einem der Ansprüche 1 bis 4 und 8, worin $R^2$ Carboxymethyl, t-Butoxycarbonylmethyl oder Allyl ist.

10. Verbindung nach Anspruch 9, nämlich 7-[2-Carboxymethoxyimino-2-(5-amino-1,2,4-thiadiazol-3-yl)-acetamido]-3-(1-pyridiniomethyl)-3-cephem-4-carboxylat (syn-Isomeres).

11. Verbindung nach Anspruch 9, nämlich 7-[2-Allyloxyimino-2-(5-amino-1,2,4-thiadiazol-3-yl)-acetamido]-3-(1-pyridiniomethyl)-3-cephem-4-carboxylat (syn-Isomeres).

12. Verfahren zur Herstellung neuer Cephemverbindungen der Formel

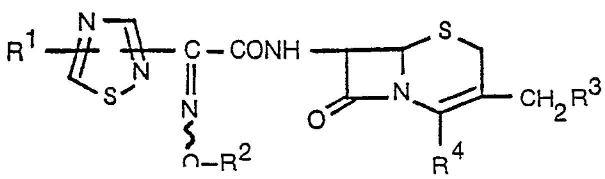

worin

$R^1$ Amino oder geschützes Amino ist;

$R^2$ Wasserstoff, $(C_1$—$C_6)$-Alkyl, das substituiert sein kann mit Phenyl, Carboxy, verestertem Carboxy, $(C_1$—$C_6)$-Alkylthio oder Halogen, $(C_2$—$C_6)$-Alkenyl, $(C_2$—$C_6)$-Alkinyl, Cyclo-$(C_3$—$C_6)$-alkyl, Cyclo-$(C_3$—$C_6)$-alkenyl oder Tetrahydrofuryl substituiert mit einer Oxogruppe ist;

$R^3$ eine Gruppe der Formel

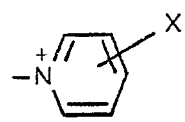

ist, worin X Wasserstoff oder Carbamoyl ist; und

$R^4$ $COO^-$ ist; oder

$R^3$ 2-$(C_1$—$C_6)$-Alkyl-5-oxo-6-hydroxy-2,5-dihydro-1,2,4-triazinylthio ist; und

$R^4$ Carboxy oder verestertes Carboxy ist, oder pharmazeutisch brauchbare Salze davon durch Reaktion einer Verbindung der Formel

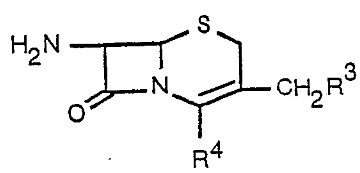

worin $R^3$ und $R^4$ jeweils wie vorstehend definiert sind oder ihres reaktiven Derivats an der Aminogruppe oder eines Salzes davon, mit einer Verbindung der Formel

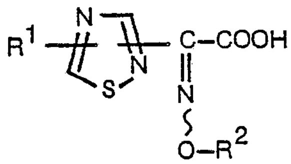

worin $R^1$ und $R^2$ jeweils wie vorstehend definiert sind oder ihrem reaktiven Derivat an der Carboxygruppe oder einem Salz davon.

13. Verfahren zur Herstellung neuer Cephemverbindungen der Formel

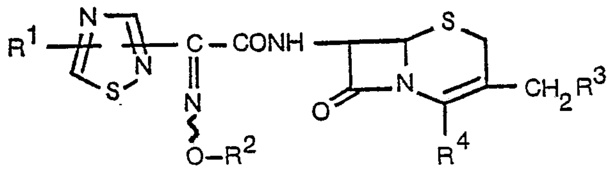

worin

$R^1$ Amino oder geschütztes Amino ist;

$R^2$ Wasserstoff, $(C_{1-6})$-Alkyl, das substituiert sein kann mit Phenyl, Carboxy, verestertem Carboxy, $(C_{1-6})$-Alkylthio oder Halogen, $(C_{2-6})$-Alkenyl, $(C_{2-6})$-Alkinyl, Cyclo-$(C_{3-6})$-alkyl, Cyclo-$(C_{3-6})$-alkenyl oder Tetrahydrofuryl substituiert mit einer Oxogruppe ist;

$R^3$ eine Gruppe der Formel

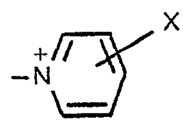

ist, worin X Wasserstoff oder Carbamoyl ist; und

$R^4$ —$COO^-$ ist; oder

$R^3$ 2-$(C_{1-6})$-Alkyl-5-oxo-6-hydroxy-2,5-dihydro-1,2,4-triazinylthio ist; und

$R^4$ Carboxy oder verestertes Carboxy ist, oder pharmazeutisch brauchbarer Salze davon durch Reaktion einer Verbindung der Formel

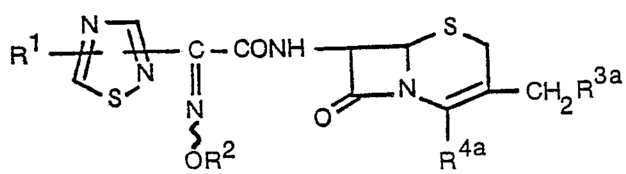

worin $R^1$ und $R^2$ jeweils wie vorstehend definiert sind, $R^{3a}$ eine Gruppe ist, die ersetzt werden kann durch eine Gruppe der Formel $R^3$, worin $R^3$ wie vorstehend definiert ist; und $R^{4a}$ Carboxy ist, wenn $R^{3b}$ eine Verbindung der Formel

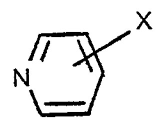

ist, worin X wie vorstehend definiert ist; oder $R^{4a}$ Carboxy oder verestertes Carboxy ist, wenn $R^{3b}$ eine Verbindung der Formel $R^{3c}$-H ist, worin $R^{3c}$ 2-$(C_{1-6})$-Alkyl-5-oxo-6-hydroxy-2,5-dihydro-1,2,4-triazinylthio ist; oder eines Salzes davon mit einer Verbindung der Formel

$$R^{3b}$$

worin $R^{3b}$ wie vorstehend definiert ist oder ihrem reaktiven Derivat.

14. Verfahren zur Herstellung einer Verbindung der Formel

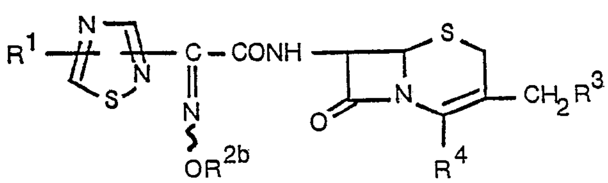

worin
$R^1$ Amino oder geschütztes Amino ist;
$R^{2b}$ Carboxy-($C_{1-6}$)-alkyl ist;
$R^3$ eine Gruppe der Formel

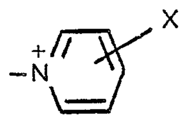

ist, worin X Wasserstoff oder Carbamoyl ist; und
$R^4$ —$COO^-$ ist; oder
$R^3$ 2-($C_{1-6}$)-Alkyl-5-oxo-6-hydroxy-2,5-dihydro-1,2,4-triazinylthio ist; und
$R^4$ Carboxy oder verestertes Carboxy ist, oder pharmazeutisch brauchbarer Salze davon durch Unterwerfen einer Verbindung der Formel

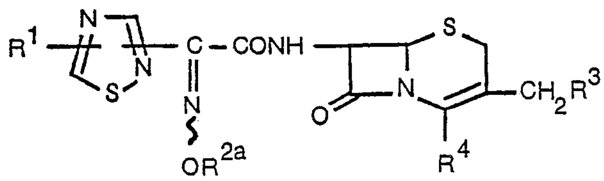

worin $R^1$, $R^3$ und $R^4$ jeweils wie vorstehend definiert sind und $R^{2a}$ verestertes Carboxy-($C_{1-6}$)-alkyl ist, oder eines Salzes davon, der Eliminierungsreaktion der Carboxy-Schutzgruppe.

15. Verfahren zur Herstellung einer Verbindung der Formel

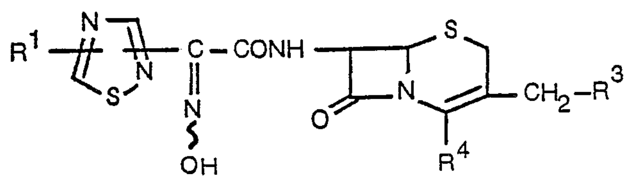

worin
$R^1$ Amino oder geschütztes Amino ist;
$R^3$ eine Gruppe der Formel

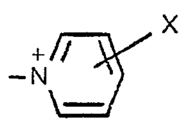

ist, worin X Wasserstoff oder Carbamoyl ist; und
$R^4$ —$COO^-$ ist; oder
$R^3$ 2-($C_{1-6}$)-alkyl-5-oxo-6-hydroxy-2,5-dihydro-1,2,4-triazinylthio ist; und
$R^4$ Carboxy oder verestertes Carboxy ist oder der pharmazeutisch brauchbaren Salze davon durch Unterwerfen einer Verbindung der Formel

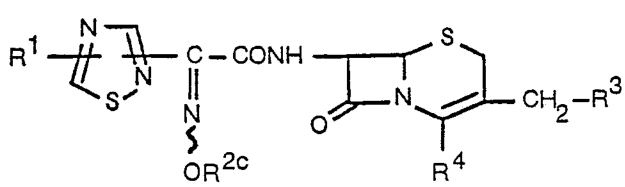

worin $R^1$, $R^3$ und $R^4$ jeweils wie vorstehend definiert sind und $R^{2c}$ eine Hydroxy-Schutzgruppe ist oder eines Salzes davon der Eliminierungsreaktion der Hydroxy-Schutzgruppe.

16. Verbindung mit der Formel

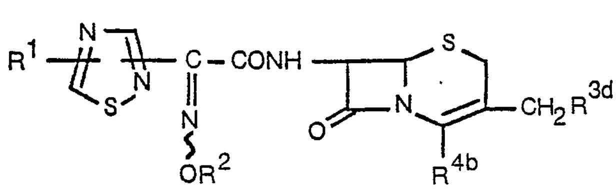

worin
$R^1$ Amino oder geschütztes Amino ist;

$R^2$ Wasserstoff, $(C_{1-6})$-Alkyl, das substituiert sein kann mit Phenyl, Carboxy, verestertem Carboxy, $(C_{1-6})$-Alkylthio oder Halogen, $(C_{2-6})$-Alkenyl, $(C_{2-6})$-Alkinyl, Cyclo-$(C_{3-6})$-alkyl, Cyclo-$(C_{3-6})$-alkenyl oder Tetrahydrofuryl substituiert mit einer Oxogruppe ist;

$R^{3d}$ niedrig-Alkanoyl-$(C_{1-6})$-alkanoyloxy ist und

$R^{4b}$ Carboxy oder verestertes Carboxy ist und ein Salz davon.

17. Verfahren zur Herstellung einer Verbindung der Formel

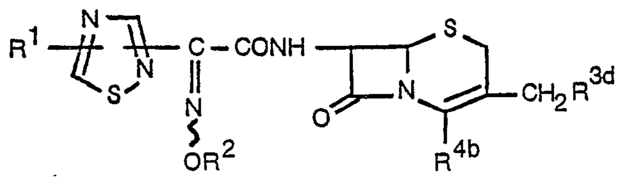

worin

$R^1$ Amino oder geschütztes Amino ist;

$R^2$ Wasserstoff, $(C_{1-6})$-Alkyl, das substituiert sein kann mit Phenyl, Carboxy, verestertem Carboxy, $(C_{1-6})$-Alkylthio oder Halogen, $(C_{2-6})$-Alkenyl, $(C_{2-6})$-Alkinyl, Cyclo-$(C_{3-6})$-alkyl, Cyclo-$(C_{3-6})$-alkenyl oder Tetrahydrofuryl substituiert mit einer Oxogruppe ist;

$R^{3d}$ $(C_{1-6})$-Alkanoyl-$(C_{1-6})$-alkanoyloxy ist und

$R^{4b}$ Carboxy oder verestertes Carboxy ist; oder eines Salzes davon durch

a) Reaktion einer Verbindung der Formel

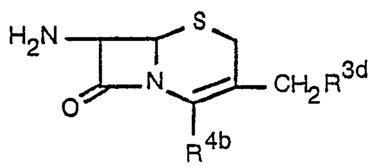

worin $R^{3d}$ und $R^{4b}$ jeweils wie vorstehend definiert sind oder ihres reaktiven Derivats an der Aminogruppe oder eines Salzes davon mit einer Verbindung der Formel

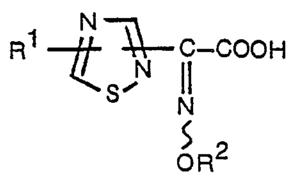

worin $R^1$ und $R^2$ jeweils wie vorstehend definiert sind oder ihrem reaktiven Derivat an der Carboxygruppe oder einem Salz davon, oder

b) Unterwerfen einer Verbindung der Formel

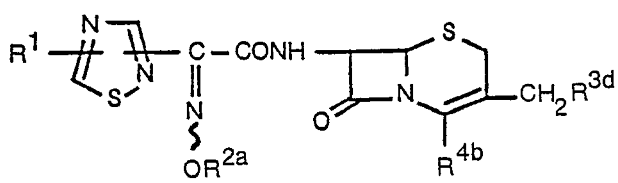

worin $R^1$, $R^{3d}$ und $R^{4b}$ jeweils wie vorstehend definiert sind und $R^{2a}$ geschütztes Carboxy-$(C_{1-6})$-alkyl ist oder eines Salzes davon der Eliminierungsreaktion der Carboxy-Schutzgruppe, unter Bildung einer Verbindung der Formel

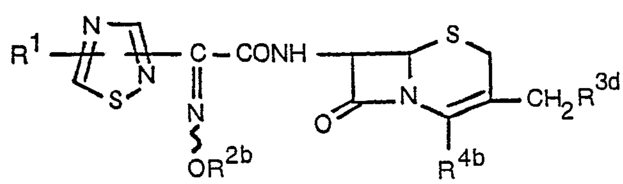

worin $R^1$, $R^{3d}$ und $R^{4b}$ jeweils wie vorstehend definiert sind und $R^{2b}$ Carboxy-$(C_{1-6})$-alkyl ist oder eines Salzes davon.

18. Pharmazeutische antibakterielle Zusammensetzung, enthaltend eine Verbindung nach Anspruch 1 zusammen mit einem pharmazeutisch brauchbaren, im wesentlichen nichttoxischen Träger oder Exzipienten.

19. Verbindung der Formel I zur Verwendung als Arzneimittel.

20. Verbindung der Formel I zur Verwendung bei der Behandlung von Infektionserkrankungen.

**Patentansprüche für den Vertragsstaat: AT**

1. Verfahren zur Herstellung neuer Cephemverbindungen der Formel

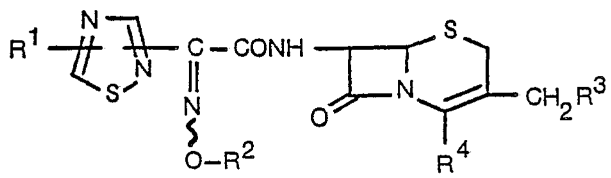

worin

$R^1$ Amino oder geschütztes Amino ist;

$R^2$ Wasserstoff, $(C_1—C_6)$-Alkyl, das substituiert sein kann mit Phenyl, Carboxy, verestertem Carboxy, $(C_1—C_6)$-Alkylthio oder Halogen, $(C_2—C_6)$-Alkenyl, $(C_2—C_6)$-Alkinyl, Cyclo-$(C_3—C_6)$-alkyl, Cyclo-$(C_3—C_6)$-alkenyl oder Tetrahydrofuryl substituiert mit einer Oxogruppe ist;

$R^3$ eine Gruppe der Formel

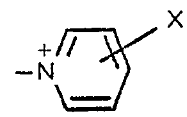

ist, worin X Wasserstoff oder Carbamoyl ist; und

$R^4$ $COO^-$ ist; oder

$R^3$ 2-$(C_1—C_6)$-Alkyl-5-oxo-6-hydroxy-2,5-dihydro-1,2,4-triazinylthio ist; und

$R^4$ Carboxy oder verestertes Carboxy ist oder der pharmazeutisch brauchbaren Salze davon durch Reaktion einer Verbindung der Formel

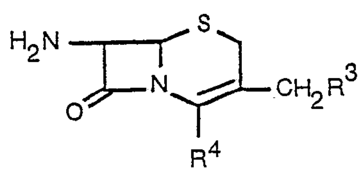

worin $R^3$ und $R^4$ jeweils wie vorstehend definiert sind oder ihres reaktiven Derivats an der Aminogruppe oder eines Salzes davon mit einer Verbindung der Formel

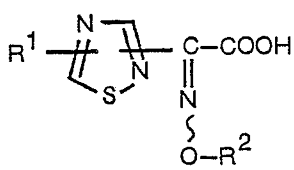

worin $R^1$ und $R^2$ jeweils wie vorstehend definiert sind, oder ihrem reaktiven Derivat an der Carboxygruppe oder einem Salz davon.

2. Verfahren zur Herstellung neuer Cephemverbindungen der Formel

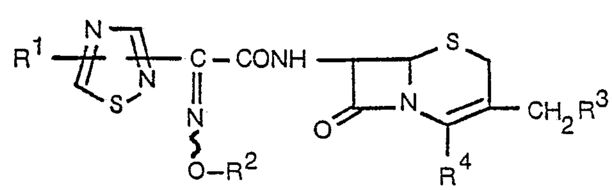

worin

$R^1$ Amino oder geschütztes Amino ist;

$R^2$ Wasserstoff, $(C_1—C_6)$-Alkyl, das substituiert sein kann mit Phenyl, Carboxy, verestertem Carboxy, $(C_1—C_6)$-Alkylthio oder Halogen, $(C_2—C_6)$-Alkenyl, $(C_2—C_6)$-Alkinyl, Cyclo-$(C_3—C_6)$-alkyl, Cyclo-$(C_3—C_6)$-alkenyl oder Tetrahydrofuryl substituiert mit einer Oxogruppe ist;

$R^3$ eine Gruppe der Formel

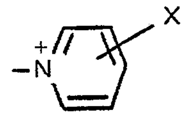

ist, worin X Wasserstoff oder Carbamoyl ist; und

$R^4$ $COO^-$ ist oder

$R^3$ 2-($C_1$—$C_6$)-Alkyl-5-oxo-6-hydroxy-2,5-dihydro-1,2,4-triazinylthio ist; und

$R^4$ Carboxy oder verestertes Carboxy ist oder der pharmazeutisch brauchbaren Salze davon durch Reaktion einer Verbindung der Formel

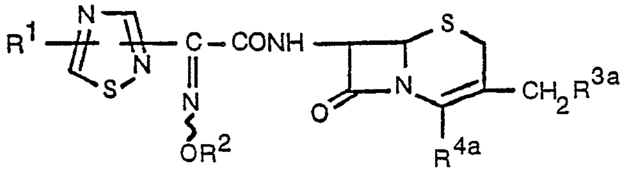

worin $R^1$ und $R^2$ jeweils wie vorstehend definiert sind, $R^{3a}$ eine Gruppe ist, die ersetzt werden kann durch eine Gruppe der Formel $R^3$ worin $R^3$ wie vorstehend definiert ist; und $R^{4a}$ Carboxy ist, wenn $R^{3b}$ eine Verbindung der Formel

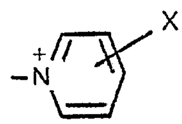

ist, worin X wie vorstehend definiert ist; oder $R^{4a}$ Carboxy oder verestertes Carboxy ist, wenn $R^{3b}$ eine Verbindung der Formel $R^{3c}$-H ist, worin $R^{3c}$ 2-($C_{1-6}$)-Alkyl-5-oxo-6-hydroxy-2,5-dihydro-1,2,4-triazinylthio ist; oder eines Salzes davon mit einer Verbindung der Formel

$R^{3b}$

worin $R^{3b}$ wie vorstehend definiert ist oder ihrem reaktiven Derivat.

3. Verfahren zur Herstellung des syn-Isomeren einer Verbindung der Ansprüche 1 oder 2.

4. Verfahren zur Herstellung einer Verbindung nach einem der Ansprüche 1 bis 3, worin die Gruppe

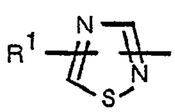 folgende Formel hat: 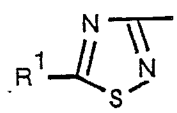 .

5. Verfahren zur Herstellung einer Verbindung gemäß einem der Ansprüche 1 bis 4, worin $R^1$ Amino ist; $R^2$ Wasserstoff, ($C_1$—$C_6$)-Alkyl, das substituiert sein kann mit 1 bis 3 Substituenten, ausgewählt aus der Gruppe von Halogen, ($C_1$—$C_6$)-Alkylthio, Carboxy, verestertem Carboxy und Aryl, ($C_2$—$C_6$)-Alkenyl, ($C_2$—$C_6$)-Alkinyl, Cyclo-($C_3$—$C_6$)-alkyl, Cyclo-($C_3$—$C_6$)-alkenyl, oder Tetrahydrofuryl substituiert mit einer Oxogruppe ist.

6. Verfahren zur Herstellung einer Verbindung nach einem der Ansprüche 1 bis 5, worin $R^2$ Wasserstoff, Methyl, Ethyl, Propyl, Isopropyl, 2,2,2-Trifluoroethyl, Methylthiomethyl, Carboxymethyl, 1-Methyl-1-carboxyethyl, 1-Carboxyethyl, Methoxycarbonylmethyl, Ethoxycarbonylmethyl, 1-Methyl-1-ethoxycarbonylethyl, t-Butoxycarbonylmethyl, 1-t-Butoxycarbonylethyl, 1-Methyl-1-t-butoxycarbonylethyl, 1-Benzyloxycarbonylethyl, Trityl, Allyl, 2-Propinyl, Cyclopentyl, 2-Cyclopenten-1-yl, 2-Cyclohexen-1-yl oder 2-Oxotetrahydrofuran-3-yl ist.

7. Verfahren zur Herstellung einer Verbindung nach einem der Ansprüche 1 bis 6, worin $R^3$ eine Gruppe ist mit der Formel

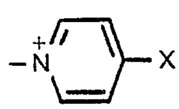

worin X Wasserstoff oder Carbamoyl ist und $R^4$ —$COO^-$ ist.

8. Verfahren zur Herstellung einer Verbindung gemäß einem der Ansprüche 1 bis 6, worin $R^3$ 2-Methyl-5-oxo-6-hydroxy-2,5-dihydro-1,2,4-triazinylthio und $R^4$ Carboxy ist.

9. Verfahren zur Herstellung einer Verbindung nach einem der Ansprüche 1 bis 5, worin $R^2$

Carboxy-($C_{1-6}$)-alkyl, ($C_{2-7}$)-Alkoxycarbonyl-($C_{1-6}$)-alkyl oder ($C_{2-6}$)-Alkenyl ist; $R^3$ eine Gruppe der Formel

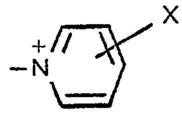

ist, worin X Wasserstoff ist und $R^4$ —$COO^-$ ist.

10. Verfahren zur Herstellung einer Verbindung nach einem der Ansprüche 1 bis 5 und 9, worin $R^2$ Carboxymethyl, t-Butoxycarbonylmethyl oder Allyl ist.

11. Verfahren zur Herstellung einer Verbindung nach Anspruch 10, nämlich 7-[2-Carboxymethoxyimino - 2 - (5 - amino - 1,2,4 - thiadiazol - 3 - yl) - acetamido] - 3 - (1 - pyridiniomethyl) - 3-cephem - 4 - carboxylat (syn-Isomeres).

12. Verfahren zur Herstellung einer Verbindung nach Anspruch 10, nämlich von 7-[2-Allyloxyimino - 2 - (5 - amino - 1,2,4 - thiadiazol - 3 - yl) - acetamido] - 3 - (1 - pyridiniomethyl) - 3-cephem - 4 - carboxylat (syn-Isomeres).

13. Verfahren zur Herstellung einer Verbindung der Formel

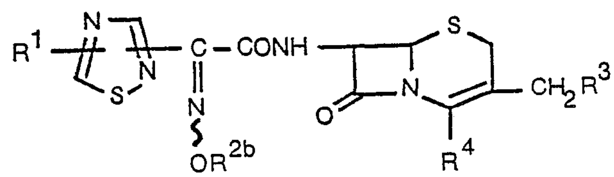

worin

$R^1$ Amino oder geschütztes Amino ist;

$R^{2b}$ Carboxy-($C_{1-6}$)-alkyl ist;

$R^3$ eine Gruppe der Formel

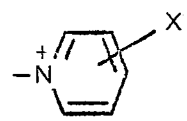

ist, worin X Wasserstoff oder Carbamoyl ist; und

$R^4$ —$COO^-$ ist; oder

$R^3$ 2-($C_{1-6}$)-Alkyl-5-oxo-6-hydroxy-2,5-dihydro-1,2,4-triazinylthio ist; und

$R^4$ Carboxy oder verestertes Carboxy ist, oder der pharmazeutisch brauchbaren Salze davon durch Unterwerden einer Verbindung der Formel

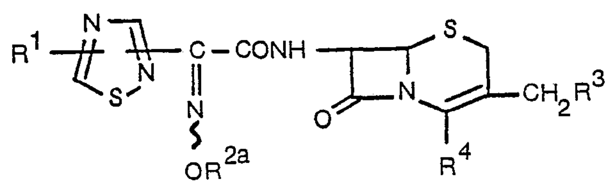

worin $R^1$, $R^3$ und $R^4$ jeweils wie vorstehend definiert sind und $R^{2a}$ verestertes Carboxy-($C_{1-6}$)-alkyl ist oder eines Salzes davon der Eliminierungsreaktion der Carboxy-Schutzgruppe.

14. Verfahren zur Herstellung einer Verbindung der Formel

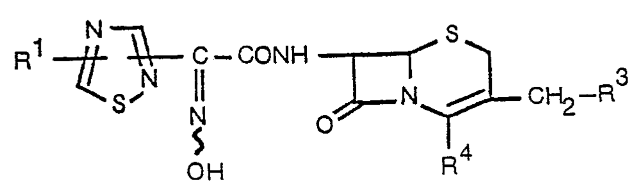

worin

$R^1$ Amino oder geschütztes Amino ist;

$R^3$ eine Gruppe der Formel

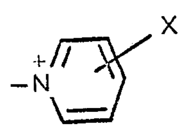

ist, worin X Wasserstoff oder Carbamoyl ist; und

$R^4$ —$COO^-$ ist; oder

$R^3$ 2-($C_{1-6}$)-Alkyl-5-oxo-6-hydroxy-2,5-dihydro-1,2,4-triazinylthio ist; und

$R^4$ Carboxy oder verestertes Carboxy ist; oder der pharmazeutisch brauchbaren Salze davon durch Unterwerfen einer Verbindung der Formel

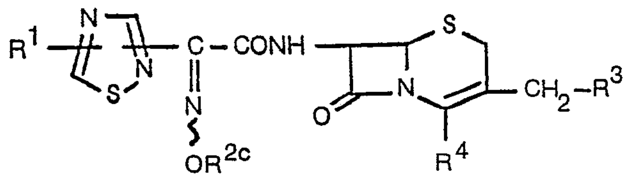

worin $R^1$, $R^3$ und $R^4$ jeweils wie vorstehend definiert sind und $R^{2a}$ eine Hydroxy-Schutzgruppe ist oder eines Salzes davon der Eliminierungsreaktion der Hydroxyschutzgruppe.

15. Verfahren zur Herstellung einer Verbindung der Formel

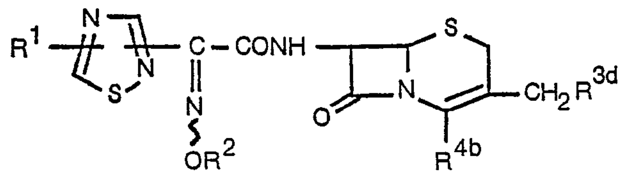

worin

$R^1$ Amino oder geschütztes Amino ist;

$R^2$ Wasserstoff, $(C_{1-6})$-Alkyl, das substituiert sein kann mit Phenyl, Carboxy, verestertem Carboxy, $(C_{1-6})$-Alkylthio oder Halogen, $(C_{2-6})$-Alkenyl, $(C_{2-6})$-Alkinyl, Cyclo-$(C_{3-6})$-alkyl, Cyclo-$(C_{3-6})$-alkenyl oder Tetrahydrofuryl substituiert mit einer Oxogruppe ist;

$R^{3d}$ $(C_{1-6})$-Alkanoyl-$(C_{1-6})$-alkanoyloxy ist und

$R^{4b}$ Carboxy oder verestertes Carboxy ist; oder eines Salzes davon durch

a) Reaktion einer Verbindung der Formel

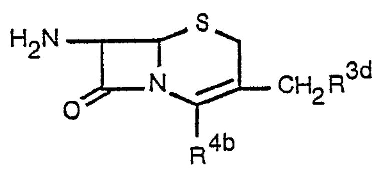

worin $R^{3d}$ und $R^{4b}$ jeweils wie vorstehend definiert sind oder ihres reaktiven Derivats an der Aminogruppe oder eines Salzes davon mit einer Verbindung der Formel

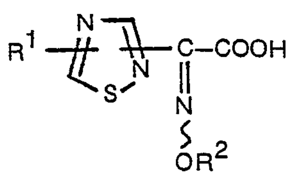

worin $R^1$ und $R^2$ jeweils wie vorstehend definiert sind oder ihrem reaktiven Derivat an der Carboxygruppe oder einem Salz davon oder

b) Unterwerfen einer Verbindung der Formel

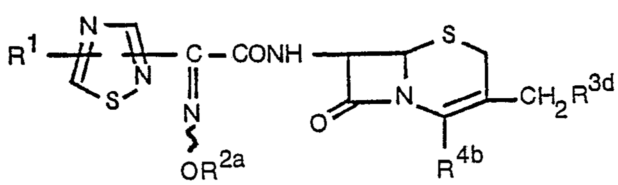

worin $R^1$, $R^{3d}$ und $R^{4b}$ jeweils wie vorstehend definiert sind und $R^{2a}$ geschütztes Carboxy-$(C_{1-6})$-alkyl ist oder eines Salzes davon der Eliminierungsreaktion für die Carboxy-Schutzgruppe unter Bildung einer Verbindung der Formel

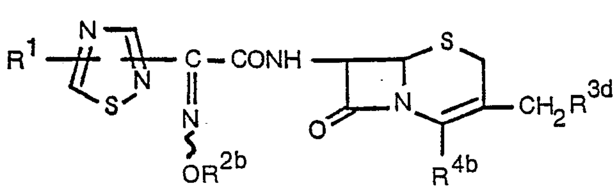

worin $R^1$, $R^{3d}$ und $R^{4b}$ jeweils wie vorstehend definiert sind und $R^{2b}$ Carboxy-$(C_{1-6})$-alkyl ist oder eines Salzes davon.